# EUROPEAN PATENT SPECIFICATION

(11) **EP 4 308 720 B1**
(45) Date of publication and mention of the grant of the patent: **07.05.2025**
(21) Application number: 22716384.7
(22) Date of filing: 18.03.2022
(51) Int. Cl.: C12Q 1/6804, C12N 15/10, C12Q 1/682

(54) **METHODS FOR THE SELECTIVE ANALYSIS OF CELLS OR ORGANELLES**
VERFAHREN ZUR SELEKTIVEN ANALYSE VON ZELLEN ODER ORGANELLEN
PROCÉDÉS D'ANALYSE SÉLECTIVE DE CELLULES OU D'ORGANITES

(30) Priority: 19.03.2021 EP 21315047
(43) Date of publication of application: 24.01.2024
(73) Proprietor: PARIS SCIENCES ET LETTRES, 75006 Paris (FR); Centre National de la Recherche Scientifique, 75016 Paris (FR); Ecole Supérieure de Physique et de Chimie Industrielles de la Ville de Paris, 75005 Paris (FR)
(72) Inventor: RONDELEZ, Yannick, 75010 Paris (FR); GRIFFITHS, Andrew, 75006 Paris (FR); BLIVET-BAILLY, Gaël, 75005 Paris (FR); IBANEZ, Pablo, 95100 Argenteuil (FR); BANERJEE, Satyam, 75014 Paris (FR); GINES, Guillaume, 75014 Paris (FR); MASURIER, Antoine, 75008 Paris (FR)
(74) Representative: Novagraaf Group
(86) International application number: PCT/EP2022/057187
(87) International publication number: WO 2022/195089

(56) References cited:
- WO-A1-2017/053905
- WO-A1-2017/205344
- WO-A1-2018/017949
- WO-A1-2018/140966
- WO-A1-2018/144813
- WO-A1-2018/147794
- WO-A1-2018/170013
- WO-A1-2019/033203
- WO-A1-2019/173460
- WO-A1-2019/213294
- WO-A1-2020/037065
- WO-A1-2020/069045
- WO-A1-2020/154247
- WO-A1-2021/067851
- WO-A1-2021/124166
- WO-A2-2018/058073
- WO-A2-2021/022085
- MICHAEL S. REID ET AL: "Exponential Isothermal Amplification of Nucleic Acids and Assays for Proteins, Cells, Small Molecules, and Enzyme Activities: An EXPAR Example", ANGEWANDTE CHEMIE INTERNATIONAL EDITION, vol. 57, no. 37, 27 April 2018 (2018-04-27), Hoboken, USA, pages 11856 - 11866, XP055628482, ISSN: 1433-7851, DOI: 10.1002/anie.201712217
- DUPIN AURORE ET AL: "Signalling and differentiation in emulsion-based multi-compartmentalized in vitro gene circuits", NATURE CHEMISTRY, NATURE PUBLISHING GROUP UK, LONDON, vol. 11, no. 1, 26 November 2018 (2018-11-26), pages 32 - 39, XP036657601, ISSN: 1755-4330, [retrieved on 20181126], DOI: 10.1038/S41557-018-0174-9
- QIAN CHENG ET AL: "Nicking enzyme-assisted amplification (NEAA) technology and its applications: A review", ANALYTICA CHIMICA ACTA, ELSEVIER, AMSTERDAM, NL, vol. 1050, 26 October 2018 (2018-10-26), pages 1 - 15, XP085581698, ISSN: 0003-2670, DOI: 10.1016/J.ACA.2018.10.054
- LI LU ET AL: "Highly Sensitive and Homogeneous Detection of Membrane Protein on a Single Living Cell by Aptamer and Nicking Enzyme Assisted Signal Amplification Based on Microfluidic Droplets", vol. 86, no. 10, 20 May 2014 (2014-05-20), US, pages 5101 - 5107, XP055832197, ISSN: 0003-2700, Retrieved from the Internet <URL:https://pubs.acs.org/doi/pdf/10.1021/ac500881p> DOI: 10.1021/ac500881p
- ZHANG ZHEN-ZHU ET AL: "Highly Sensitive Detection of Protein with Aptamer-Based Target-Triggering Two-Stage Amplification", vol. 84, no. 3, 7 February 2012 (2012-02-07), US, pages 1623 - 1629, XP055832203, ISSN: 0003-2700, Retrieved from the Internet <URL:https://pubs.acs.org/doi/pdf/10.1021/ac2029002> DOI: 10.1021/ac2029002

## Description

### Field of the invention

The present invention relates to methods for the selective analysis of cells or organelles.

### Technical background

Single cell multi-omic analysis is a growing field. The 2018 market is $ 300M and it is expected to be $ 1.6B in 2022 (Biolnformatics, LLC).

Single-cell analysis has become an indispensable tool to overcome the cellular heterogeneity of healthy and malignant tissues, since analysing a population of cells cannot reveal its heterogeneity.

Besides, a better understanding of complex and heterogeneous biological systems is highly needed and this requires -omic information of subpopulations of interest defined by their phenotype (expressed ligands, membrane receptors, expressed proteins, ...) and which are very little represented (for example less than 1% of the starting sample).

The analysis of -omics or phenotype is now possible at high flow rate at the single cell scale, but requires sequencing all the cells of a sample.

Reducing the number of cells to be sequenced would make it possible to focus on rare events, complex sets of cell populations or to provide competitive tools for theranostic purpose. The possibility of selecting a subpopulation for sequencing is not currently proposed by any commercial solution, apart from using flow cytometry before the single-cell analysis. However, flow cytometry followed by single-cell analysis requires expensive equipment and is not suitable for some kinds of cells, nor to temporal tracking of cells.

Thus, there is currently no technology for single cell compartmentalization with high throughput ability to select a subpopulation to be analysed by sequencing. Existing technologies for example have the following drawbacks:
- Flow cytometry, FACS: a large number of cells have to be sorted for the presence of certain membrane markers at a given time. It requires coupling to another technology in order to perform the single cell analysis,
- Drop microfluidics, "10X Genomics": analysis on single cell level without temporal visualization or sorting,
- Micro-fluidic trap, "Fluidigm C1": analysis of up to few hundred to thousand single cells with visualization but without sorting,
- Micro-well, nano-dispenser, "CellenONE": analysis of only few single cells with temporal visualization and sorting.

A microfluidic system for the detection of membrane protein on a single living cell has been developed using aptamer and nicking enzyme assisted signal amplification (Li et al. (2014) Anal. Chem. 86: 5101-5107). Its use is however limited by its low sensitivity as many markers exist at a concentration too low to be detected by such a method.

Polymerase amplification approaches have also been employed. Some have developed a method for the detection of the platelet-derived growth factor BB (PDGF-BB) based on aptamer-based target triggering two-stage amplification (Zhang et al. (2012) Anal. Chem. 84:1623-1629). Alternatively, the use of DNA-antibody conjugates associated to nicking enzymes and polymerases has also been described to amplify a signal by the accumulation of single stranded DNA (WO2020/069045). These methods are however not suitable for single cell analysis. The existing systems thus make it possible to isolate cells, mark them individually and carry out sequencing, but do not enable coupling the phenotype of the cell to its -omic features (for example genomic features, transcriptomic features, ...), nor selecting cells of interest from a heterogenous pool and sequence them individually.

There is thus a need of improved methods for the selective analysis of cells or organelles.

### Detailed description of the invention

The Inventors have designed a method which "transforms" a phenotypic marker of a cell or organelle into an amplified DNA signal, via the implementation of an adapted DNA-toolbox, which signal is used to select compartmentalized single cells or organelles displaying this phenotypic marker, for selective analysis of these cells or organelles, for example for sequencing. The phenotype selection can advantageously be made according to a surface marker, an internal marker, a secreted or released marker, a membrane marker or a combination of several markers. This new method allowing sorting and sequencing a subpopulation based on phenotypic criteria appears as a technological breakthrough.

The present invention advantageously makes it possible to perform high-speed cell selection operations (more than 10 000 cells per analysis) under very competitive cost conditions.

The present invention is based on dynamic circuits of molecular biology reactions called DNA-toolbox, developed by the team of Yannick Rondelez at the University of Tokyo and ESPCI Paris (Baccouche et al. (2014) Methods 67:234-249). These circuits are made from short stretch of oligonucleotide sequences (also called templates) and enzymes including polymerase, exonuclease and nickase.

The method according to the invention thus relies on DNA amplification reactions, for which the conditions of implementation appear incompatible with phenotypic analysis of living cells. However, surprisingly, the method of the invention can also be used for selective analysis of living cells.

A first object of the invention is thus a method for the selective analysis of a subpopulation of cells or organelles based on determining the level of at least one cell or organelle marker, said method comprising:
a) partitioning said cells or organelles into a plurality of compartments, so that the majority of the compartments do not comprise more than a single cell of one cellular type or a single organelle of one cellular type,
   together with at least one cell or organelle marker-specific ligand coupled to at least one activator oligonucleotide or repressor oligonucleotide and
   with a DNA toolbox mixture comprising at least one polymerase, at least one restriction or nicking enzyme, at least one amplification oligonucleotide able to bind and amplify a signal oligonucleotide, and a buffer comprising less than 0.7 mM phosphate ions,
b) in each compartment, letting the activator oligonucleotide(s) and/or the repressor oligonucleotide(s) bound to the cell or organelle marker, interact together and with the components of the DNA toolbox mixture, to generate one or more signal oligonucleotides and/or one or more anti-signal oligonucleotides and to amplify said signal oligonucleotide(s),
c) analyzing the cells or organelles of the compartments in which at least one signal oligonucleotide is present at a concentration over or below a threshold value.

Another object of the invention is a kit for implementing the method as defined above, wherein said kit comprises:
a) at least one polymerase, at least one nicking enzyme or restriction enzyme, and optionally at least one enzyme selected from the group consisting of an exonuclease, an endonuclease, a reverse transcriptase, a ligase, a recombinase, a glycosylase and a DNA-processing enzyme,
b) at least one cell or organelle marker-specific ligand coupled to at least one activator or repressor oligonucleotide,
c) at least one amplification oligonucleotide able to bind and amplify a signal oligonucleotide, and, optionally, at least one oligonucleotide selected from the group consisting of a leak-absorption oligonucleotide to avoid nonspecific amplification, a conversion oligonucleotide able to convert the activator or repressor oligonucleotide into a signal or anti-signal oligonucleotide, a reporting probe allowing detection of a signal oligonucleotide and a quantification oligonucleotide allowing determining the concentration of a signal oligonucleotide,
d) a set of barcoded nucleic acids comprising a barcoded primer, a barcoded template switching oligonucleotide, a barcoded adapter or a barcoded recombination site, and
e) optionally, a microfluidic device.

Another object of the invention is the use of the kit as defined above for the selective analysis of a subpopulation of cells or organelles, in particular based on determining the level of at least one cell or organelle marker.

### Cells or organelles

The present invention allows selecting a subpopulation of cells or organelles of interest, in particular based on their phenotype, such as the level of at least one cell or organelle marker.

By "selecting a subpopulation of cells or organelles based on their phenotype", it is herein meant selecting a set of cells or organelles having the phenotype of interest, in particular among a heterogeneous population of cells or organelles.

The cells may for example be living cells, frozen cells or permeabilized cells, for example fixed permeabilized cells.

The cells may be permeabilized by any method well known by the skilled person.

The cells may for example be eukaryotic cells or prokaryotic cells.

The cells are preferably eukaryotic cells, more preferably mammalian cells, for example human cells.

Non-limiting examples of non-human mammalian cells include rat cells, murine cells, hamster cells or monkey cells.

The mammalian cells are preferably human cells.

The cells may be obtained from a cell line or from a sample of a subject.

The cells are preferably obtained from a sample of a subject, for example a human subject. Said subject may be a heathy subject or a subject at risk of suffering of a disease or suffering from a disease.

The sample may for example be a tissue sample or a fluid sample, such as a blood sample.

The tissue sample may for example be a tumor sample or an inflammation tissue sample.

The cells may comprise two different kinds of cells, such as for example a T cell and an antigen-presenting cell (APC).

The organelle may for example be a mitochondria or a nucleus.

The organelles may be obtained from cells as defined above, in particular by any method well known by the skilled person.

### Cell or organelle marker

To select a subpopulation of cells or organelles of interest, as defined above, in particular based on their phenotype, the present invention allows detecting the presence or absence of at least one cell or organelle marker.

The cell or organelle marker may for example be a receptor.

The cell or organelle marker may be for example be a protein, a glycoprotein, a lipoprotein, a glycolipid or a carbohydrate.

A cell marker is used for selecting a subpopulation of cells of interest.

The cell marker may for example be selected from the group consisting of a surface marker, an internal marker, a secreted or released marker and/or a membrane marker.

When the phenotype of interest in based on the presence or absence of an internal marker, the cells are preferably permeabilized.

When the phenotype of interest in based on the presence or absence of a cell secreted or released marker, the cells are living cells.

When the method is further used for sequencing the DNA or RNA of the selected subpopulation of cells, the cells are preferably living cells.

An organelle marker is used for selecting a subpopulation of organelles of interest.

The organelle marker may for example be selected from the group consisting of a surface marker, a membrane marker, an internal marker, a secreted or released organelle marker.

### Cell or organelle marker-specific ligand

A cell or organelle marker-specific ligand is used for detecting the cell or organelle marker as defined above.

The cell or organelle marker-specific ligand may be any molecule able to specifically bind to said cell or organelle marker.

The cell or organelle marker-specific ligand may for example be selected from the group consisting of a protein, a peptide, an antibody or fragment thereof, an aptamer, a RNA (*Ribonucleic Acid*), a DNA (*Deoxyribonucleic Acid*), a lectin and a membrane anchor.

The antibody is preferably a monoclonal antibody.

By the term "aptamer", it is herein meant a nucleic acid which, through its ability to adopt a specific three-dimensional conformation, is able to specifically bind to a target, for example a cell or organelle marker as defined above. The aptamer may comprise ribonucleotides, deoxyribonucleotides or a mixture of both types of nucleotide residues. The aptamer may further comprise one or more modified bases, sugars or phosphate backbone units.

An aptamer specific of Ramos cells may for example comprise or consist of sequence SEQ ID NO: 16 or SEQ ID NO: 17.

The membrane anchor binds to cell membranes. The membrane anchor may for example be cholesterol. When using a non-specific membrane anchor, such as cholesterol, the subpopulation of cells corresponds to the whole cell population.

The cell or organelle marker-specific ligand is preferably an antibody or an aptamer, as defined above.

The cell or organelle marker-specific ligand used according to the invention is coupled to at least one activator or repressor oligonucleotide, using covalent or noncovalent bonds.

Coupling of the activator or repressor oligonucleotide to the marker specific ligand may be performed by any suitable method well known by the skilled person.

For example, a spacer, which may be a DNA spacer or a non-nucleic spacer, may be used. Said spacer may be attached in 3' or 5', in the case of protein peptidic ligands (such as an antibody). Coupling may be performed by biotin streptavidin linkage or by covalent modifications (see for example Dovgan, Igor, et al. "Antibody-oligonucleotide conjugates as therapeutic, imaging, and detection agents." Bioconjugate chemistry 30.10 (2019): 2483-2501).

In the case of an aptamer, the two sequences may be simply fused together or, optionally, with spacer or linker.

By "activator oligonucleotide", it is herein meant an oligonucleotide able to lead to the production of a signal oligonucleotide, or a signal oligonucleotide.

An oligonucleotide able to lead to the production of a signal oligonucleotide may for example be (i) a precursor oligonucleotide, (ii) a conversion oligonucleotide or (iii) a precursor oligonucleotide bound to or fused to a conversion oligonucleotide or to a sequence complementary to the signal sequence.

By "repressor oligonucleotide", it is herein meant an oligonucleotide able to lead to the production of an anti-signal oligonucleotide.

An oligonucleotide able to lead to the production of an anti-signal oligonucleotide may as example be (i) a precursor oligonucleotide, (ii) an anti-signal conversion oligonucleotide or (iii) a precursor oligonucleotide bound to or fused to an anti-signal conversion oligonucleotide or to a sequence complementary to the anti-signal sequence.

A precursor oligonucleotide is an oligonucleotide complementary to a region of a conversion or anti-signal conversion oligonucleotide, in particular to a region located in 3' of the conversion or anti-signal conversion oligonucleotide.

By the term "conversion oligonucleotide" (also referred to as "conversion template" "converter template" or "cT"), it is herein meant an oligonucleotide, which converts the presence of at least one cell or organelle marker to a signal oligonucleotide (also called herein "trigger oligonucleotide" or "trigger signal").

By the term "anti-signal conversion oligonucleotide", it is herein meant an oligonucleotide, which converts the presence of at least one cell or organelle marker to an anti-signal oligonucleotide (also called herein "trigger oligonucleotide" or "trigger signal").

The precursor oligonucleotide bound to or fused to a conversion oligonucleotide is a conversion oligonucleotide that is already primed for polymerisation.

The precursor oligonucleotide bound to or fused to a conversion oligonucleotide is herein also called a "source template", "sT" or "self-primed template".

The source template preferably comprises, from 5' to 3':
- a region complementary to the signal oligonucleotide or to the anti-signal oligonucleotide,
- optionally, an optimization sequence, for example GTTT or GAGA,
- an enzyme recognition site, preferably a nicking enzyme recognition site, and
- a loop allowing the 3' extremity of the source template to hybridize to the restriction site and to be ready to be extended by a polymerase.

In presence with the DNA toolbox mixture, the source template constitutively produces the signal oligonucleotide or anti-signal oligonucleotide.

The source template is bound to the cell or organelle marker-specific ligand, preferably via its 5" extremity, for example via a biotin-streptavidin linkage.

Biotin is preferably attached in 5' of the source template.

An anti-signal oligonucleotide is an oligonucleotide, which is at least substantially complementary to a signal oligonucleotide and comprises a 5' overhang.

Said 5' overhang can be used as a template by a polymerase to extend a signal oligonucleotide, thereby deactivating it for extension on the amplification template.

The 5' overhang thus enables adding a deactivating tail to the signal oligonucleotide.

The anti-signal oligonucleotide is thus able to bind to the signal oligonucleotide, deactivate the signal oligonucleotide but is not able to amplify the signal oligonucleotide.

Advantageously, the 5' overhang is short, so that (i) the anti-signal production rate from the repressor oligonucleotide is high and (ii) the extended signal oligonucleotide can de-hybridize spontaneously from the anti-signal oligonucleotide at the working temperature, so that one anti-signal oligonucleotide can perform multiple rounds of deactivation of multiple signal oligonucleotides.

The anti-signal oligonucleotide preferably comprises from 10 to 22 nucleotides, more preferably from 10 to 15 nucleotides, for example 12, 13, 14, 15, 16 or 17 nucleotides.

The mechanism by which anti-signal oligonucleotides can deactivate signal oligonucleotides is for example described in document WO2017141067 or in Montagne et al., 2016, Nat Commun 7, 13474 (doi.org/10.1038/ncomms13474).

The activator or repressor oligonucleotide may comprise from 8 to 80 nucleotides, preferably from 10 to 50 nucleotides, more preferably from 10 to 30 nucleotides, for example from 15 to 25 nucleotides or from 16 to 22 nucleotides.

The activator or repressor oligonucleotide is a single-stranded oligonucleotide or a partially-double-stranded oligonucleotide.

The expression "an oligonucleotide X able to bind to an oligonucleotide Y" herein means that the oligonucleotide X comprises a sequence that is at least partially complementary to a region of the sequence of said oligonucleotide Y, said complementarity being sufficient to yield specific binding, in particular under the conditions of the claimed method.

A sequence that is at least partially complementary to a second sequence is either complementary to said second sequence or partially complementary to said second sequence.

A sequence that is "complementary to" a second sequence is herein meant the reverse complement counterpart of the second sequence. A sequence is "partially complementary to" a second sequence if there are one or more mismatches, preferably at most four mismatches, or if the first sequence is shorter than the second sequence.

The activator oligonucleotide for use according to the invention may for example comprise or consist of sequence SEQ ID NO: 6, sequence SEQ ID NO: 18, sequence SEQ ID NO: 10 or a fragment thereof, for example a fragment consisting of nucleotides 11 to 32 of sequence SEQ ID NO: 10.

An aptamer specific of Ramos cells coupled to an activator oligonucleotide for use according to the invention may for example comprise or consist of sequence SEQ ID NO: 8 or SEQ ID NO: 9.

### DNA toolbox

As explained above, the method of the invention "transforms" the presence of a cell or organelle marker into an amplified DNA signal, via the implementation of an adapted DNA toolbox.

The DNA toolbox relies on a set of short oligonucleotides, herein also referred to as templates, and a mixture of at least two enzymes, which process the information fluxes by producing and degrading DNA strands, which in turn activate or inhibit other components of the DNA toolbox.

The method of the invention thus comprises the use of a DNA toolbox mixture.

The DNA toolbox mixture preferably comprises at least two enzymes and at least one oligonucleotide.

### Enzyme

The DNA toolbox mixture comprises at least two enzymes, preferably at least one polymerase and at least one restriction or nicking enzyme.

By the term "enzyme", it is herein meant a protein having catalytic function and which is able to catalyze the transformation of a molecular substrate.

The enzyme as defined above is preferably selected from the group consisting of a polymerase, a nicking enzyme, a restriction enzyme, an endonuclease, an exonuclease, a reverse transcriptase, a ligase, a recombinase, a glycosylase and a DNA-processing enzyme.

The polymerase and the nicking enzyme and/or the restriction enzyme particularly allow producing and amplifying the signal oligonucleotide.

The polymerase for use according to the invention is preferably selected from the group consisting of Bst 2.0 DNA polymerase, Bst large fragment DNA polymerase, Klenow fragment (3'->5'exo-), Phi29 DNA polymerase, Vent(exo-) DNA polymerase, more particularly, the polymerase is Vent(exo-) DNA polymerase (for example purchased from New England Biolabs (NEB)). More than one polymerase can be used simultaneously.

The nicking enzyme, also called nickase, for use according to the invention is preferably selected from the group consisting of Nb.BbvCl, Nb.Bstl, Nb.BssSI, Nb.BsrDl, Nb.Bsml and Nt.BstNBI, more preferably Nb.Bsml and/or Nt.BstNBI (for example purchased from New England Biolabs (NEB)). More than one nickase can be used simultaneously.

According to one embodiment, the nicking enzyme may be replaced by a restriction enzyme.

Contrary to the nicking enzymes, which cut only one strand of a DNA duplex, the restriction enzymes cut the two strands. Thus, when using restriction enzymes instead of nicking enzymes, it may be necessary to protect the templates used in the method of the invention. This protection may be performed for example by performing chemical modification of the templates. Such modifications comprise backbone modification such as phosphorothioate linkage.

More than one restriction enzyme or a combination of nicking enzyme and restriction enzyme can be used simultaneously.

Non-limitative examples of DNA-processing enzyme are APE1 or UDG.

The exonuclease particularly allows avoiding saturation of the system.

The exonuclease for use according to the invention is preferably selected from the group consisting of RecJf, Exonuclease I, Exonuclease VII and ttRecJ exonuclease. The exonuclease is preferably ttRecJ exonuclease, such as obtained following the protocol described by Yamagata (Yamagata et al., 2001). More than one exonuclease can be used simultaneously.

The DNA toolbox mixture may further comprise a reverse transcriptase, in particular when the method is further used for sequencing the DNA or RNA of the selected population of cells.

The DNA toolbox mixture preferably comprises at least one polymerase and at least one enzyme selected among a restriction enzyme and a nicking enzyme.

The DNA toolbox mixture preferably comprises:
- at least one polymerase, in particular
   ∘ to synthetize the complementary strand of the conversion oligonucleotide using the bound or fused precursor oligonucleotide as a primer, thereby producing a double-stranded conversion oligonucleotide,
   ∘ to synthetize the complementary strand of the anti-signal conversion oligonucleotide using the bound or fused precursor oligonucleotide as a primer, thereby producing a double-stranded anti-signal conversion oligonucleotide,
   ∘ to synthetize the complementary strand of the amplification oligonucleotide using the bound signal oligonucleotide as a primer, thereby producing a double-stranded amplification oligonucleotide,
   ∘ to synthetize the complementary strand of the leak-absorption oligonucleotide or of the anti-signal oligonucleotide using the bound signal oligonucleotide as a primer, to produce a double-stranded leak absorption oligonucleotide or a double-stranded anti-signal oligonucleotide,
   ∘ to synthetize the complementary strand of the reporting probe using the bound signal oligonucleotide as a primer,
   ∘ in the case where the polymerase used has strand displacement activity, to strand-displace an oligonucleotide bound to a template oligonucleotide by extension of a primer, and/or
- at least one enzyme selected from a restriction enzyme and/or a nickase, in particular allowing releasing the signal oligonucleotide from the double-stranded conversion oligonucleotide and/or releasing the anti-signal oligonucleotide from the double-stranded anti-signal conversion oligonucleotide and/or releasing the signal oligonucleotide from the double-stranded amplification oligonucleotide, and/or releasing the mismatched signal oligonucleotide from the double-stranded leak-absorption or double-stranded anti-signal oligonucleotide, and/or releasing barcoded primers from a solid support,
- optionally, at least one exonuclease, at least one endonuclease, at least one ligase, at least one recombinase, at least one glycosylase and/or at least one DNA-processing enzyme, and
- optionally, at least one reverse transcriptase.

### Oligonucleotides

The DNA toolbox mixture comprises at least one oligonucleotide.

As it is clear for the skilled person, the expression "the DNA toolbox mixture comprise an oligonucleotide X" means that said mixture comprises molecules of said oligonucleotide X. For example, the DNA toolbox mixture may comprise at least one oligonucleotide at the desired concentration, for example from 0.1 nM to 100 nM.

The oligonucleotide may be selected from the group consisting of a conversion oligonucleotide, anti-signal conversion oligonucleotide, an amplification oligonucleotide, a leak-absorption oligonucleotide and a reporting probe.

The conversion oligonucleotide, the amplification oligonucleotide and the leak-absorption oligonucleotide used in the DNA toolbox mixture comprises at least one region able to bind the signal oligonucleotide.

Consequently, these oligonucleotides are linked by the sequence of the signal oligonucleotide.

The signal oligonucleotide is preferably an artificial oligonucleotide. The signal oligonucleotide is designed to preferably have the following features:
- a low melting temperature,
- a short sequence, preferably from 8 to 20 nucleotides, more preferably from 10 to 15 nucleotides, for example 10, 11, 12, 13, 14 or 15 nucleotides, and
- a high ratio of AT/ CG.

These features particularly allow the reactions of amplification to function at a temperature of 37°C, which temperature is particularly suitable when the method is carried out on living cells.

The signal oligonucleotide is a single-stranded oligonucleotide.

The signal oligonucleotide may for example comprise or consist of sequence SEQ ID NO: 1 or SEQ ID NO: 11.

Each oligonucleotide used in the DNA toolbox mixture has a specific function, finally allowing the conversion of the presence of at least one cell or organelle marker to a signal oligonucleotide and the amplification of the obtained signal oligonucleotide, preferably without inducing at the same time a background amplification, i.e. a signal amplification reaction happening in absence of the cell or organelle marker.

The DNA toolbox thus comprises at least one amplification oligonucleotide, which is able to bind and amplify a signal oligonucleotide.

By the term "amplification oligonucleotide" (also referred to as "autocatalytic template" or "aT"), it is herein meant an oligonucleotide, which is able to exponentially amplifies the signal oligonucleotide as defined above.

The amplification oligonucleotide is preferably protected against degradation, particularly the one to five first nucleotides in 5', for example the three or four first nucleotides in 5'.

The amplification oligonucleotide preferably comprises, from 5' to 3', a first region able to bind to the signal oligonucleotide, an enzyme recognition site (for example of a restriction enzyme or of a nicking enzyme) and a second region able to bind to the signal oligonucleotide.

The amplification oligonucleotide preferably comprises from 16 to 45 nucleotides, more preferably from 20 to 40 nucleotides, for example 20, 21, 22, 23, 24, 25, 26, 27, 28, 29 or 30 nucleotides.

Upon binding of the signal oligonucleotide to the amplification oligonucleotide and subsequent amplification by a polymerase, a double-stranded amplification oligonucleotide is obtained. Upon action of an enzyme recognizing said enzyme recognition site, at least two signal oligonucleotides are released.

According to a preferred embodiment, the 3' end of the amplification oligonucleotide preferably has a reduced affinity for the signal oligonucleotide.

The DNA toolbox mixture may further comprise at least one conversion oligonucleotide.

As disclosed above, the "conversion oligonucleotide" converts the presence of at least one cell or organelle marker to a signal oligonucleotide.

In some embodiments, the conversion oligonucleotide is not in the DNA toolbox mixture, but is provided in the form of an activator oligonucleotide, which is bound to a cell or organelle specific ligand.

The conversion oligonucleotide may comprise from 8 to 50 nucleotides, preferably from 10 to 45 nucleotides, more preferably from 10 to 30 nucleotides, for example from 15 to 25 nucleotides or from 16 to 22 nucleotides.

The conversion oligonucleotide may or may not be protected against degradation (in particular by restriction enzymes or nucleases).

The conversion oligonucleotide is able to bind to a precursor oligonucleotide, as defined above.

The conversion oligonucleotide preferably comprises, from 5' to 3', a first region complementary to the signal oligonucleotide, an enzyme recognition site, preferably a nicking enzyme recognition site, optionally a spacer region of arbitrary sequence, and a second region able to bind a precursor oligonucleotide.

The conversion oligonucleotide may also comprise, from 5' to 3', a region complementary to a first precursor oligonucleotide, a linker, a spacer, a first region complementary to the signal oligonucleotide, an enzyme recognition site, preferably a nicking enzyme recognition site, optionally a spacer region of arbitrary sequence, and a second region able to bind a second precursor oligonucleotide. Such a conversion oligonucleotide may be useful when a pair of ligands is used, wherein:
- the conversion oligonucleotide is bound to one ligand of said pair, by hybridization to a first precursor oligonucleotide coupled to said ligand, and
- a second precursor oligonucleotide is bound to the second ligand of said pair, by hybridization to a third precursor oligonucleotide coupled to said ligand, wherein the second precursor oligonucleotide comprises a region complementary to the third precursor oligonucleotide, a linker and a region complementary to said conversion oligonucleotide. The first and third precursor oligonucleotides may be identical or different. The first ligand and the second ligand of the pair may be identical.

The linker may for example be a sequence consisting of n T, wherein n is an integer greater or equal to 2, preferably greater or equal to 5, more preferably greater or equal to 10, such as sequence SEQ ID NO: 25.

The spacer may for example be a spacer region of arbitrary sequence or spacer sp18.

Spacer sp18 (also herein referred to as intsp18) is the compound of the following formula:

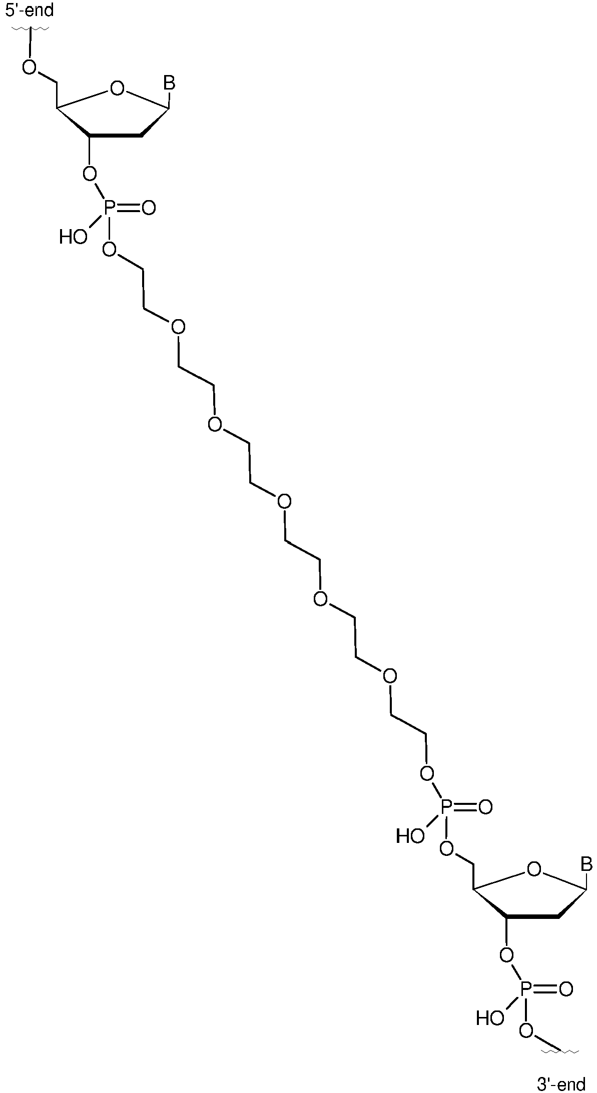

The conversion oligonucleotide as defined above can be a single-stranded oligonucleotide or a partially double-stranded oligonucleotide.

Upon binding of the conversion oligonucleotide to the precursor oligonucleotide and subsequent amplification by a polymerase, a double-stranded conversion oligonucleotide is obtained. Upon action of an enzyme recognizing said enzyme recognition site, preferably a nicking enzyme, a single-stranded oligonucleotide, which is called the signal oligonucleotide, is obtained.

The DNA toolbox mixture may further comprise at least one anti-signal conversion oligonucleotide.

The anti-signal conversion oligonucleotide may comprise from 8 to 50 nucleotides, preferably from 10 to 45 nucleotides, more preferably from 10 to 30 nucleotides, for example from 15 to 25 nucleotides or from 16 to 22 nucleotides.

The anti-signal conversion oligonucleotide may or may not be protected against degradation (in particular by restriction enzymes or nucleases).

The anti-signal conversion oligonucleotide is able to bind to a precursor oligonucleotide, as defined above.

The anti-signal conversion oligonucleotide preferably comprises, from 5' to 3', a first region comprising the signal oligonucleotide fused to second oligonucleotide (overall forming the complementary sequence of the anti-signal sequence), an enzyme recognition site, preferably a nicking enzyme recognition site, optionally a spacer region of arbitrary sequence and a second region able to bind a precursor oligonucleotide.

The anti-signal conversion oligonucleotide as defined above can be a single-stranded oligonucleotide or a partially double-stranded oligonucleotide.

The anti-signal conversion oligonucleotide is preferably a single-stranded oligonucleotide.

Upon binding of the anti-signal conversion oligonucleotide to the precursor oligonucleotide and subsequent amplification by a polymerase, a double-stranded anti-signal conversion oligonucleotide is obtained. Upon action of an enzyme recognizing said enzyme recognition site, preferably a nicking enzyme, a single-stranded oligonucleotide which is an anti-signal oligonucleotide is obtained.

In some embodiments, the anti-signal conversion oligonucleotide is not in the DNA toolbox mixture, but is provided in the form of a repressor oligonucleotide, which is bound to a cell or organelle specific ligand.

The DNA toolbox mixture may further comprise at least one precursor oligonucleotide.

The precursor oligonucleotide is as defined above.

In some embodiments, the precursor is not in the DNA toolbox mixture, but is provided in the form of an activator or repressor oligonucleotide, which is bound to a cell or organelle specific ligand.

The DNA toolbox may further comprise at least one leak-absorption oligonucleotide.

By the term "leak-absorption oligonucleotide" (also referred to as "pseudotemplate" or "pT"), it is herein meant an oligonucleotide, which binds the signal oligonucleotide more strongly than the 3' region of the amplification oligonucleotide, and only adds a few nucleotides at its 3' end and thus deactivates it for further priming on the amplification oligonucleotide (because its 3' end is now mismatched on the amplification oligonucleotide). The leak-absorption oligonucleotide allows avoiding nonspecific amplification, also called herein background amplification (i.e. the amplification occurring in absence of the cell or organelle marker). The leak-absorption oligonucleotide drives the deactivation of the signal sequence synthesized from leaky reactions. The leak-absorption oligonucleotide may be protected against degradation, exactly like the amplification oligonucleotide is.

The leak-absorption oligonucleotide preferably comprises from 10 to 22 nucleotides, more preferably from 10 to 15 nucleotides, for example 12, 13, 14, 15, 16 or 17 nucleotides.

The leak-absorption oligonucleotide comprises from 5' to 3' a first region enabling to add a deactivating tail to the signal oligonucleotide and a second region complementary to the signal oligonucleotide.

The leak-absorption oligonucleotide is thus an anti-signal oligonucleotide directly added in the DNA toolbox mixture, instead of being produced from a repressor oligonucleotide.

The concentrations of the amplification and leak absorption oligonucleotides are preferably selected so that a reaction of the amplification oligonucleotide is faster than the reaction on the leak-absorption oligonucleotide at high concentration of the amplified sequence, but the reaction on the leak-absorption oligonucleotide is faster than the reaction of the amplification oligonucleotide at low concentration of the amplified sequence, thereby effectively eliminating amplification unless the stimulus threshold is crossed.

As disclosed above, one or more of the oligonucleotides of the DNA toolbox mixture are preferably protected against enzymatic degradation.

Protection of an oligonucleotide as defined above from degradation may be obtained by any method well known by the skilled person, such as by at least one chemical modification.

Said chemical modification may be selected from the group consisting of (i) a backbone modification, for example, incorporation of a phosphorothioate, (ii) a base modification or (iii) a terminal modification. The oligonucleotide may for example be protected by incorporation of at least one, at least two, at least three or at least four, phosphorothioates at its 5' end and/or by other exonuclease-blocking means, such as biotin-streptavidin modification(s), inverted nucleotide(s) and/or modified nucleotide(s).

The DNA toolbox mixture preferably comprises (i) at least one amplification oligonucleotide, (ii) optionally, at least one conversion oligonucleotide or at least one precursor oligonucleotide and (iii) optionally, at least one leak absorption oligonucleotide.

The DNA toolbox mixture may additionally comprise a reporting probe.

By "reporting probe" (also referred to as "reporting template" or "rT"), it is herein meant a compound able to translate the presence of the signal oligonucleotide as defined above into a detectable signal. Such detectable signal is for example particles aggregation, media jellification, an electrochemical signal, a chemo-luminescent signal or a fluorescent signal.

The reporting probe comprises an oligonucleotide, preferably a single-stranded oligonucleotide, which is able to bind to the signal oligonucleotide.

In one preferred embodiment, the reporting probe comprises an oligonucleotide, preferably a single-stranded oligonucleotide, which binds to the signal oligonucleotide and is coupled to a detectable compound, such as a fluorophore.

In yet another preferred embodiment, the reporting probe is a self-complementary DNA strand modified at both extremities by a fluorophore and/or a quencher and further comprising a region binding to the signal oligonucleotide. As used herein, the term "self-complementary" means that two different parts of the same molecules can hybridize to each other due to base complementary (A-T and G-C). In the case of the invention, the two extremities of the single-stranded probe (for example a few nucleotides on the 3' and 5' part) can hybridize to each other and induce the quenching of the fluorophore by the quencher. The binding of the signal oligonucleotide to a portion of the reporting probe leads to the opening of stem-loop structure and thus the enhancement of the fluorescence signal.

The reporting probe may also comprise a loop, which includes a nicking recognition site.

The DNA toolbox mixture may additionally comprise at least one quantification oligonucleotide, in particular so as to generate a token oligonucleotide.

The token oligonucleotide is preferably an artificial oligonucleotide. The token oligonucleotide allows the quantification, for example by sequencing, of the concentration of at least one signal oligonucleotide.

The token oligonucleotide may comprise from 8 to 50 nucleotides, preferably from 15 to 45 nucleotides, more preferably from 20 to 40 nucleotides, for example from 25 to 35 nucleotides.

The token oligonucleotide preferably comprises, from 5' to 3', a region complementary to a primer for amplification and/or sequencing, optionally, a region comprising an arbitrary sequence to identify the token after sequencing and, optionally, a single-stranded region complementary to the barcoded nucleic acid, as defined below.

The token oligonucleotide is a single-stranded oligonucleotide.

The quantification oligonucleotide converts the presence of at least one signal oligonucleotide at a concentration over or below a threshold value to a token oligonucleotide. The quantification oligonucleotide thus allows determining the concentration of a signal oligonucleotide, via the generation of a token oligonucleotide.

The quantification oligonucleotide may comprise from 16 to 70 nucleotides, preferably from 25 to 55 nucleotides, more preferably from 30 to 50 nucleotides, for example from 35 to 45 nucleotides or from 36 to 42 nucleotides.

The quantification oligonucleotide may or may not be protected against degradation (in particular by restriction enzymes or nucleases).

The quantification oligonucleotide is able to bind to a signal oligonucleotide, as defined above.

The quantification oligonucleotide preferably comprises, from 5' to 3', a first region complementary to the token oligonucleotide, an enzyme recognition site, preferably a nicking enzyme recognition site, optionally a spacer region of arbitrary sequence, and a second region able to bind a signal oligonucleotide.

The quantification oligonucleotide as defined above can be a single-stranded oligonucleotide or a partially double-stranded oligonucleotide.

Upon binding of the quantification oligonucleotide to the signal oligonucleotide and subsequent amplification by a polymerase, a double-stranded quantification oligonucleotide is obtained. Upon action of an enzyme recognizing said enzyme recognition site, preferably a nicking enzyme, a single-stranded oligonucleotide, which is called the token oligonucleotide, is obtained.

The DNA toolbox mixture for use according to the invention for example comprises a conversion oligonucleotide of sequence SEQ ID NO: 5, an amplification oligonucleotide of sequence SEQ ID NO: 2, optionally a leak absorption oligonucleotide of sequence SEQ ID NO: 3 and, optionally, a reporting probe comprising the oligonucleotide of sequence SEQ ID NO: 4. This DNA toolbox mixture is able to produce the signal oligonucleotide of sequence SEQ ID NO: 1 in the presence of an activator oligonucleotide of sequence SEQ ID NO: 6 or of sequence SEQ ID NO: 18.

Another DNA toolbox mixture for use according to the invention for example comprises a conversion oligonucleotide of sequence SEQ ID NO: 7, an amplification oligonucleotide of sequence SEQ ID NO: 2, optionally a leak absorption oligonucleotide of sequence SEQ ID NO: 3 and, optionally, a reporting probe comprising the oligonucleotide of sequence SEQ ID NO: 4. This DNA toolbox mixture is able to produce the signal oligonucleotide of sequence SEQ ID NO: 1 in the presence of an activator oligonucleotide of sequence SEQ ID NO: 10.

Another DNA toolbox mixture for use according to the invention for example comprises a conversion oligonucleotide of sequence SEQ ID NO: 15, an amplification oligonucleotide of sequence SEQ ID NO: 12, optionally a leak absorption oligonucleotide of sequence SEQ ID NO: 13 and, optionally, a reporting probe comprising the oligonucleotide of sequence SEQ ID NO: 14. This DNA toolbox mixture is able to produce the signal oligonucleotide of sequence SEQ ID NO: 11 in the presence of an activator oligonucleotide of sequence SEQ ID NO: 18.

The DNA toolbox mixture as defined above thus preferably comprises:
- at least one polymerase as defined above,
- at least one enzyme selected from a restriction enzyme and/or a nickase as defined above, in particular allowing releasing the signal oligonucleotide from the double-stranded conversion oligonucleotide, releasing the anti-signal oligonucleotide from the double-stranded anti-signal conversion oligonucleotide and/or releasing the signal oligonucleotide from the double-stranded amplification oligonucleotide, and/or releasing the signal oligonucleotide from the reporting template, and/or releasing the barcoded primers from a solid support,
- at least one amplification oligonucleotide as defined above,
- optionally, at least one exonuclease, at least one endonuclease, at least one ligase, at least one recombinase, at least one glycosylase and/or at least one DNA-processing enzyme, as defined above,
- optionally, at least one reverse transcriptase, as defined above,
- optionally, at least one conversion oligonucleotide as defined above or at least one precursor oligonucleotide as defined above,
- optionally, at least one anti-signal conversion oligonucleotide as defined above,
- optionally, at least one leak absorption nucleotide as defined above,
- optionally, at least one quantification oligonucleotide,
- optionally, at least one reporter probe as defined above, and/or
- a set of barcoded primers as defined below.

### Other components

The DNA toolbox mixture also comprises a buffer.

The buffer is adapted to the oligonucleotides present in the DNA toolbox mixture and preferably also to the cells or organelles. In one embodiment, the buffer is particularly suitable to maintain living cells or organelles intact and without inducing any stress to the cells or organelles.

The buffer preferably does not comprise PBS, in particular to avoid inhibition of the reactions.

According to the invention, the buffer comprises low levels of phosphate ions, without inhibiting the reactions. According to the invention, the buffer comprises less than 6 % of PBS that is about 0.7 mM phosphate ions.

More generally, the buffer preferably does not comprise phosphate ions.

The buffer preferably comprises Tris-HCl.

The buffer is preferably suitable for reverse transcription by a reverse transcriptase, particularly when the method is used for sequencing.

The buffer thus preferably further comprises MgSO₄, for example at a concentration from 3 mM to 6 mM.

The buffer may further comprise a cellular medium, in particular when using living cells, for example at a concentration lower than or equal to 50%, preferably lower than or equal to 25%, preferably lower than or equal to 15%, preferably at a concentration lower than or equal to 12,5%, the percentage being expression in v/v.

Any suitable cell culture medium may be used, such as for example a cell culture medium comprising RPMI, FBS (*Fetal Bovine Serum*) and at least one antibiotic (for example penicillin and/or streptomycin).

The buffer as defined above may for example comprise Tris-HCl pH 8.3, KCI, MgCl₂, MgSO₄ and, optionally, a cell culture medium.

For example, a buffer suitable for selecting a subpopulation of living cells may comprise 50 mM Tris HCl pH 8.3, 75 mM KCI, 3 mM MgCl₂, 3 mM MgSO₄ and, optionally, a cell culture medium.

For example, a buffer suitable for selecting a subpopulation of fixed cells may comprise 50 mM Tris HCl pH 8.3, 75 mM KCI, 3 mM MgCl₂, 10 mM DTT and 6 mM MgSO₄.

The DNA toolbox mixture preferably further comprise a dNTP (deoxyribonucleotide triphosphate) mix, i.e. a mix comprising dATP, dTTP, dCTP and dGTP, for example at a concentration of from 25 µM to 500 µM for each dNTP.

The dNTPs are particularly needed for DNA synthesis by the polymerase and/or reverse transcription by the reverse transcriptase.

The DNA toolbox mixture may further comprise netropsin, for example at a concentration of 2 µM.

The DNA toolbox may further comprise a set of barcoded primers, in particular when the method of the invention is used for sequencing.

The set of barcoded primers comprises subsets of barcoded primers, the barcoded primers of one subset being identical, but different from another subset.

When partitioning the DNA toolbox mixture into a plurality of compartments, each compartment thus comprises a subset of barcoded primers specific to this compartment.

Alternatively, the barcoded primers are not provided in the DNA toolbox mixture.

The barcoded nucleic acids may be single-stranded oligonucleotides or double-stranded oligonucleotides.

A barcoded nucleic acid preferably comprises:
- a region complementary to a primer for amplification and/or sequencing,
- optionally, a random sequence used as a unique molecule identifier (UMI),
- optionally, a region comprising the barcode sequence,
- optionally, a single-stranded region complementary to the signal oligonucleotide,
- optionally, a region, preferably in 3', which is able to bind a complementary sequence on an RNA molecule or DNA molecule, or
- optionally, a single-stranded region, preferably in 3', complementary to the token oligonucleotide, and
- optionally, a photo-cleavable linker, a chemically-cleavable linker and/or an enzyme recognition site, preferably located between the solid support and the region which is the same in all the barcoded primers.

The region which is able to bind a complementary sequence on an RNA molecule for example comprises a polyT sequence, which allows binding to the poly(A) tail of the RNAs.

For example, the barcoded nucleic acid as defined above is a barcoded primer, which may comprise:
- a single-stranded region complementary to a primer for amplification and/or sequencing,
- optionally, a single-stranded random sequence used as a unique molecule identifier (UMI),
- a single-stranded region comprising the barcode sequence,
- optionally, a single-stranded region complementary to the signal oligonucleotide,
- a single-stranded region, preferably in 3', which is able to bind a complementary sequence on an RNA molecule or a DNA molecule,
- optionally, a single-stranded region, preferably in 3', complementary to the token oligonucleotide, and
- optionally, a photo-cleavable linker, a chemically-cleavable linker and/or an enzyme recognition site, preferably located between the solid support and the region which is the same in all the barcoded primers.

For example, the barcoded nucleic acid as defined above is a barcoded template switching oligonucleotide, which may comprise:
- a single-stranded region complementary to a primer for amplification and/or sequencing,
- optionally, a single-stranded random sequence used as a unique molecule identifier (UMI),
- optionally, a single-stranded region complementary to the signal oligonucleotide,
   a single-stranded region, preferably in 3', which is able to bind a complementary sequence on an RNA,
- optionally, a single-stranded region, preferably in 3', complementary to the token oligonucleotide, and
- optionally, a photo-cleavable linker, a chemically-cleavable linker and/or an enzyme recognition site, preferably located between the solid support and the region which is the same in all the barcoded primers.

In such case, the barcoded primer is used in combination with a single-stranded oligonucleotide comprising a region comprising the barcode sequence in 5' and a region complementary to a primer for amplification and/or sequencing in 3' and, optionally, a UMI.

For example, the barcoded nucleic acid as defined above is a barcoded adapter, which may comprise:
- a double-stranded region complementary to a primer for amplification and/or sequencing,
- optionally, a double-stranded random sequence used as a unique molecule identifier (UMI),
- a double-stranded region comprising the barcode sequence, and
- optionally, a photo-cleavable linker, a chemically-cleavable linker and/or an enzyme recognition site, preferably located between the solid support and the region which is the same in all the barcoded primers.

For example, the barcoded nucleic as defined above is barcoded recombination site, which may comprise:
- a double-stranded Mosaic End Sequence,
- a region complementary to a primer for amplification and/or sequencing,
- optionally, a random sequence used as a unique molecule identifier (UMI),
- a region comprising the barcode sequence, and
- optionally, a photo-cleavable linker, a chemically-cleavable linker and/or an enzyme recognition site, preferably located between the solid support and the region which is the same in all the barcoded primers.

The barcoded primers are preferably attached to a solid support, such as a bead or a DNA microarray.

When the solid support is a DNA microarray, the barcode encodes the position of the barcoded primers on the solid support, for example the position of a compartment. Each compartment of the DNA microarray comprises a subset of identical barcoded primers, which is different from the subsets of the other compartments.

When the solid support is a bead, each solid bead bears a subset of identical barcoded primers, which is different from the subsets of the other beads.

### Method for selecting a subpopulation of cells or organelles

The present invention relates to a method for the selective analysis of a subpopulation of cells or organelles based on determining the level of at least one cell or organelle marker.

Said method for example allows selecting a subpopulation of cells or organelles having a phenotype of interest, detecting the presence of specific proteins in the subpopulation of cells or organelles and/or sequencing the genomic DNA or RNA of this subpopulation of cells.

The "cells or organelles" and the "cell or organelle marker" are particularly as defined above.

The level of at least one cell or organelle marker is determined by the concentration of at least one signal oligonucleotide, which production depends on the level of said at least one cell or organelle marker.

The present invention thus relates to a method for the selective analysis of a subpopulation of cells or organelles based on determining the level of at least one cell or organelle marker, said method comprising:
a) partitioning said cells or organelles into a plurality of compartments, so that the majority of the compartments do not comprise more than a single cell of one cellular type or a single organelle of one cellular type,
   together with at least one cell or organelle marker-specific ligand coupled to at least one activator oligonucleotide or repressor oligonucleotide and
   with a DNA toolbox mixture comprising at least one polymerase, at least one restriction or nicking enzyme, at least one amplification oligonucleotide able to bind and amplify a signal oligonucleotide, and a buffer comprising less than 0.7 mM phosphate ions,
b) in each compartment, letting the activator oligonucleotide(s) and/or the repressor oligonucleotide(s) bound to the cell or organelle marker, interact together and with the components of the DNA toolbox mixture, to generate one or more signal oligonucleotides and/or one or more anti-signal oligonucleotides and to amplify said signal oligonucleotide(s),
c) analyzing the cells or organelles of the compartments in which at least one signal oligonucleotide is present at a concentration over or below a threshold value.

### Step a)

Step a comprises partitioning said cells or organelles into a plurality of compartments, so that the majority of the compartments do not comprise more than a single cell of one cellular type or a single organelle of one cellular type, together with at least one cell or organelle marker-specific ligand coupled to at least one activator oligonucleotide or repressor oligonucleotide and with a DNA toolbox mixture comprising at least one polymerase, at least one restriction or nicking enzyme, at least one amplification oligonucleotide able to bind and amplify a signal oligonucleotide, and a buffer comprising less than 0.7 mM phosphate ions.

The compartment may for example be a microdroplet, a microcompartment, a microchamber or a cage.

The "activator oligonucleotide", "repressor oligonucleotide", "DNA toolbox mixture", "polymerase", "restriction or nicking enzyme" and "amplification oligonucleotide able to bind and amplify a signal oligonucleotide" are particularly as defined above.

By the expression "the majority of the compartments", it is herein meant at least 50% of the compartments, preferably at least 70% of the compartments, more preferably at least 75% of the compartments, and even more preferably at least 80% of the compartments.

The sentence "a compartment does not comprise more than a single cell of one cellular type or a single organelle of one cellular type" means that if the compartment comprises at least two cells, each of said cells are of a different cellular type, or if the compartment comprises at least two organelles, each of said organelles are of a different cellular type.

In step a), the cells or organelles may be provided in the form of a mixture comprising said cells or organelles, said at least one cell or organelle marker-specific ligand and/or said DNA toolbox mixture or they may be provided separately from said at least one cell or organelle marker-specific ligand(s) and/or from the DNA toolbox mixture.

The cell or organelle marker is preferably selected from the group consisting of a surface marker, an internal marker, a secreted or released marker and a membrane marker.

When at least two cell or organelle marker-specific ligands are used, they can bind two different types of markers as defined above.

When the cell or organelle marker-specific ligand is a membrane anchor, the amount of activator or repressor oligonucleotide(s) present in the compartment in step b) is proportional to the surface of the membrane of the enclosed cell(s), so that the concentration of the signal oligonucleotide(s) in step c) is a function of the presence or absence of a compartmentalized cell and/or of the number of compartmentalized cell(s) and/or of the size of the compartmentalized cell(s).

In one embodiment wherein the marker is not a secreted or released marker, the method as defined above may comprise, before step a):
- mixing said cells or organelles with at least one cell or organelle marker-specific ligand coupled to an activator or repressor oligonucleotide, and
- optionally, washing the cells or organelles to remove unbound cell or organelle marker-specific ligands coupled to an activator or repressor oligonucleotide.

In step a), the DNA toolbox mixture may further comprise at least one enzyme selected from the group consisting an exonuclease, an endonuclease, a reverse transcriptase, a ligase, a recombinase, a glycosylase and a DNA-processing enzyme.

When the activator oligonucleotide is a signal oligonucleotide itself, no corresponding conversion oligonucleotide nor precursor oligonucleotide is needed in the DNA toolbox mixture. The DNA toolbox mixture may thus comprise an amplification oligonucleotide corresponding to said signal oligonucleotide and, optionally, a leak-absorption oligonucleotide corresponding to said signal oligonucleotide.

When the activator or repressor oligonucleotide is a precursor oligonucleotide, the DNA toolbox mixture preferably comprises (i) a conversion oligonucleotide or an anti-signal conversion oligonucleotide corresponding to said precursor oligonucleotide, (ii) optionally, an anti-signal oligonucleotide corresponding to said precursor oligonucleotide, (iii) an amplification oligonucleotide corresponding to the signal oligonucleotide produced or deactivated from said conversion oligonucleotide or anti-signal conversion oligonucleotide (via the anti-signal oligonucleotide it produces), respectively and (iv) optionally, a leak-absorption oligonucleotide corresponding to said signal oligonucleotide.

When the activator or repressor oligonucleotide is a conversion oligonucleotide or an anti-signal conversion oligonucleotide, the DNA toolbox mixture preferably comprises a precursor oligonucleotide corresponding to said conversion oligonucleotide or anti-signal conversion oligonucleotide, an amplification oligonucleotide corresponding to the signal oligonucleotide produced or deactivated from said conversion oligonucleotide or anti-signal conversion oligonucleotide (via the anti-signal oligonucleotide it produces), respectively and, optionally, a leak-absorption oligonucleotide corresponding to said signal oligonucleotide.

When the activator oligonucleotide is a conversion or anti-signal conversion oligonucleotide fused to the corresponding precursor oligonucleotide, no conversion/anti-signal conversion oligonucleotide nor precursor oligonucleotide is needed in the DNA toolbox mixture. The DNA toolbox mixture thus comprises an amplification oligonucleotide corresponding to the signal oligonucleotide produced or deactivated from said conversion oligonucleotide or anti-signal conversion oligonucleotide (via the anti-signal oligonucleotide it produces), respectively and, optionally, a leak-absorption oligonucleotide corresponding to said signal oligonucleotide.

In one embodiment, the activator oligonucleotide coupled to the cell or organelle marker-specific ligand generates one or more signal oligonucleotide by acting as template and/or as a primer for the polymerase, and/or the repressor oligonucleotide coupled to the cell or organelle marker-specific ligand generates one or more anti-signal oligonucleotide by acting as a template and/or as a primer for the polymerase.

In one embodiment, the cell or organelle marker-specific ligands coupled to at least one activator oligonucleotide or repressor oligonucleotide may comprise at least one pair of ligands, said pair of ligands comprising a first cell or organelle marker-specific ligand and a second cell or organelle marker-specific ligand, and wherein the binding of said first and second ligands to their marker induces proximity extension or proximity ligation between the activator or repressor oligonucleotide coupled to said first ligand and the activator or repressor oligonucleotide coupled to said second ligand, said proximity extension or proximity ligation leading to the generation of one or more signal oligonucleotides or one or more anti-signal oligonucleotide(s) and to amplification of said signal oligonucleotide(s).

A pair of ligands is particularly used when using a secreted or released marker, or to remove the need for washing, or to limit the number of wash steps, or to study the presence of a plurality of markers on the same cell or organelle.

A pair of ligands may for example be used to target one cell surface marker (using for example only one ligand, half of said ligand molecules being tagged with a first activator or repressor oligonucleotide and half of said ligand molecules being tagged with a second one activator oligonucleotide or repressor oligonucleotide) or two cell surface markers (using two different ligands specific to two different surface markers, each tagged with one activator oligonucleotide or repressor oligonucleotide).

The marker-specific ligands preferably comprise at least one pair of ligands as defined above, the first and second ligands of said pair being specific of a secreted or released marker, and the first and second ligands of said pair can thus bind simultaneously to said secreted marker, thereby inducing a proximity extension or ligation between the activator or repressor oligonucleotide coupled to said first ligand and the activator or repressor oligonucleotide coupled to said second ligand, leading to the generation of one or more signal oligonucleotides or one or more anti-signal oligonucleotide(s) and to amplification of said signal oligonucleotide(s).

Proximity extension and proximity ligation are well known by the skilled person, such as for example in Gullberg et al., 2004, PNAS, 101 (22) (doi.org/10.1073/pnas.0400552101), Fredriksson et al., 2002, Nature Biotechnology vol 20, p473-477 (https://doi.org/10.1038/nbt0502-473), Di Giusto et al., 2005, Nucleic Acids Research, Vol 33(6), p e64 (https://doi.org/10.1093/nar/gni063) or Lundberg, 2011, Nucleic Acids Research, Vol 39(15), p e102, (doi.org/10.1093/nar/gkr424).

The binding of two ligands of a pair to a marker present on the same cell or organelle increases the generation rate of one or more signal oligonucleotide or one or more anti-signal oligonucleotide(s) compared to the effect of the unbound oligonucleotides, so that washing steps prior to step a) can be decreased or suppressed.

The DNA toolbox mixture preferably comprises one or more leak-absorption oligonucleotides able to bind the signal oligonucleotides.

The leak-absorption oligonucleotides are particularly as defined above.

The leak-absorption oligonucleotide allows false positives to be avoided and/or prevents signal amplification if the stimulation generated by the activator oligonucleotide(s) is below a threshold.

Leak absorption oligonucleotides that may be used are for example described in document WO2017141067 or in Montagne et al., 2016, Nat Commun 7, 13474 (doi.org/10.1038/ncomms13474).

A leak-absorption oligonucleotide is specific of a signal oligonucleotide.

Thus, when at least two signal oligonucleotides are generated and when leak-absorption oligonucleotides are present in the DNA toolbox mixture, each leak-absorption oligonucleotide is specific of one signal oligonucleotide.

When the activator or repressor oligonucleotide is a source template as defined above, the method preferably comprises, before step a):
- mixing said cells or organelles with at least one cell or organelle marker-specific ligand coupled to an activator or repressor oligonucleotide, and
- washing the cells or organelles to remove unbound cell or organelle marker-specific ligands coupled to an activator or repressor oligonucleotide.

### Step b)

Step b) comprises, in each compartment, letting the activator oligonucleotide(s) and/or the repressor oligonucleotide(s) bound to the cell or organelle marker, interact together and with the components of the DNA toolbox mixture, to generate one or more signal oligonucleotides and/or one or more anti-signal oligonucleotides and to amplify said signal oligonucleotide(s),

The expression "to generate one or more signal oligonucleotides" means that one type of signal oligonucleotide or at least two different types of signal oligonucleotide is/are generated.

The expression "to generate one or more anti-signal oligonucleotides" means that one type of signal oligonucleotide or at least two different types of signal oligonucleotide is/are generated.

In step b), only the signal oligonucleotides are amplified. The anti-signal oligonucleotides are not amplified.

Step b) may be performed at a temperature comprised from 20°C to 65°C, preferably from 20°C to 55°C, more preferably from 20°C to 45°C, for example from 35°C to 40°C.

### Step c)

Step c) comprises analyzing the cells or organelles of the compartments in which at least one signal oligonucleotide is present at a concentration over or below a threshold value.

In step c), when at least two different types of signal oligonucleotides can be generated in step b), the cells or organelles in the compartment are analyzed when each signal oligonucleotide is present at a concentration over or below its corresponding threshold value. The thresholds are indeed preferably set independently for each signal oligonucleotide.

Step c) as defined above may comprises analyzing the cells or organelles of the compartments in which each signal oligonucleotide or a combination of signal oligonucleotides is present at a concentration over or below its corresponding threshold value.

In one embodiment, the level of at least one cell or organelle marker may be modified by the interaction between at least two co-compartmentalized cells or organelles and step c) allows analysis of the interaction between the cells or organelles.

The generation of one or more signal oligonucleotides and one or more anti-signal oligonucleotides, followed by the amplification of the signal oligonucleotide(s), can lead to the amplification of a new signal oligonucleotide, which is called master signal oligonucleotide, wherein the sequence of the master signal oligonucleotide is not related to that of any signal oligonucleotide directly produced from the activator oligonucleotide(s).

In one embodiment, the signal oligonucleotide is thus a master signal oligonucleotide produced according to at least two different signal oligonucleotides.

In such case, step b) as defined above may allow integration of inputs from multiple activator or repressor oligonucleotides to create a single output in the form of a master signal oligonucleotide.

Step c) as defined above may comprise determining the concentration of a signal oligonucleotide by adding a reporter probe, wherein said reporter probe is specifically activated by said signal oligonucleotide, and detecting a signal emitted by the reporter probe bound to said signal oligonucleotide.

The reporter probe may be as defined above.

The reporter probe is preferably an optical reporter probe, even more preferably a fluorogenic reporter probe.

The term "activated" herein means that the interaction between the reporter probe and the specific signal oligonucleotide leads to a change in the fluorescence intensity of the reporter probe.

The interaction may include simple hybridization, strand displacement or hybridization followed by enzymatic activity, such as polymerase extension and/or restriction enzyme digestion.

Step c) as defined above may thus comprise selectively analyzing the cells or organelles of the compartments wherein the signal(s) emitted by the reporter probes(s) is/are above or below a threshold value.

Step c) as defined above may comprise recovering the contents of the compartments in which at least one signal oligonucleotide is present at a concentration over or below a threshold value.

Step c) as defined above may comprise determining the concentration of at least one signal oligonucleotide by adding a quantification oligonucleotide, wherein said quantification oligonucleotide is specifically amplified when said at least one signal oligonucleotide is present at a concentration over or below a threshold value.

The quantification oligonucleotide is particularly as defined above.

Said amplification of the quantification oligonucleotide preferably consists of a linear amplification, more preferably using at least one polymerase and at least one restriction or nicking enzyme.

The quantification oligonucleotide(s) located in a same compartment is/are associated with a compartment-specific barcode sequence.

The quantification oligonucleotide preferably comprises a compartment-specific barcode sequence further to extension of a cDNA on a barcoded primer, extension of a cDNA on a barcoded template switching oligonucleotide, ligation of a barcoded adapter, or recombination with a barcoded recombination site.

Said recovered contents may then be analyzed by sequencing cellular or organellar nucleic acids and/or labeling nucleic acid sequence(s).

By "labeling nucleic acids", it is herein meant nucleic acids bound to cells or organelles via ligands binding to cellular or organellar markers, or molecules reacting with cellular or organellar markers, wherein the sequence of said nucleic acid is specific to the ligand or reacting molecule. In a preferred embodiment, the labeling nucleic acid is associated with an antibody binding to a cellular or organellar marker.

Step c) as defined above may comprise recovering the cells of the compartments in which at least one signal oligonucleotide is present at a concentration over or below a threshold value and cultivating the cells prior to further analysis.

In one preferred embodiment, the cellular or organellar nucleic acids and/or labeling nucleic acid sequence(s) located in a same compartment are associated with a compartment-specific barcode sequence.

The cellular or organellar nucleic acids and/or labeling nucleic acid may for example comprise a compartment-specific barcode sequence further to extension of a cDNA on a barcoded primer, extension of a cDNA on a barcoded template switching oligonucleotide, ligation of a barcoded adapter, or recombination with a barcoded recombination site.

In a preferred method as defined above, accumulation of the signal oligonucleotide, for example of the master signal oligonucleotide, results in the selective sequencing of cellular or organellar nucleic acids and/or labeling nucleic-acid sequence(s) located in the same compartment.

The cells may be lysed at the end of step b), in particular after accumulation of the signal oligonucleotide.

The barcoded primer, the barcoded template-switching oligonucleotide, the barcoded adaptor or the barcoded recombination site as defined above may comprise at least one photolabile protecting group and, in step c), for the compartments wherein the signal(s) from the reporter probe(s) is above or below a threshold value, photodeprotection of said barcoded primer, barcoded template-switching oligonucleotide, barcoded adaptor or barcoded recombination site occurs, thereby allowing the selective association of the cellular or organellar nucleic acids and/or labeling nucleic acids with a compartment-specific barcode sequence.

The barcoded primer, the barcoded template-switching oligonucleotide, the barcoded adaptor or the barcoded recombination site as defined above may comprise photocleavable linkage and, in step c), for the compartments wherein the signal(s) from the reporter probe(s) is above or below a threshold value, photocleavage of said barcoded primer sequence, barcoded template-switching oligonucleotide, barcoded adaptor or barcoded recombination site occurs, thereby allowing the selective release of the cellular or organellar nucleic acids and/or labeling nucleic acid comprising primer sequences, template-switching oligo sequences, adapter sequences or recombination sites and a compartment-specific sequence barcode from a solid support.

In step c) as defined above, the binding, and optionally extension, of the signal oligonucleotide may result in the selective release of the cellular or organellar nucleic acids and/or labeling nucleic acids comprising a barcoded primer, a barcoded template-switching oligonucleotide, a barcoded adaptor or a barcoded recombination site and a compartment-specific sequence barcode from a solid support.

In one embodiment, binding, and optionally extension, of the signal oligonucleotide, results in the creation of a recognition site for a restriction enzyme or an endonuclease and release from the solid support is by cleavage with the corresponding restriction enzyme or endonuclease.

In another embodiment, binding and extension of the signal oligonucleotide by a polymerase with strand displacement activity, results in strand invasion and release from the solid support.

The expression "strand invasion" herein means that a nucleic acid to be released is attached to a support by hybridization to a surface tethered oligonucleotide, wherein said surface tethered oligonucleotide comprises a signal oligonucleotide binding domain and that polymerase extension of the signal oligonucleotide on the tethered oligonucleotide (thus acting as template) strand-displaces the nucleic acid to be released.

When the solid support is a bead, each compartment preferably comprises no more than a single bead, and the barcoded primers, the barcoded template-switching oligonucleotides, the barcoded adaptors or the barcoded recombination sites carry a barcode sequence specific to the bead of said compartment.

When the solid support is the wall of a microcompartment or cage, the barcoded primers, barcoded template-switching oligonucleotides, barcoded adaptors or barcoded recombination sites preferably carry a compartment-specific barcode sequence.

The wall of the microcompartment or cage may be the top, the bottom or the sides of the microcompartment or cage.

In one embodiment,
- the DNA toolbox mixture in step a) comprises a polymerase with reverse transcriptase activity,
- each compartment in step a) comprises oligonucleotides carrying a compartment-specific barcode sequence and a region able to bind RNA, wherein said oligonucleotides are attached to a solid support,
- in step c), the cells or organelles are lysed thereby releasing RNA and the oligonucleotides carrying a compartment-specific barcode sequence and a region able to bind RNA are used to prime cDNA synthesis, catalysed by a polymerase with reverse transcriptase activity, on the released RNA, resulting in cDNA carrying the compartment-specific barcode,
- in step c), the cDNA carrying the compartment-specific barcode is released from the solid support when at least a signal oligonucleotide is present at a concentration over or below a threshold value and the released barcoded cDNAs and/or the non-released barcoded cDNAs are analyzed by sequencing, and
- optionally, in step c), token oligonucleotides are hybridized to compartment-specific barcode, and optionally are used to prime DNA synthesis, catalysed by a DNA polymerase, resulting in DNA carrying the compartment-specific barcode.

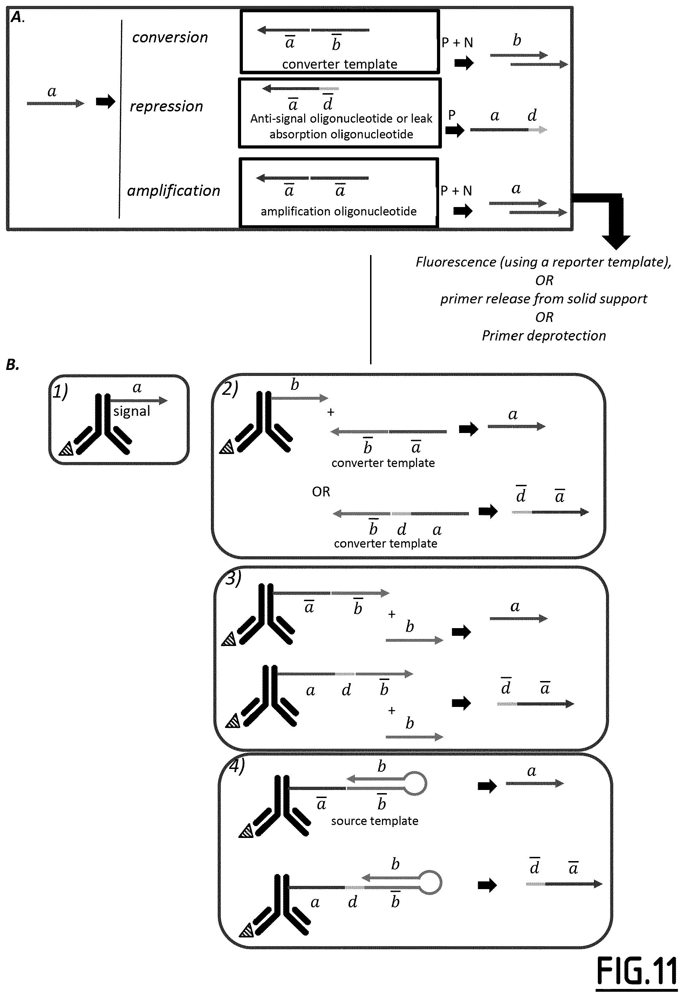

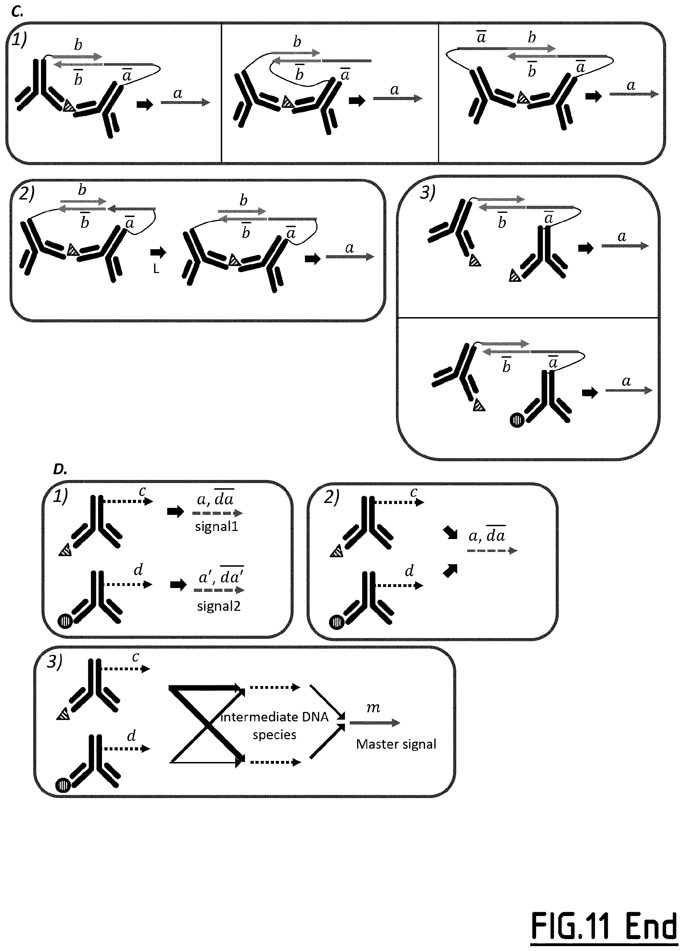

Non-limitative embodiments of the methods of the inventions are illustrated in Figures 11 to 14.

**Figure 11** illustrates different embodiments relating to DNA toolbox components, surface markers, proximity effects for secreted or surface markers and combination of markers.

**Figure 11A** illustrates DNA toolbox components. The combination of enzymes and oligonucleotides present in the DNA toolbox (in solution) can be used to perform various functions on incoming oligonucleotides (here a): conversion (for example from oligonucleotide a to oligonucleotide b) is performed by a conversion template (also called conversion oligonucleotide), under the action of a polymerase (P) and a nickase (N); repression is performed by an anti-signal oligonucleotide or a leak-absorption oligonucleotide, under the action of a polymerase (P), by adding a 3' tail to the incoming oligonucleotide (for example here converting a to ad); amplification is performed by an amplification template, under the action of a polymerase (P) and a nickase (N), for example by producing two copies of a starting from one copy of a. Depending on the balance between conversion/amplification and repression effect, an oligonucleotide can be amplified or not. The amplified oligonucleotide can be used for various purposes for example to produce a fluorescent readout using a reporter template, to release primers from a solid support, or to deprotect primers.

**Figure 11B** illustrates coupling oligonucleotide-conjugated marker-specific ligands to DNA toolbox. In 1), the 3' part of the antibody-conjugated activator oligonucleotide *a* primes directly on the amplification template; In 2), the activator or repressor oligonucleotide *b*, in combination with a cognate converter template (also called conversion oligonucleotide) (in solution) produces the signal *a* or anti-signal oligonucleotide *̅d̅*̅a̅. In 3), the converter template (*b̅a̅* or *b̅da*) can be attached on the marker specific ligand (antibody) and the corresponding precursor oligonucleotide (b) provided in solution. As before, this architecture can produce either the signal oligonucleotide *a* or anti-signal oligonucleotide *̅d̅*̅a̅ depending on the converter template's output. 4) The activator or repressor oligonucleotide can be a converter template (also called conversion oligonucleotide) that is already primed for polymerisation. This design is called a source template. The source template can be a stable duplex (not shown), or the priming part (*b*) can be covalently fused to the template part (e.g. with a DNA loop or a non-DNA linker). This self-primed template constitutively produces the signal oligonucleotide *a* or anti-signal oligonucleotide *d̅a̅* (depending on the source template's output).

**Figure 11C** illustrates proximity effects for secreted or surface markers. In 1), proximity extension design: 2 marker-specific ligands are functionalized with activator oligonucleotides. When brought in close proximity by the binding on the soluble target, the interaction between the 2 activator oligonucleotides induces the production of signal oligonucleotide *a* (or anti-signal *̅d̅*̅a̅ in case of a repressor oligonucleotide). Various geometries are possible (from left to right): one ligand is coupled to the 5' end of an activator oligonucleotide (b) and the other to the 5' end of a conversion oligonucleotide (*b̅a̅*); Same, but the conversion oligonucleotide is coupled via its 3' end; both marker specific ligands are coupled to the 5' end of conversion templates with cross-complementary 3' extremities, and thus able to prime each other. 2) In the proximity-ligation design, the target-induced proximity between two marker specific ligand-bound activator oligonucleotides enables a ligation reaction (L), which creates a conversion oligonucleotide (here, primed by b). 3) The proximity effect can also be induced by the binding of the two marker specific ligands, not to the same target, but to the same cell; the two target markers can be chemically identical (up), or chemically different (down).

**Figure 11D** illustrates combination of markers. For the detection of specific combinations of markers (i.e. the triggering of the amplification if the respective levels of multiple markers fit particular conditions). Here, in all cases 1 to 3, examples are shown with two markers, but the same principle applies for 3 or more markers. 1) The two ligand-bound activator or repressor oligonucleotides act in parallel to activate or repress specifically multiple signal oligonucleotides. As in panel B, each ligand-bound activator or repressor oligonucleotide can be a signal (*a)*, a conversion oligonucleotide (*b̅a̅* or *b̅da*) or (*a̅b̅b or ad̅b̅b*), etc, as shown in panel B. 2) The two ligand-bound activator or repressor oligonucleotides produce the same signal and/or anti-signal oligonucleotide and the balance between the two determines if the signal oligonucleotide is eventually amplified. 3) More sophisticated biomolecular architectures, where activator or repressor oligonucleotides and DNA toolbox components produce intermediate species that eventually induce the amplification of a master signal *m*, can be designed to select cells or organelles that display more complex marker patterns.

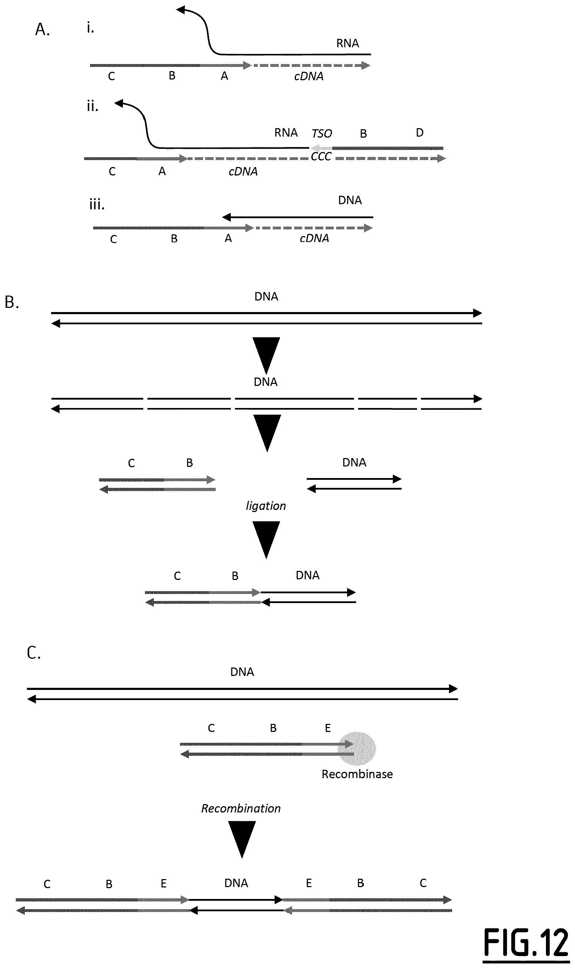

**Figure 12** illustrates single-cell barcoding.

In **Figure 12** **A,** the combination of enzymes and oligonucleotides present in a compartment containing a single cell, organelle or a determined number of cells or organelles can be used to perform barcoding of single-stranded sequences of interest via primer hybridisation.

In (i), a nucleic acid comprises a single-stranded sequence "A" that hybridises to a complementary sequence on an RNA molecule, a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). Hybridisation of the primer to the RNA and extension of the primer leads to production of a cDNA carrying a PCR primer site for amplification and/or sequencing, a compartment-specific barcode and, optionally, a UMI.

In (ii), a first nucleic acid comprises a single-stranded sequence "A" that hybridises to a complementary sequence on an RNA molecule and a sequence "C" complementary to a primer for amplification and/or sequencing. A second nucleic acid comprises a sequence (preferably rGrGrG) complementary to the non-templated "CCC" 3'-overhang appended by the reverse transcriptase at the end of the full-length cDNA, a sequence "B" that comprises a compartment-specific barcode sequence for identification of the nucleic acids present in the same compartment, a sequence "D" complementary to a primer for amplification and/or sequencing and, optionally, a UMI. Hybridisation of the first nucleic acid to the RNA and extension of the primer leads to production of a first strand cDNA molecule with a non-templated "CCC" 3'-overhang. Hybridisation of the second nucleic acid to the "CCC" 3'-overhang, followed by "template switching" of the reverse transcriptase results in the generation of a cDNA carrying PCR primer sites for amplification and/or sequencing, a compartment-specific barcode and, optionally, a UMI.

In (iii), a nucleic acid comprises a single-stranded sequence "A" that hybridises to a complementary sequence on a DNA molecule, a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). Hybridisation of the primer to the DNA and extension of the primer leads to production of a DNA carrying a PCR primer site for amplification and/or sequencing, a compartment-specific barcode and, optionally, a UMI.

In **Figure 12B****,** the combination of enzymes and oligonucleotides present in a compartment containing a single cell, organelle or a determined number of cells or organelles can be used to perform barcoding via adapter ligation. In a compartment, DNA from a cell or organelle is fragmented to generate double-stranded DNA fragments with either sticky or blunt ends. DNA adapter molecules compatible with ligation the double-stranded DNA fragments (via blunt-end sticky-end ligation), containing a sequence "B" that comprises a compartment-specific barcode sequence for identification of the nucleic acids present in the same compartment, and a sequence "C" complementary to a primer for amplification and/or sequencing and, optionally, a random sequence used as a Unique Molecule Identifier (UMI) are present in the same compartment. Ligation of the fragmented DNA from the cell to the adapter leads to the production of a DNA carrying a PCR primer site for amplification and/or sequencing, a compartment-specific barcode and, optionally, a UMI.

In **figure 12C****,** the combination of enzymes and oligonucleotides present in a compartment containing a single cell, organelle or a determined number of cells or organelles can be used to perform barcoding of double-stranded nucleic acids of interest via recombination. Recombinases (e.g. Tn5 transposase) are loaded with nucleic acids comprising a double-stranded Mosaic End Sequence "E", a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). Recombination results in simultaneous fragmentations and barcoding of double-stranded nucleic acids from the cell or organelle ("tagmentation").

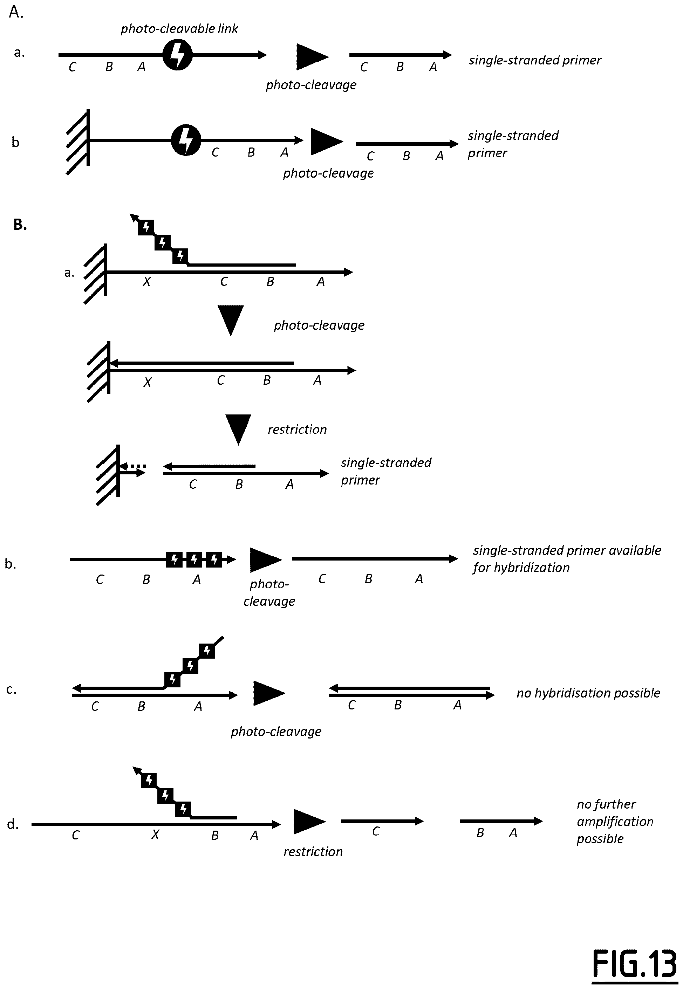

**Figure 13** illustrates photo-cleavage mediated selection following fluorescent readout, using a fluorogenic reporter template.

In **Figure 13A****,** the amplified signal oligonucleotide of the DNA toolbox can be used to produce a fluorescent readout using a reporter template. The information given by the detection of a fluorescent signal in a compartment is used to trigger via photo-cleavable spacer deprotection (a.) or release from a solid support (b.) by selective UV irradiation of the compartment of interest. The released nucleic acid comprises a single-stranded sequence "A" that hybridises to a complementary sequence on an RNA or DNA molecule, a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). The structure of the released nucleic acid can be adapted to allow barcoding of cellular nucleic acid by ligation and recombination (see figure 12). In this case, the released nucleic acid comprises only sequences "B" and "C" and functions as an adapter that is ligated to fragmented cellular DNA or additionally comprises a double-stranded Mosaic End Sequence "E" to allow recombination with cellular nucleic acids.

In **figure 13B****,** the amplified signal oligonucleotide of the DNA toolbox can be used to produce a fluorescent readout using a reporter template. The information given by the detection of a fluorescent signal in a compartment is used to trigger, by selective UV irradiation of photo-caged nucleobases in the compartment of interest, hybridisation, to form a stretch of double-stranded DNA containing a restriction site "X" for an endonuclease to release nucleic acids from a solid support (a.). Alternatively, the UV irradiation of photo-caged nucleobases can trigger de-protection of the single-stranded sequence "A" that hybridises to a complementary sequence on an RNA or DNA molecule (b.), or protection via hybridisation to block hybridisation to single-stranded DNA or RNA (c.). Alternatively, the UV irradiation of photo-caged nucleobases can trigger de-protection of the barcoded primer to prevent amplification or sequencing by separating, a sequence "C" complementary to a primer for amplification and/or sequencing and, optionally, a random sequence used as a Unique Molecule Identifier (UMI), and a single-stranded sequence "A" that hybridises to a complementary sequence on an RNA molecule (d.). The released nucleic acid comprises a single-stranded sequence "A" that hybridises to a complementary sequence on an RNA or DNA molecule, a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). The structure of the released nucleic acid can be adapted to allow barcoding of cellular nucleic acid by ligation and recombination (see figure 12). In this case, the released nucleic acid comprises only sequences "B" and "C" and functions as an adapter that is ligated to fragmented cellular DNA or additionally comprises a double-stranded Mosaic End Sequence "E" to allow recombination with cellular nucleic acids.

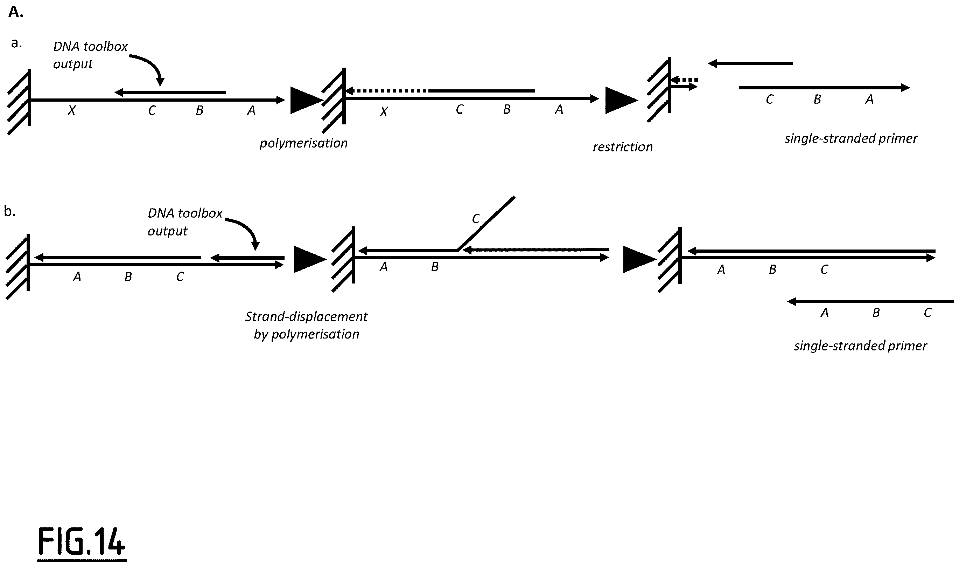

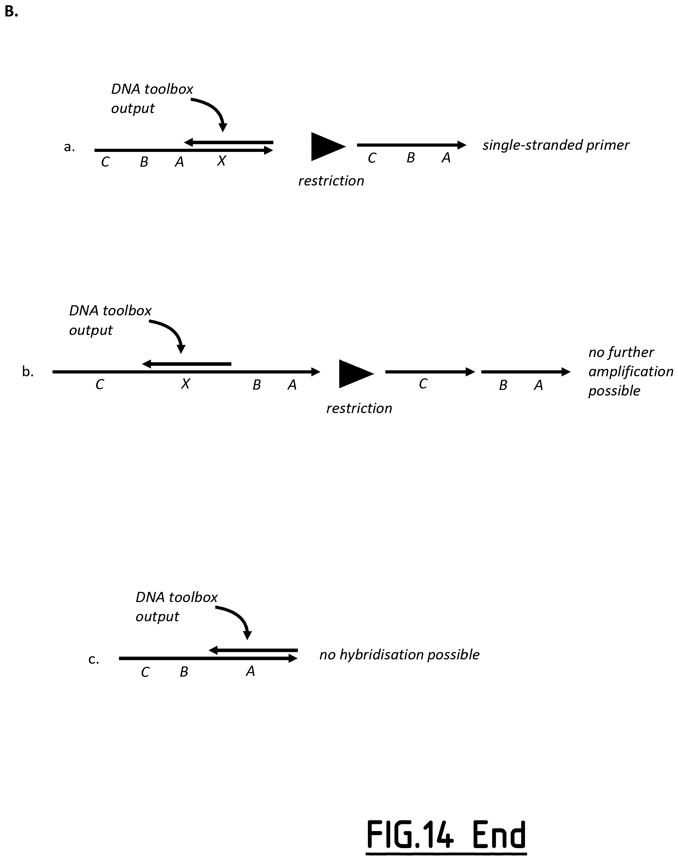

**Figure 14** illustrates DNA toolbox-based direct selection.

In **Figure 14A****,** the amplified signal oligonucleotide of the DNA toolbox, when hybridised, can initiate polymerisation to form a stretch of double-stranded DNA containing a restriction site "X" for an endonuclease to release nucleic acids from a solid support (a.) or, if a polymerase with strand-displacement activity is used, release single-stranded nucleic acids previously hybridised on nucleic acids grafted on a solid support by strand-displacement (b.). The released nucleic acid comprises a single-stranded sequence "A" that hybridises to a complementary sequence on an RNA or DNA molecule, a sequence "B" that comprises a compartment-specific barcode sequence for identification of the nucleic acids present in the same compartment, a sequence "C" complementary to a primer for amplification and/or sequencing and, optionally, a random sequence used as a Unique Molecule Identifier (UMI). The structure of the released nucleic acid can be adapted to allow barcoding of cellular nucleic acids by ligation and recombination (see figure 12). In this case, the released nucleic acid comprises only sequences "B" and "C" and functions as an adapter that is ligated to fragmented cellular DNA or additionally comprised a double-stranded Mosaic End Sequence "E" to allow recombination with cellular nucleic acids.

In **Figure 14B****,** the amplified signal oligonucleotide of the DNA toolbox, when hybridised, can form a stretch of double-stranded DNA. This double-stranded DNA stretch contains a restriction site "X" for an endonuclease to de-protect the barcoded primer (a.). Alternatively, double-stranded DNA contains a restriction site "X" for an endonuclease to de-protect the barcoded primer to prevent amplification or sequencing by separating, a sequence "C" complementary to a primer for amplification and/or sequencing and, optionally, a random sequence used as a Unique Molecule Identifier (UMI), and a single-stranded sequence "A" that hybridises to a complementary sequence on an RNA molecule (b.). Alternatively, the hybridisation of the amplified signal oligonucleotide of the DNA toolbox directly blocks hybridisation to single-stranded DNA or RNA (c.). This protocol can be adapted to allow barcoding of cellular nucleic acid by ligation and recombination (see figure 12). In this case, the released nucleic acid comprises only sequences "B" and "C" and functions as an adapter that is ligated to fragmented cellular DNA or additionally comprises a double-stranded Mosaic End Sequence "E" to allow recombination with cellular nucleic acids.

### Kit for implementing the claimed method and use thereof

The present invention also relates to a kit for implementing the method for the selective analysis of a subpopulation of cells or organelles based on determining the level of at least one cell or organelle marker, as defined above.

The kit as defined above for example comprises:
a) at least one polymerase, at least one nicking enzyme or restriction enzyme, and optionally at least one enzyme selected from the group consisting of an exonuclease, an endonuclease, a reverse transcriptase, a ligase, a recombinase, a glycosylase and a DNA-processing enzyme,
b) at least one cell or organelle marker-specific ligand coupled to at least one activator or repressor oligonucleotide,
c) at least one amplification oligonucleotide able to bind and amplify a signal oligonucleotide, and, optionally, at least one oligonucleotide selected from the group consisting of a leak-absorption oligonucleotide to avoid nonspecific amplification, a conversion oligonucleotide able to convert the activator or repressor oligonucleotide into a signal or anti-signal oligonucleotide, a reporting probe allowing detection of a signal oligonucleotide and a quantification oligonucleotide allowing determining the concentration of a signal oligonucleotide,
d) a set of barcoded nucleic acids comprising a a barcoded primer, a barcoded template switching oligonucleotide, a barcoded adapter or a barcoded recombination site, and
e) optionally, a microfluidic device.

The enzymes of a), the cell or organelle marker-specific ligand coupled to at least one activator or repressor oligonucleotide, the oligonucleotides of b) and the set of barcoded nucleic acids are particularly as defined above.

The barcoded nucleic acids are preferably attached to a solid support, such as a bead or to the wall of a microcompartment or cage.

The barcoded primers preferably comprise a sequence able to bind to the signal oligonucleotide produced by the components of the DNA toolbox mixture, a phot-cleavable, enzymatic-cleavable or chemically-cleavable region and a region able to bind to RNA.

The kit as defined above may comprise in b) at least one pair of cell or organelle marker-specific ligands, wherein said pair of ligands comprises a first cell or organelle marker-specific ligand and a second cell or organelle marker-specific ligand, wherein the binding of said first and second ligands to their marker induces a proximity extension or proximity ligation, in particular as defined above, between the activator or repressor oligonucleotide coupled to said first ligand and the activator or repressor oligonucleotide coupled to said second ligand.

The kit may further comprise a partitioning agent, preferably a continuous hydrophobic phase, such as undecane-1-ol, silicon oil, mineral oil and/or perfluorinated oil.

The present invention also relates to the use of the kit as defined above for the selective analysis of a subpopulation of cells or organelles, in particular based on determining the level of at least one cell or organelle marker.

The present invention particularly relates to the use of the kit as defined above in the method for the selective analysis of a subpopulation of cells or organelles based on determining the level of at least one cell or organelle marker, as defined above.

The invention will be further illustrated by the following figure and examples. However, these examples and figure should not be interpreted in any way as limiting the scope of the present invention.

### Brief description of the sequences

Sequence SEQ ID NO: 1 corresponds to the nucleotide sequence of a signal oligonucleotide, herein called B11a.
Sequence SEQ ID NO: 2 corresponds to the nucleotide sequence of an amplification oligonucleotide, herein called CB11a-2PS3.
Sequence SEQ ID NO: 3 corresponds to the nucleotide sequence of a leak absorption oligonucleotide, herein called pTB11a PS3.
Sequence SEQ ID NO: 4 corresponds to the nucleotide sequence of a reporting probe. The reporting probe herein called MBB11aBsmlAtto633 consists of the oligonucleotide of sequence SEQ ID NO: 4 coupled to the fluorophore Atto633 in 5' and to the quencher BHQ2 in 3'.
Sequence SEQ ID NO: 5 corresponds to the nucleotide sequence of a conversion oligonucleotide, herein called TSBtoB11a-2+2P.
Sequence SEQ ID NO: 6 corresponds to the nucleotide sequence of an activator oligonucleotide, herein called TotalSeqB.
Sequence SEQ ID NO: 7 corresponds to the nucleotide sequence of a conversion oligonucleotide, herein called Let7atoB11a-2+2P.
Sequence SEQ ID NO: 8 corresponds to the nucleotide sequence of an aptamer coupled to an activator oligonucleotide, herein called TD05_TotalSeqB.
Sequence SEQ ID NO: 9 corresponds to the nucleotide sequence of an aptamer coupled to an activator oligonucleotide, herein called TE02_TotalSeqB.
Sequence SEQ ID NO: 10 corresponds to the nucleotide sequence of a linker sequence (T)₁₀ (in 5') linked to an activator oligonucleotide (in 3'). A cholesterol moiety linked to a Prolinol by a (6-amino-6-oxohexyl) carbamic acid linker is attached at the 5' extremity of the oligonucleotide herein called DLet7a T10 ChoPro.
Sequence SEQ ID NO: 11 corresponds to the nucleotide sequence of a signal oligonucleotide, herein called Bc12.
Sequence SEQ ID NO: 12 corresponds to the nucleotide sequence of an amplification oligonucleotide, herein called CBc12-2PS4.
Sequence SEQ ID NO: 13 corresponds to the nucleotide sequence of a leak absorption oligonucleotide, herein called pTBc12T5SP.
Sequence SEQ ID NO: 14 corresponds to the nucleotide sequence of a reporting probe. The reporting probe herein called MBBcBsmlAtto633 consists of the oligonucleotide of sequence SEQ ID NO: 14 coupled to the fluorophore Atto633 in 5' and to the quencher BHQ2 in 3'.
Sequence SEQ ID NO: 15 corresponds to the nucleotide sequence of a conversion oligonucleotide, herein called TSBtoBc-2+2P.
Sequence SEQ ID NO: 16 corresponds to the nucleotide sequence of an aptamer.
Sequence SEQ ID NO: 17 corresponds to the nucleotide sequence of an aptamer.
Sequence SEQ ID NO: 18 corresponds to the nucleotide sequence of an activator oligonucleotide.
Sequence SEQ ID NO: 19 corresponds to the DNA sequence of an oligonucleotide, which may be attached to particles and has a 3' part complementary to a signal oligonucleotide.
Sequence SEQ ID NO: 20 corresponds to the nucleotide sequence of a signal oligonucleotide.
Sequence SEQ ID NO: 21 corresponds to the nucleotide sequence of a signal oligonucleotide.
Sequences SEQ ID NO: 22 and 23 are shown in Table 8.
Sequence SEQ ID NO: 24 is a common oligonucleotide sequence between SEQ ID NO: 22 and SEQ ID NO: 23.
Sequence SEQ ID NO: 25 is a flexible linker.
Sequences SEQ ID NO: 26 to 27 are shown in Table 13.
Sequences SEQ ID NO: 29 to 31 are shown in Table 15.
Sequences SEQ ID NO: 32 to 35 are shown in Table 18.
Sequences SEQ ID NO: 36 to 38 are shown in Table 19.
Sequences SEQ ID NO: 39 to 42 are shown in Table 20.
Sequences SEQ ID NO: 43 to 45 are shown in Table 21.
Sequences SEQ ID NO: 46 to 98 are shown in Table 22.
Sequence SEQ ID NO: 99 is shown in Table 24.
Sequences SEQ ID NO: 100 to 102 are shown in Table 25.
Sequences SEQ ID NO: 103 to 107 are shown in Table 26.
Sequences SEQ ID NO: 108 and 109 are shown in Table 27.
Sequences SEQ ID NO: 110 and 111 are shown in Table 28.
Sequences SEQ ID NO: 112 and 113 are shown in Table 29.
Sequence SEQ ID NO: 114 is shown in Table 8 and Sequence SEQ ID NO: 115 in Table 15.

### Figures

**Figure 1****. Phosphate buffer saline is toxic for the isothermal amplification reaction of the DNA toolbox**
   An autocatalytic reaction is setup to amplify the oligonucleotide Bc12. The amplification mixture is modified to contain various concentrations of PBS. Amplification curves in absence (left) or presence (1 nM, right) of the trigger Bc12.
**Figure 2****. Adapting a molecular program to work a 37°C**
   **(a)** A range of temperature was used for a molecular program optimized to detect TotalSeqB at 50°C. The ratio Cq_{NC} / Cq_{10pM} characterizes the efficiency of the detection: the higher the ratio, the better the detection of TotalSeqB. There is a clear dose response between the concentration of TotalSeqB target and the amplification time (Cq) at high temperature (>46.3°C). At lower temperature, there is no substantial difference between the amplification time of the samples with different concentration of TotalSeqB, highlighting the importance of the temperature on this amplification system.
   **(b)** A range of MgSO₄ was realized at 46°C for the same molecular program than in figure **a** but in a buffer close to the one used for Reverse-Transcription . The ratio Cq_{NC} / Cq_{10pM} characterizes the efficiency of the detection: the higher the ratio, the better the detection of TotalSeqB. In this case, 3 mM of MgSO₄ seems to be the best choice.
   (**c)** A range of TotalSeqB was realized at 37°C with a new molecular program in the optimized new buffer: the output of the DNA Toolbox is correlated to the concentration of TotalSeqB: the lower the Cq, the higher the concentration of TotalSeqB.

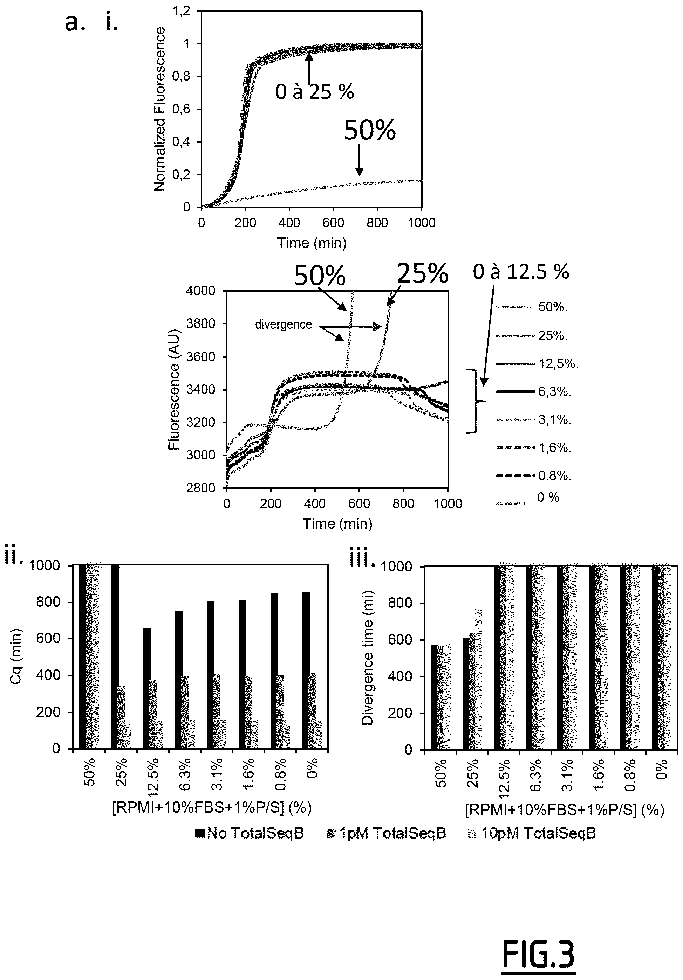

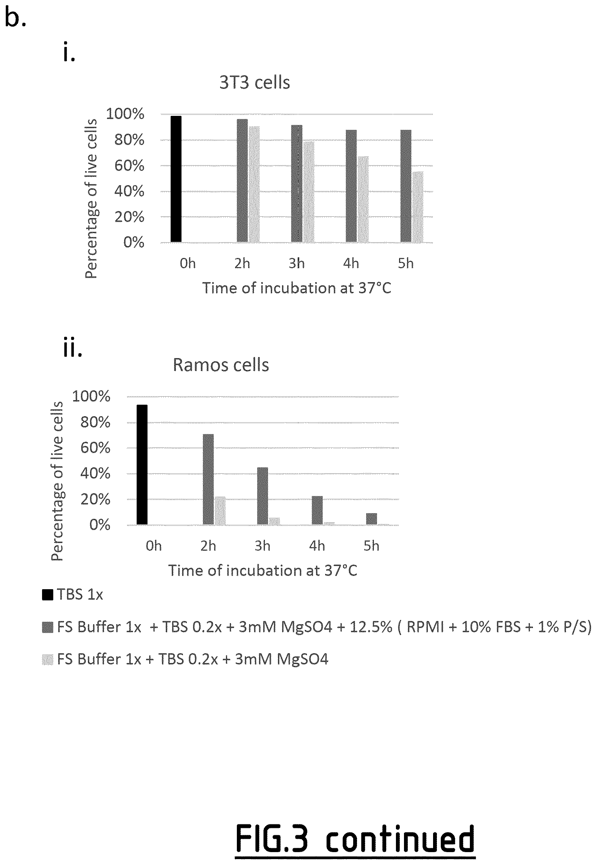

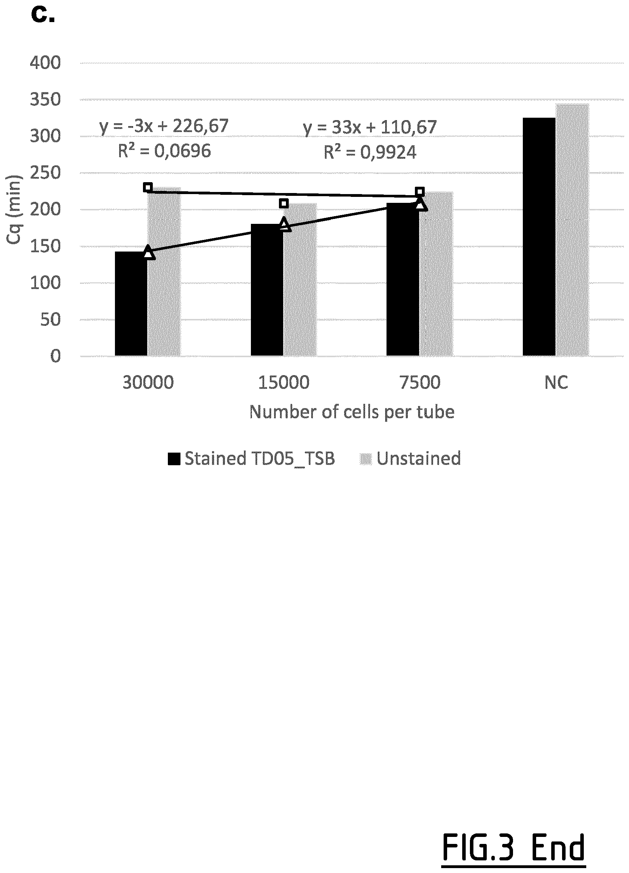

**Figure 3****: Impact of cellular media on both cell viability and the amplification reaction from the molecular program**
   **(a)** A range of cellular media (RPMI + 10% FBS + 1% P/S) was realized with respectively no TotalSeq B, 1pM of TotalSeqB and 10pM of TotalSeqB. Atto633 fluorescence was monitored (recorded time: 1002 minutes). (i) Amplification curves for Atto633 (left) and Evagreen intercalating dye (right) for 10 pM TotalSeqB samples. From these curves are extracted the amplification starting time Cq (ii) and the divergence time (iii): at 50% of cellular media, no amplification started, from 25% and below, the cellular media did not hamper the amplification reaction triggered by the TotalSeqB sequence. At 50% and 25%, divergence was observed while at 12.5% and below, no divergence was observed.
   **(b)** Effect of the buffer on the cell survival. 3T3 (i) and Jurkat (ii) cells were let overnight (16h) at RT in FS Buffer 1x with 3mM MgSO₄ supplemented or not with 12.5% of cellular media. Cells were stained with propidium iodine and and analyzed by flow cytometry to extract the proportions of alive and dead cells. Adding 12.5 % of cellular media increased the proportion of live cells from 27% to 79% (i) and from 6% to 25% (ii) for 3T3 and Jurkat cells respectively.
   **(c)** A range of unstained or DLet7aT10ChoPro-stained Ramos cells was incubated with the amplification mixture containing 12.5% of (RPMI + 10% FBS + 1% P/S) : the output of the DNA Toolbox is correlated to the quantity of cells, as suggested by the negative correlation coefficient of the Cq in function of the number of cells, while no amplification was observed independently of the concentration of unstained cells. The cellular media doesn't have an impact on the detection of stained cells.

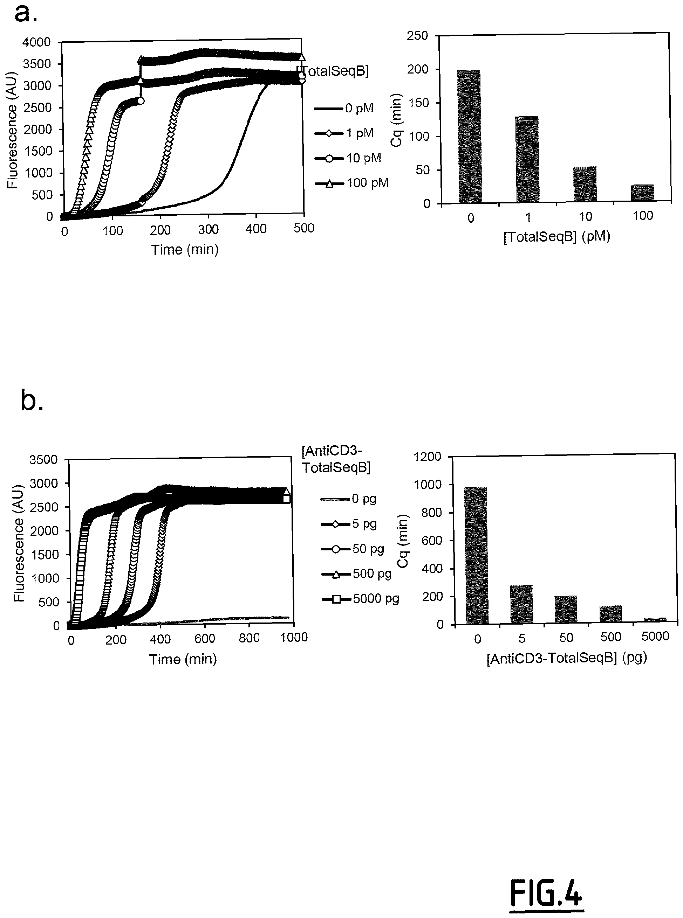

**Figure 4****. Antibody-grafted oligonucleotides are detected as well as free oligonucleotides by the DNA toolbox**
   **(a)** TotalSeqB oligo detection. The amplification curves (left) of samples spiked with various concentration of TotalSeqB were monitored at 37 °C and the Cq were extracted (left).
   **(b)** TotalSeqB-conjugated antibody detection. The amplification curves (left) of samples spiked with various concentration of AntiCD3-TotalSeqB antibody were monitored at 37 °C and the Cq were extracted (left).

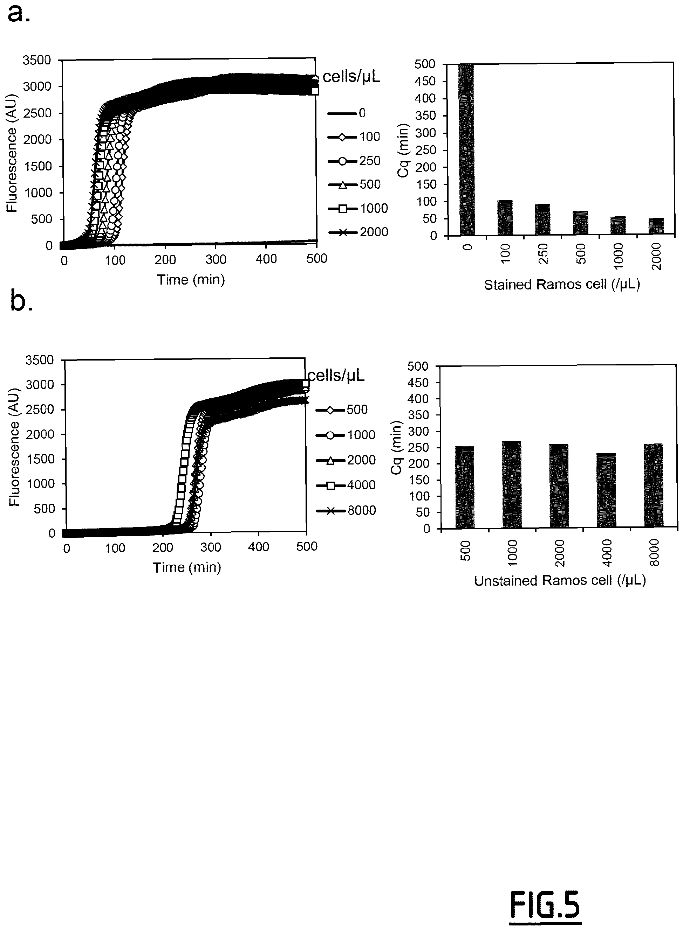

**Figure 5****. Detection of Ramos cells using an oligonucleotide-conjugated antibody**
   **(a)** Ramos cells, stained with TotalSeqB-conjugated antiCD3 antibody, were incubated at various concentration in the ToalSeqB-specific molecular program. Amplification curves were monitored in real-time (left) and the amplification time extracted (right). Cq value of amplification curve is inversely proportional to number of stained cells.
   **(b)** Negative controls were performed with unstained cells at various concentration. Cq values of the amplification curves are very close to each other, not differentiating between number of cells.

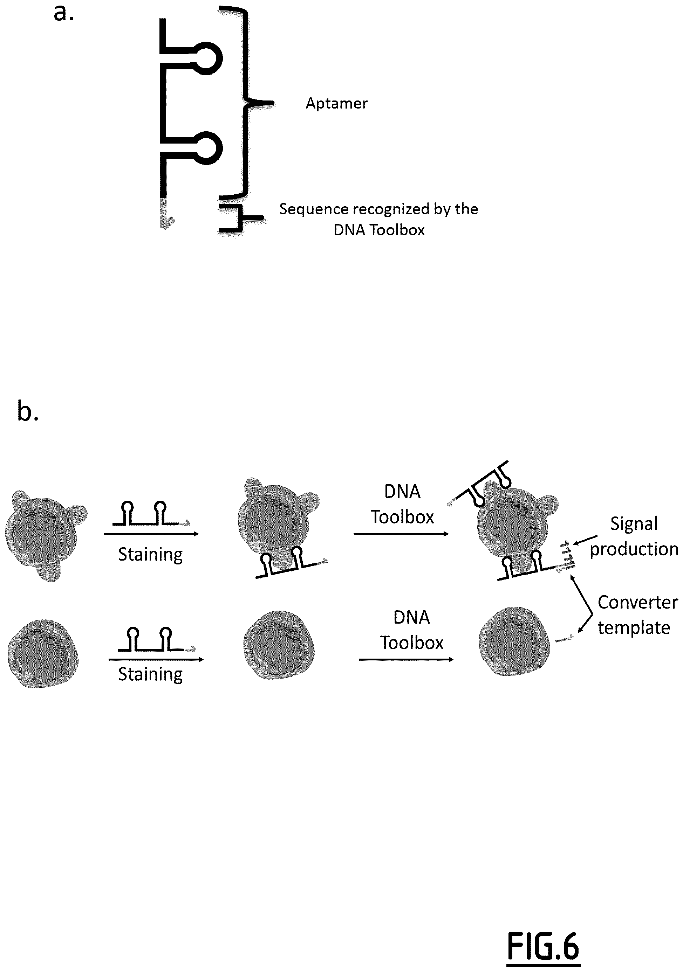

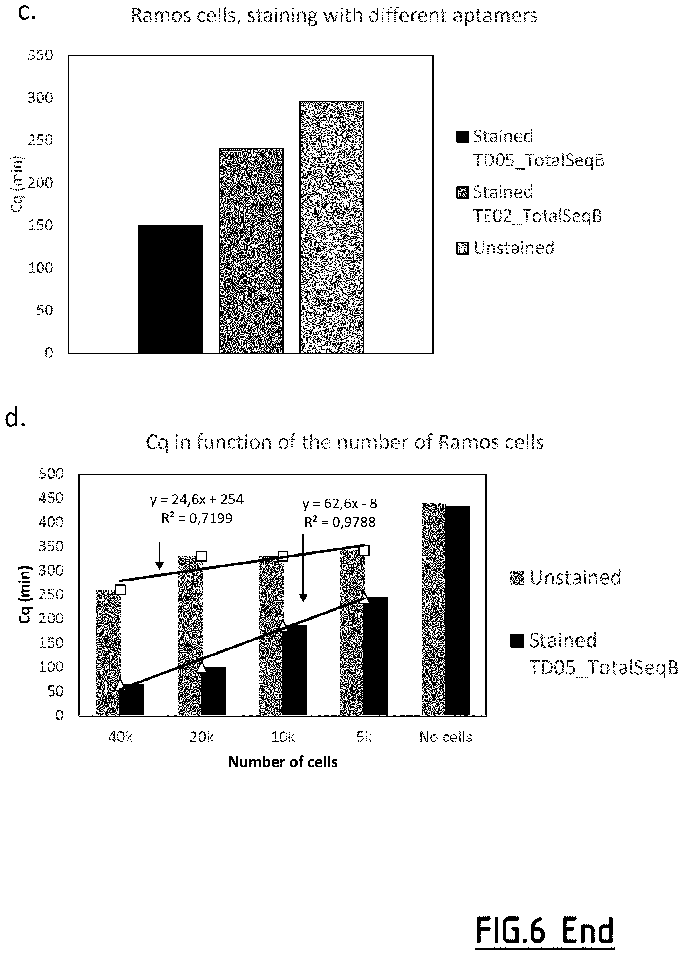

**Figure 6****: Detection of cells stained with a cell-specific aptamer**
   **(a)** Schematic structure showing the way the aptamers were modified to activate the DNA toolbox: a specific sequence (TotalSeqB) was added in 3' of reported aptamer sequences.
   **(b)** Cells are stained with a specific aptamer. If the cell presents the targeted markers, the aptamer binds to it and can therefore be used as input by the DNA Toolbox.
   **(c)** Both aptamers bind to the Ramos cells, as suggested by the lower Cq in comparison to unstained cells. TD05_TotalSeqB was shown to best initiate the amplification.
   **(d)** A range of TD05_TotalSeqB stained Ramos cells was realized: the output of the DNA Toolbox is proportional to the quantity of cells while it is not the case for stained without aptamer Ramos cells, as suggested by the correlation coefficient of the Cq in function of the number of cells.

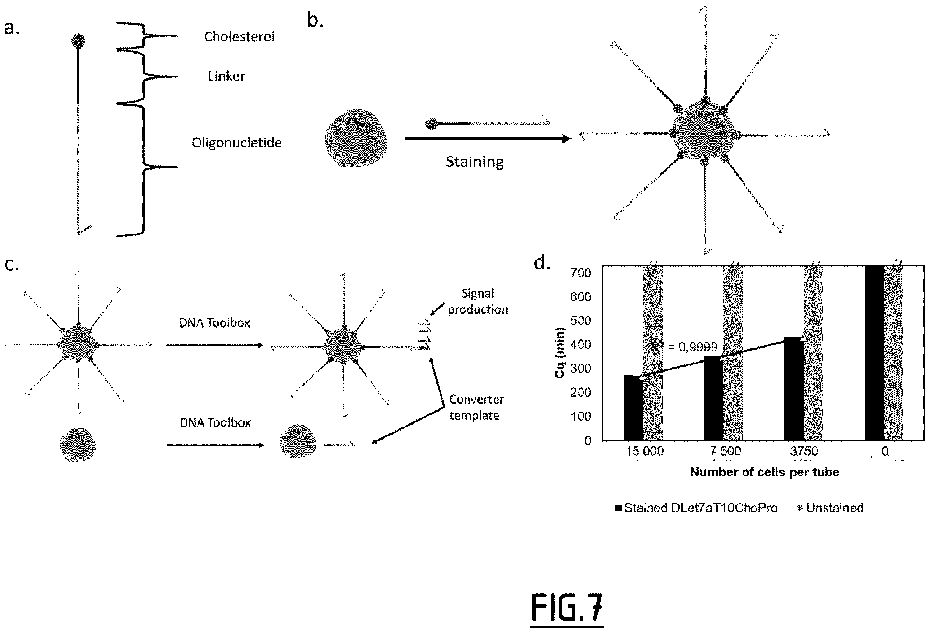

**Figure 7****: Detection of cells stained thanks to oligonucleotide conjugated with cholesterol**
   **(a)** Schematic structure of the used oligonucleotide: a cholesterol molecule is attached to the 5' extremity of the desired oligonucleotide using a T10 linker.
   **(b)** Cells are stained with a cholesterol-modified oligonucleotide. Due to its properties, the cholesterol inserts in the cell membrane.
   **(c)** Cells stained with the cholesterol-modified oligonucleotide can be detected with the DNA Toolbox: the cholesterol modified oligonucleotide is used as input. If the cells are not stained with this oligonucleotide, there is no input for the DNA Toolbox.
   **(d)** A range of unstained or DLet7aT10ChoPro-stained Ramos cells was incubated with the amplification mixture: the output of the DNA Toolbox is correlated to the quantity of cells, as suggested by the correlation coefficient of the Cq in function of the number of cells, while no amplification was observed independently of the concentration of unstained cells.

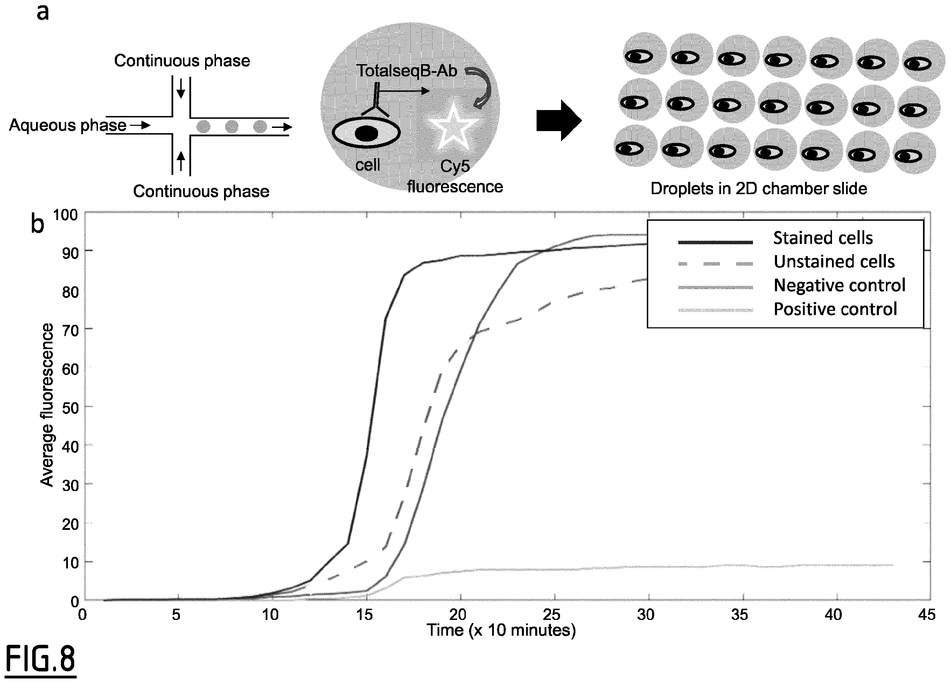

**Figure 8****. Single cell identification using oligonucleotide-conjugated antibody and DNA toolbox detection.**
   **(a)** Schematic diagram of droplet experiment.
      The fluorescence signals generated in the droplets were captured every 10 minutes over a period of seven hours in a 2D chamber. The droplets of the Stained population contained master mix, T-cells stained with anti-PD1 TotalseqB Antibody and barcoded with FITC. This population was gated using FITC+Cy5 fluorescence.
      The droplets of the Unstained population contained master mix, unstained T-cells and barcoded with Cascade Blue. This population was gated using Cascade Blue+Cy5 fluorescence.
      The droplets of the Positive Control population had master mix, anti-PD1 TotalseqB Antibody and barcoded with Cascade Blue and FITC. This population was gated using Cascade Blue+FITC+Cy5 fluorescence.
      The droplets of the Negative Control population had only master mix. This population was gated using Cy5 fluorescence.
   **(b)** Average fluorescence of droplets present in each reaction condition. The average was calculated from the fluorescence value of each population.

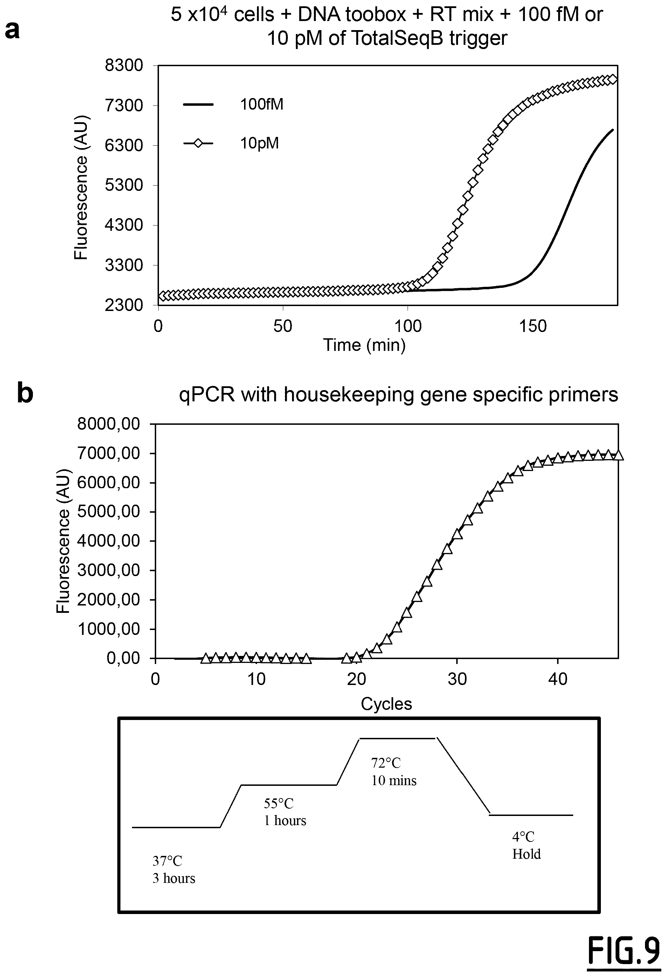

**Figure 9****. DNA toolbox reaction in the presence of fixed cells and compatibility with reverse transcription**
   **(a)** Fluorescence signal generated by reaction containing 5×10⁴ fixed Jurkat cells, DNA toolbox and reverse transcription components triggered by 10 pM and 100 fM TotalSeqB respectively.
   **(b)** Amplification curve generated from reaction containing 2 µl reaction mix from the reaction containing 5×10⁴ fixed Jurkat cells, DNA toolbox and reverse transcription components and subjected to PCR with gene specific house-keeping gene primers.

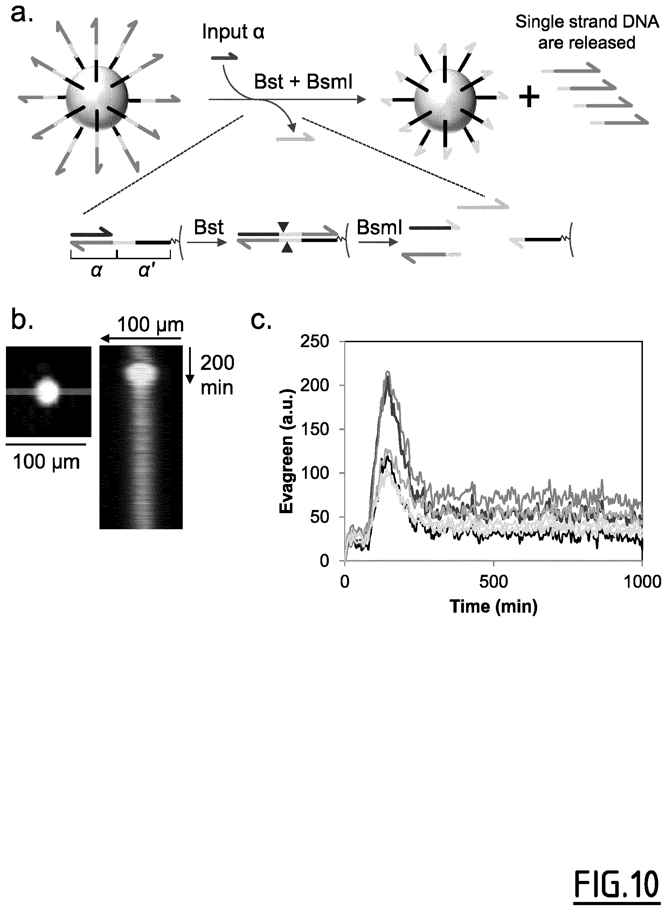

**Figure 10****. Single-stranded DNA release from microparticles**
   **(a)** DNA-conjugated particles are immersed in a reaction mixture. In presence of the α strands, the 3' extremity of the particle-bound oligo is released in solution using a DNA polymerase (Bst) and a restriction enzyme (Bsml).
   **(b)** Kymogram (time vs position) of one particle cross-section (Evagreen fluorescence).
   **(c)** Fluorescence time traces of 6 oligonucleotide-conjugated particles.
**Figure 11****. Different embodiments of A. DNA toolbox components, B. Surface markers, C. Proximity effects for secreted or surface markers and D. Combination of markers**
**Figure 12****. Single-cell barcoding strategies.** Arrowheads indicate the 3'-end of nucleic acids
**Figure 13****. Photo-Cleavage mediated selection following fluorescent readout** (using a fluorogenic reporter template)
**Figure 14****. DNA toolbox-based direct selection**

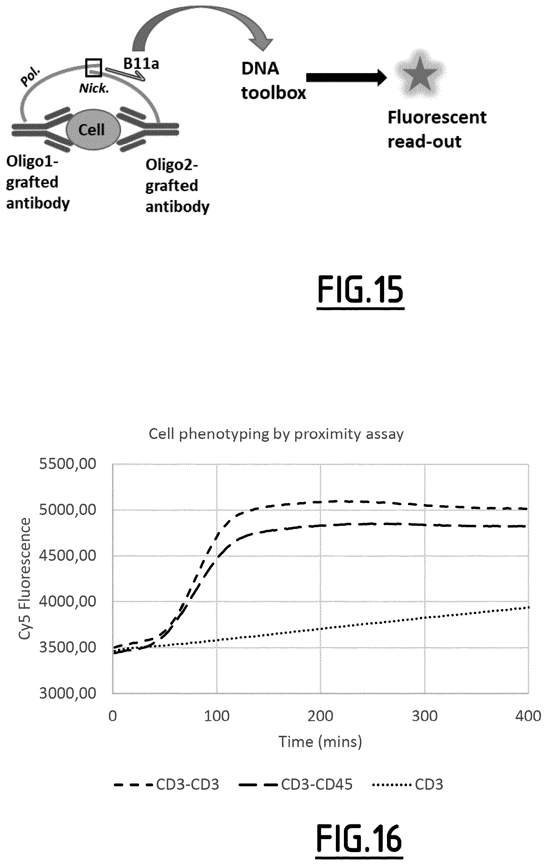

**Figure 15****. Schematic representation of phenotyping a cell targeting two surface markers using a pair of oligo-conjugated antibodies.** The oligos complement each other when the antibodies bind on target and produce B11a due to DNA polymerase and nickase activity. The B11a triggers the DNA toolbox which produces a fluorescent read-out confirming the presence of cell of the particular 2-marker phenotype.

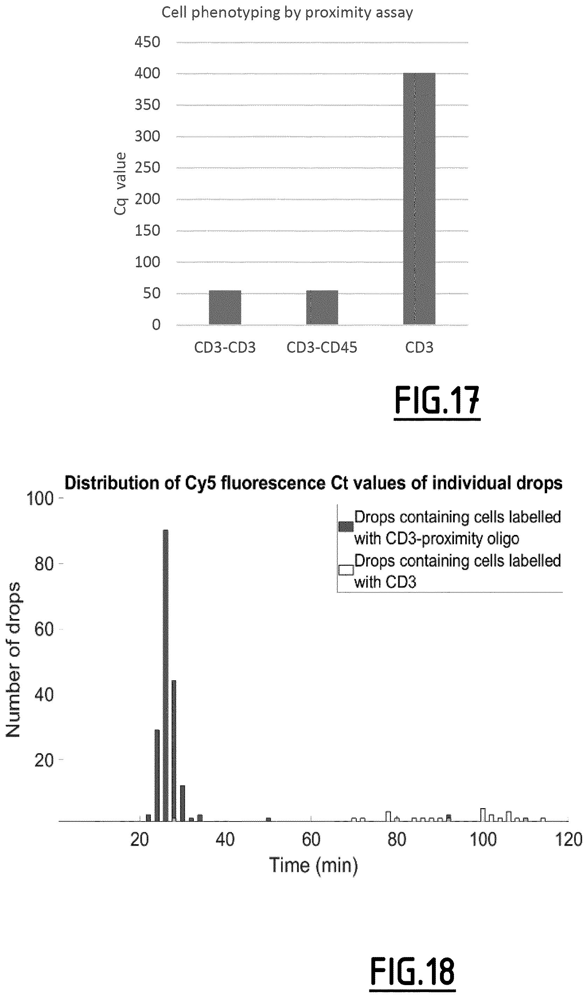

**Figures 16** **and** **17****: Phenotyping cell type targeting two surface markers using proximity oligo-conjugated antibody pair.**
Figure 16: Amplification curves of Jurkat cells phenotyped with CD3-totalseqB-aTSB-B11b-2 and CD3-totalseqB-aTSB-B11b-2toa (CD3-CD3), CD3-totalseqB-aTSB-B11b-2 and CD45-totalseqB-aTSB-B11b-2toa (CD3-CD45). Jurkat cells labelled with CD3-totalseqB-aTSB-B11b-2 only (CD3) was used a negative control.
Figure 17: Cq values from amplification curves shows successful phenotyping of cells targeting two surface markers using the proximity oligos conjugated on the antibody pairs.
**Figure 18****: Single cell identification using proximity oligonucleotide-conjugated antibody pair and DNA toolbox.** The Cy5 fluorescence intensity of individual droplets was digitally acquired using a droplet analysis pipeline. Fluorescence intensities was normalized by dividing the fluorescence intensity values of droplets at each time point by fluorescence intensity values at time point zero. A threshold of 1.5 was considered for plotting number of drops of each population above the threshold over the time period of imaging.
**Figure: 19** **Schematic diagram explaining the concept of proximity assay based detection of target protein**

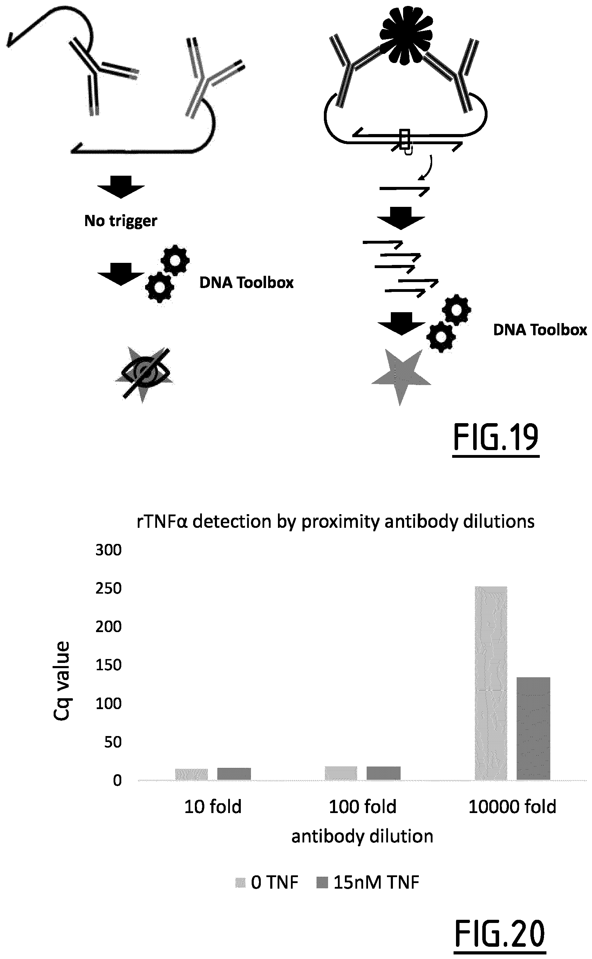

**Figure 20****: Determination of antibody dilution for rTNFα detection using proximity assay.** 1:10⁴ fold dilution of each oligo-conjugated antibody was found to detect and differentiate between 0 and 15nM rTNFα. Lower dilutions of the antibodies produced false proximity.

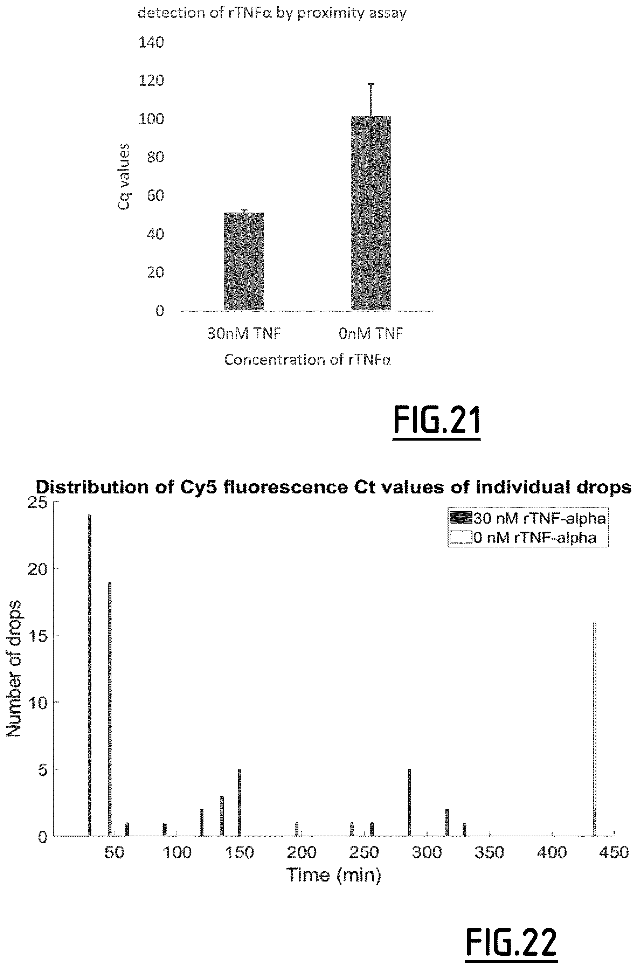

**Figure 21****: Average Cq values of amplification curves of proximity assay reactions containing 30 and 0nM TNFα.**
**Figure 22****: 30nM rTNFα detection by proximity assay in droplets.** The Cy5 fluorescence intensity of individual droplets was digitally acquired using a droplet analysis pipeline. Fluorescence intensities was normalized by dividing the fluorescence intensity values of droplets at each time point by fluorescence intensity values at time point zero. A threshold of 1.5 was considered for plotting number of drops of each population above the threshold over the time period of imaging. Majority of the drops containing 30nM rTNFα crossed the Cy5 fluorescence threshold within 30 to 48 minutes. Very few drops containing 30nM rTNFα crossed the Cy5 fluorescence threshold at various later time points. Drops not containing TNFα crossed the Cy5 fluorescence threshold after 430 minutes.

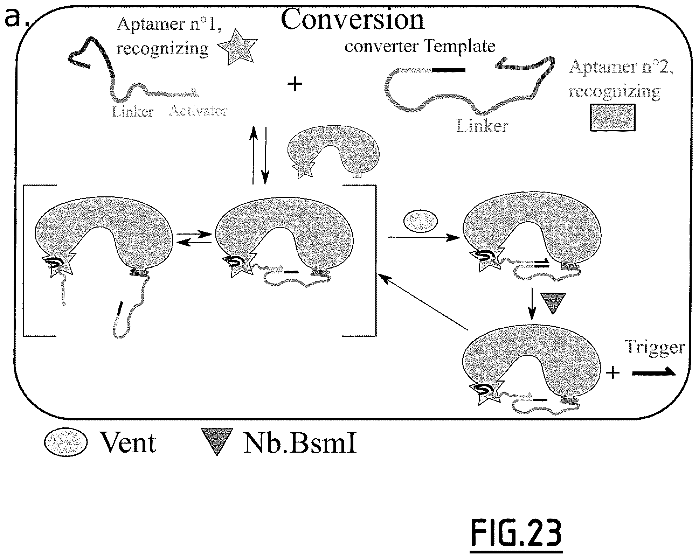

**Figure 23****: Principle scheme of proximity extension assay based PUMA.** Two aptamers (black and grey) recognizing parts of a protein, respectively represented by the grey star and the grey rectangle, are chosen. One of the aptamer is carrying a cT at its 5' extremity while the other one is carrying an activator, able to hybridize to the cT, at its 3' end. A linker is present between the aptamers and the cT or activator. In presence of the targeted protein, the aptamers bind to their respective target first, bringing the cT and the activator close. Then, the activator hybridizes to the cT, is extended by a polymerase and nicked to produce a trigger molecule. The oligonucleotides return to their initial states as the design is reversible. In absence of the targeted protein, the activator and the cT doesn't hybridize, leading to no trigger production.

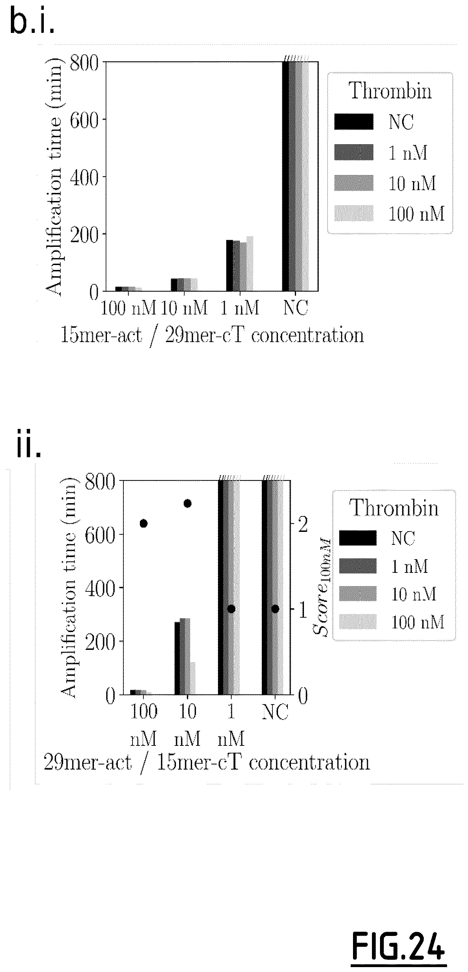

**Figure 24****. Proximity extension assay.** Different aptamer combinations for human alpha-Thrombin detection. The 15mer and the 29mer, two aptamers, were chosen for the detection of human alpha-Thrombin. **ii** A range of human alpha-Thrombin has been realized while the 15mer was carrying the cT and the 29 mer the activator. An equimolar range of aptamer was conducted. **i.** Range of human alpha-Thrombin while the 15mer was carrying the activator and the 29 mer the cT. An equimolar range of aptamer was conducted. The amplification times for the different human alpha-Thrombin concentrations are given by bar charts (left axis) and the scores_{100nM} are given by the black dots (right axis).

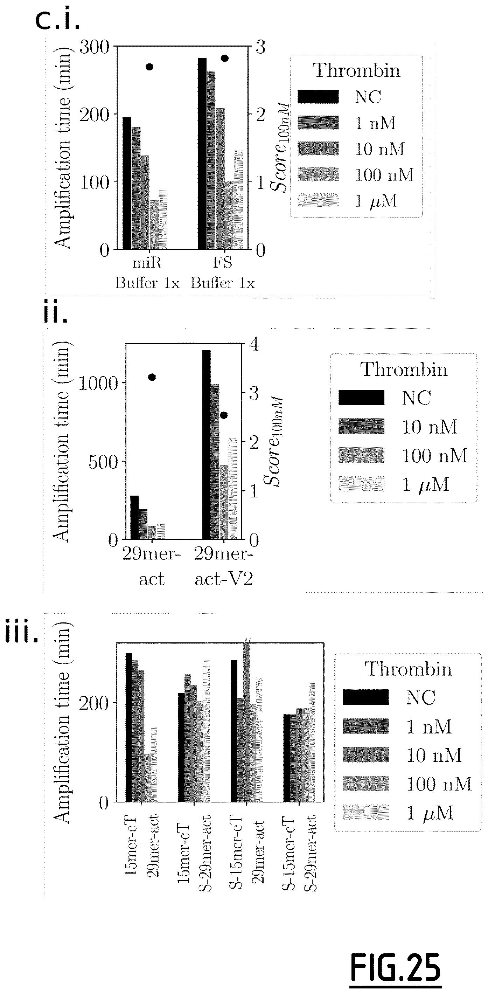

**Figure 25****. Proximity extension assay: human alpha-Thrombin detection optimization. i.** A range of human alpha-Thrombin was realized in mirBuffer 1x or in FS Buffer 1x in presence of 10 nM of 15mer-cT and 29mer-activator (29mer-act). **ii.** A range of human alpha-Thrombin was realized in FS Buffer 1x, in presence of 10 nM of 15mer-cT and 10 nM of either 29mer-act of 29mer-act-V2. The 29mer-act-V2 matches the cT on one base less than the 29mer-act. The amplification times for the different human alpha-Thrombin concentrations are given by bar charts (left axis) and the scores100nM are given by the black dots (right axis). **iii.** A range of human alpha-Thrombin was realized in FS Buffer containing 10 nM of 15mer-cT and 29mer-act. Controls with 10 nM of scrambled aptamers have been realized. Scrambled aptamers are indicated by the "S" before the name of the aptamer.

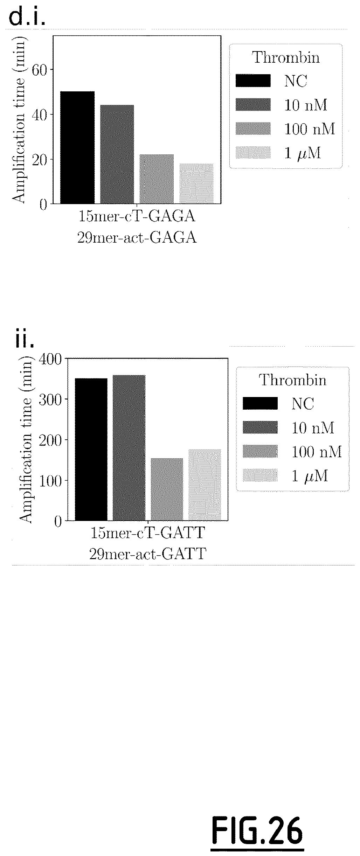

**Figure 26****. Proximity extension assay: human alpha-Thrombin detection.** A range of human alpha-Thrombin was realized in FS Buffer with 100nM of 15mer-cT-GAGA / 29mer-act-GAGA (a.) and with 100nM of 15mer-cT-GATT / 29mer-act-GATT (b.). The amplification times for the different human alpha- Thrombin concentrations are given by bar charts (left axis) and the scores_{1µM} are given by the black dots (right axis).

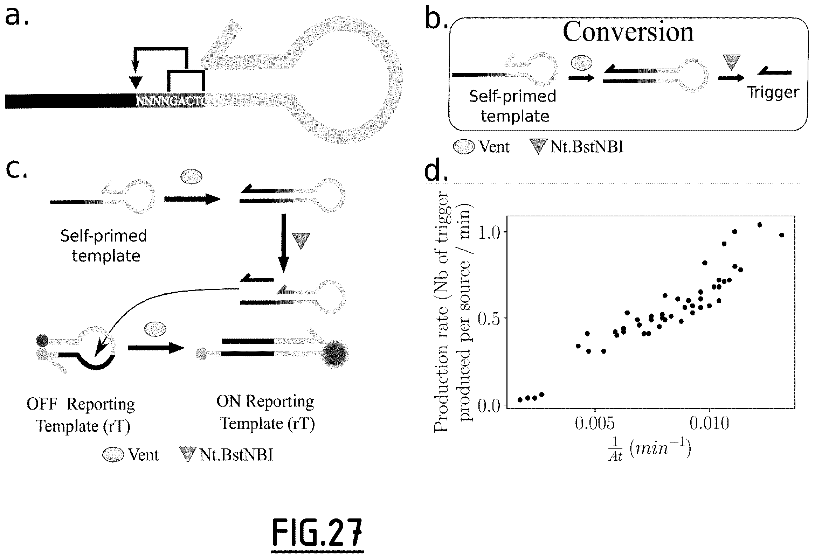

**Figure 27****. Optimization. a.** Scheme of a self-primed template. 4 bases are present between the Nt.Bst.NBI nicking site and the trigger complementary sequence. N denotes either a A, T, C or G base. **b.** Conversion module used for the PUMA: a sT undergoes polymerisation / nicking cycles, thus producing trigger. **c.** Principle of the production rate experiment. A sT undergoes polymerization / nicking cycles in presence of a rT. The trigger is linearly produced and the opening rate of the rT is correlated to the production rate of the sT. **d.** Production rate in function of the inverse of amplification time obtained for each tested sequence.

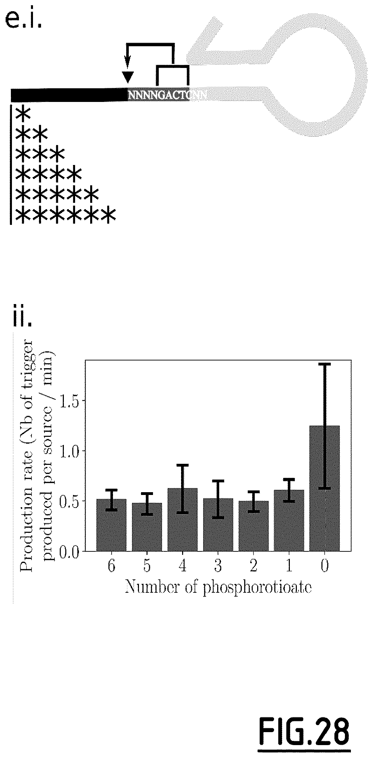

**Figure 28****. Self-primed template: phosphorotioates optimization.e.i.** Scheme of a sT. Phophorothioates, symbolized by "*", can be added to the backbone of the sT. The choice has been made to add some at its 5' extremity, corresponding to the trigger complementary part. **ii.** Production rate in function of the number of phosphorotioates added to the sT backbone. Except the number of phosphorotioates, the sequences of the sTs were the same for all tested conditions.

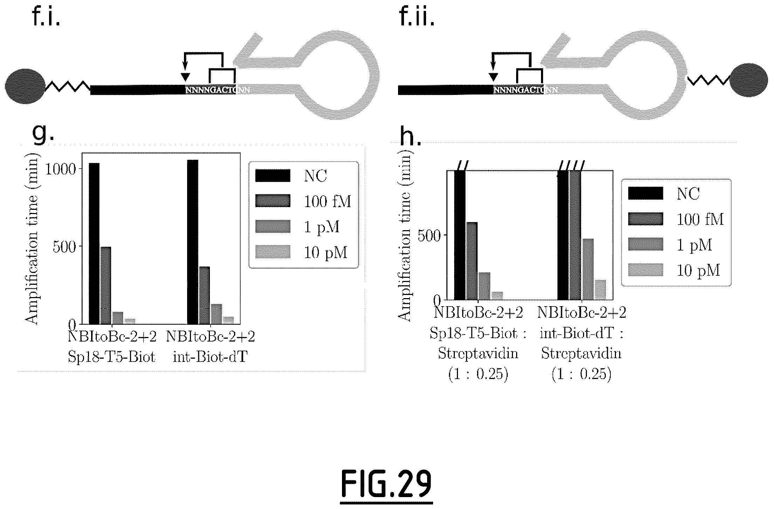

**Figure 29****. Self-primed template: biotin position optimization.** f. sT biotin (black circle) positions. The biotin has been placed either at the 5' extremity (i.), via a T5-Sp18 linker, or inside the stem loop of the self-primed template (ii.) via an amino-dT building block. g. sTs ranges. h. sTs ranges in presence of streptavidin.

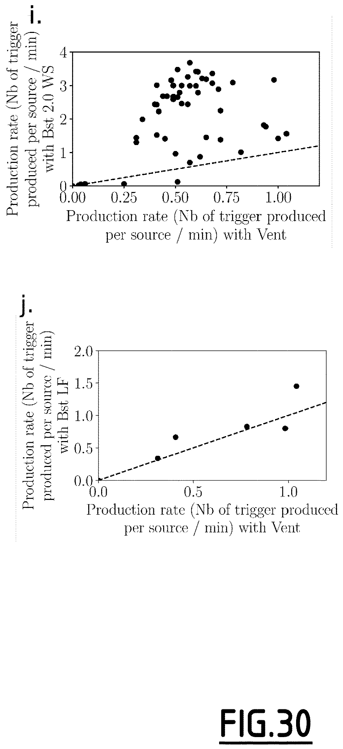

**Figure 30****. i.** Production rates obtained with Bst 2.0 WS in function of the production rates obtained with Vent for different sTs. The dotted line represents the equality of production rates: the points above have a higher production rate with Bst 2.0 WS than with Vent(exo- ). **j.** Production rates of B11a trigger obtained with Bst LF at 37°C in function of the production rates of Bc trigger obtained with Vent at 50°C for different self-primed template. The dotted line represents the equality of production rates.

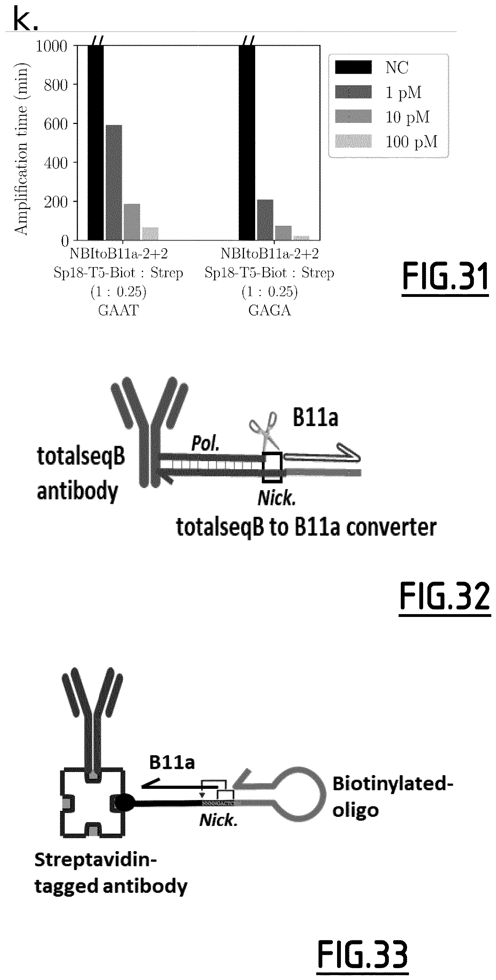

**Figure 31****. Self-primed template ranges in presence of 0.25 equivalent of streptavidin.** Two combinations of bases between the trigger complementary and the Nt.BstNBI nicking site were tested: GTTT and GAGA.
**Figure 32****. TSB_full_toB11a-2+2 design of self-primed template.**
**Figure 33****. NBltoB11a-2+2Sp18T5Biot GAGA design of self-primed template.**

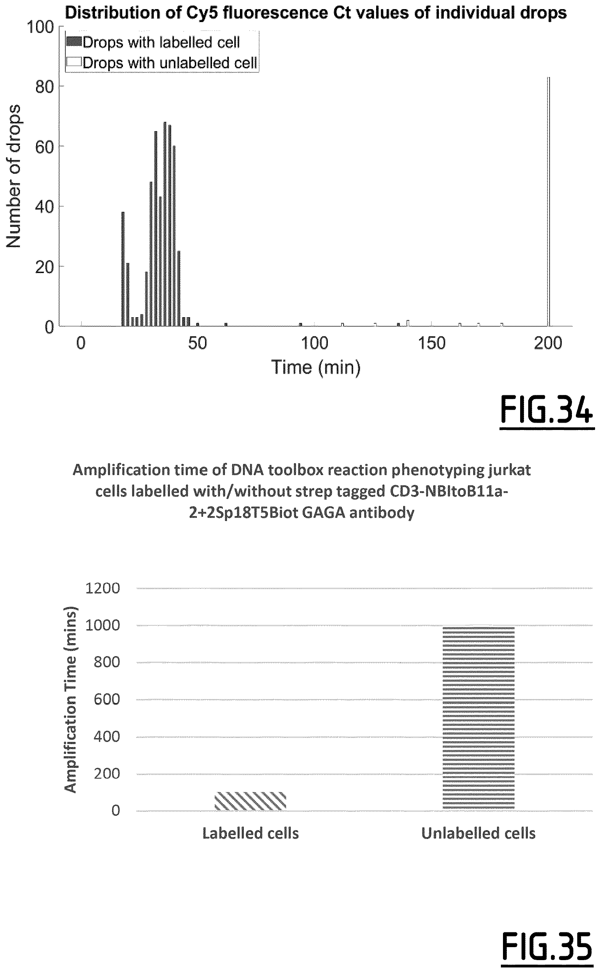

**Figure 34****. Single cell identification using anti-CD3 totalseqB antibody hybridized to TSB_full_toB11a-2+2 and DNA toolbox.** The Cy5 fluorescence intensity of individual droplets was digitally acquired using a droplet analysis pipeline. Fluorescence intensities was normalized by dividing the fluorescence intensity values of droplets at each time point by fluorescence intensity values at time point zero. A threshold of 1.5 was considered for plotting number of drops of each population above the threshold over the time period of imaging.
**Figure 35****. Phenotyping Jurkat cells using streptavidin tagged anti-CD3 antibody conjugated with NBltoB11a-2+2Sp18T5Biot GAGA.** Cq value of amplification curves of DNA toolbox bulk reaction phenotyping Jurkat cells labelled with/without self-primed template tagged antibody.

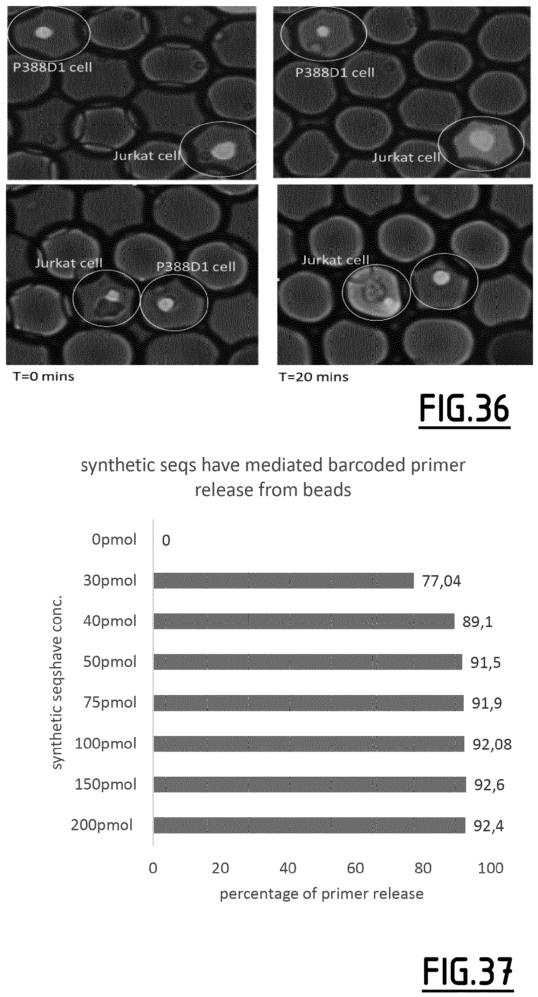

**Figure 36****. Snapshot of 2D time lapse imaging of drops containing mouse cell and jurkat cell at 0- and 20-minutes time points.**
**Figure 37****. Graph showing percentage of primer release when treated with a concentration range of synthetic seqshave after 2 hours at 37°C.** 30pmol synthetic seqshave leads to 77% release of barcoded primers from hydrogel beads. 40pmol seqshave causes approximately 90% release and further increase of seqshave concentration does not change the percentage of release significantly.

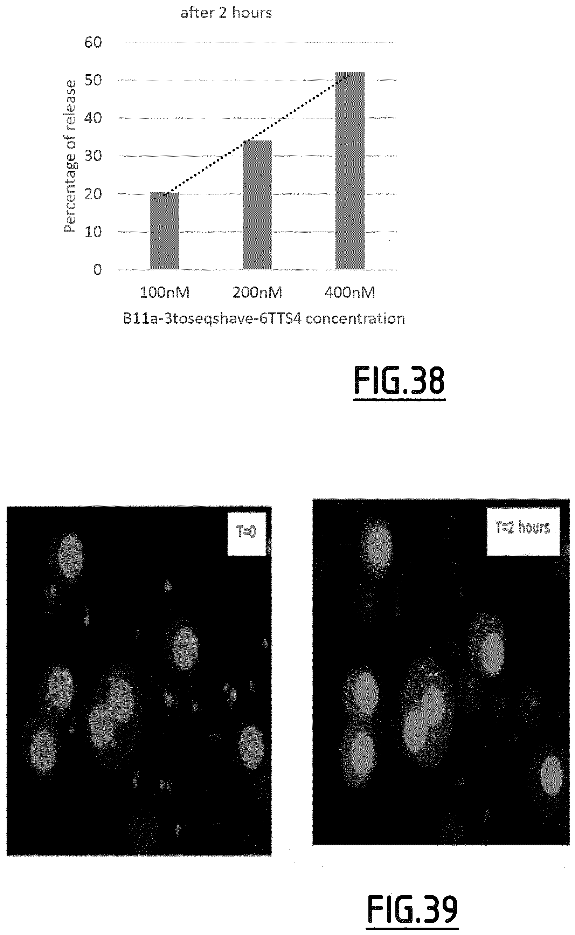

**Figure 38****: DNA toolbox mediated primer release from hydrogel beads.** The graph shows percentage of primer release by DNA tool box containing increasing concentrations of seqshave converter template (B11a-3toseqshave-6TTS4). The percentage of release linearly increases with increasing concentration of the converter template.
**Figure 39****. Snap shot of time lapse imaging of droplets** containing hydrogel bead containing Cy5 labelled fluorescent primers and self-primed template tagged antibody labelled cells. After 2 hours only the drops containing labelled cell and barcoded hydrogel bead show release of fluorescent primers whereas the drops not containing labelled cell do not show primer release.

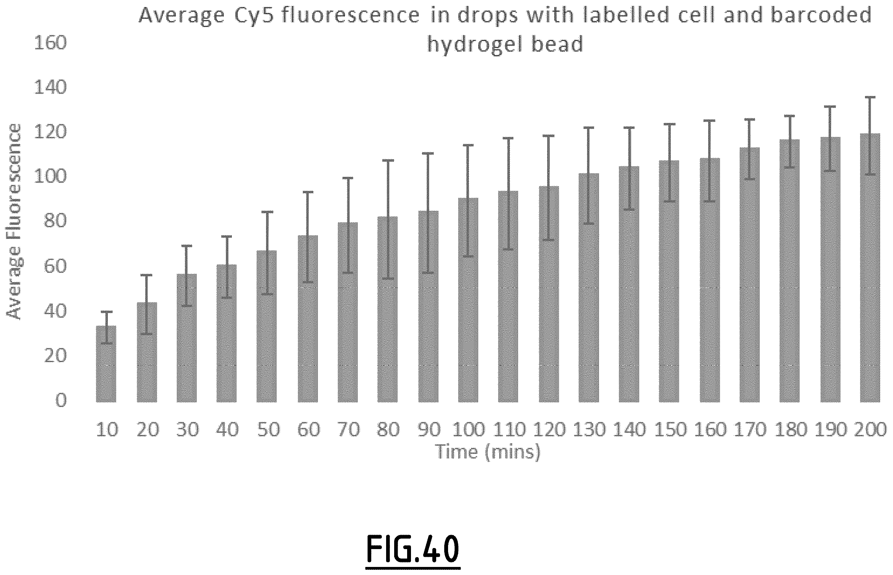

**Figure 40****. Average Cy5 Fluorescence intensity over time** in drops containing labelled cell, barcoded hydrogel bead and DNA tool box was calculated and plotted over time. Time lapse imaging of the drops was taken every 10 minutes. Cy5 fluorescence intensity of drops at every time point were evaluated using ImageJ. The self-primed-template conjugated antibody bound on cell surface triggers DNA toolbox mediated release of fluorescent primers from hydrogel beads. The Cy5 intensity in drops increases as the reaction progresses with time. The release of primer increases significantly from 10 to 120 minutes and saturates as time progresses.

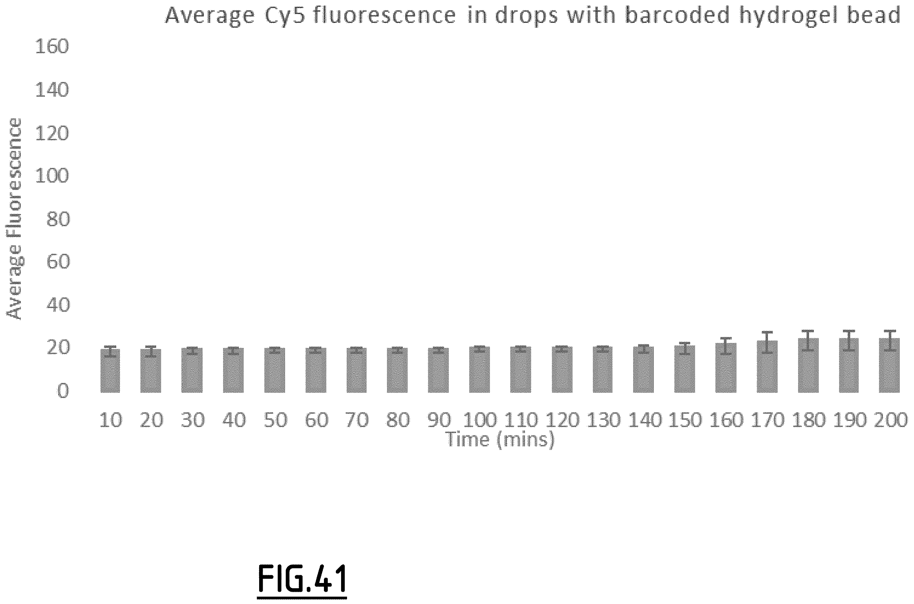

**Figure 41****. Average Cy5 Fluorescence intensity over time** in drop containing barcoded hydrogel bead and DNA tool box. Due to absence of self-primed-template labelled cell, the DNA toolbox in these drops remain inactivated; thus primer release is not observed as indicated by the absence of increase of Cy5 fluorescence intensity over time.

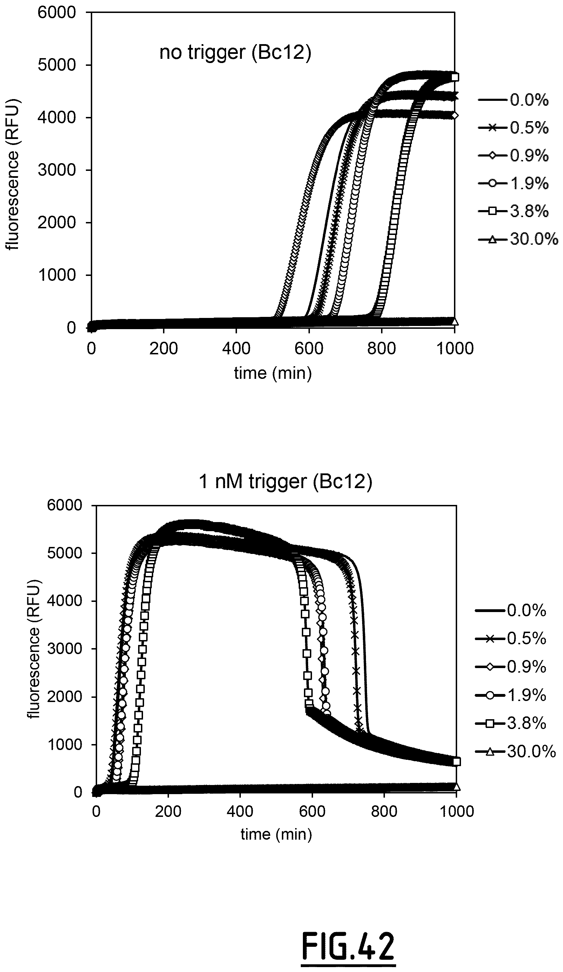

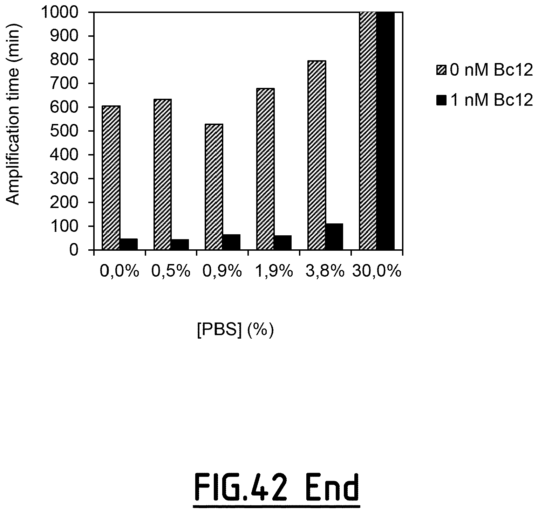

**Figure 42****. Low concentrations of phosphate ions are compatible with the isothermal amplification reaction of the DNA toolbox.** An autocatalytic reaction is setup to amplify the oligonucleotide Bc12 in presence of a varying concentration of PBS (0, 0.5, 0.9, 1.9, 3.8 or 30 %). (a) Amplification curves in absence (left) or presence (1 nM, right) of the trigger Bc12. (b) Amplification times extracted from panel (a) reveal that low concentrations of PBS do not inhibit the amplification reaction.

### EXAMPLES

### Methods and materials

### Oligonucleotides

All oligonucleotides (templates and aptamers) used in the present invention were purchased either from Biomers (Germany) and purified by HPLC or Eurofins with high purity salt-free grade (cartridge purification). Template sequences are protected from degradation by the exonuclease ttRecJ using 5' phosphorothioate modifications.

Said oligonucleotides are shown in Table 1 below:
Table 1. Oligonucleotide sequences used in the invention. "*" denotes phosphorothioate backbone modification. "p" denotes 3' phosphate modification. "ChoPro" denotes a Cholesterol linked to a Prolinol by a (6-amino-6-oxohexyl) carbamic acid linker in the 5' extremity of the oligonucleotide. "aT", "pT", "rT" and "cT" correspond to the autocatalytic template, pseudo template, reporting template and conversion template respectively. Atto633 and BHQ2 correspond to the fluorophore/quencher pair. Underlined sequences correspond to reported aptamer sequences (Tang et al. (2007) Analytical Chemistry, 79:4900-4907).

**Table 1**

| **SEQ ID NO** | **Name** | **Sequence** | **Function** |
|---|---|---|---|
| **1** | B11a | CATTCAGTTAG | Trigger/sign al |
| **2** | CB11a-2PS3 | C*T*A*ACTGAATGCTAACTGAA p | aT |
| **3** | pTB11a PS3 | A*A*C*TAACTGAATG p | pT |
| **4** | MBB11aBs mlAtto633 | Atto663-C*A*T*CGATATACTAACTGAATGCGATG-BHQ2 | rT |
| **5** | TSBtoB11a -2+2P | TGCTAACTGAAGTTTGACTCATTGCTAGGACCGG CTT p | cT |
| **6** | TotalSeqB | GCTTTAAGGCCGGTCCTAGCAA | Target |
| **7** | Let7atoB11 a-2+2P | TGCTAACTGAAGTTTGACTCAAACTATACAACCTACT ACCTCA p | cT |
| **8** | TD05_Total SeqB | AAGGAGCAGCGTGGAGGATAAACACCGTGGAGGAT AGTTCGGTGGCTGTTCAGGGTCTCCTCCCGGTGTTA GGGTGTGTCGTCGTGGTAAGCCGGTCCTAGCAA | Modified aptamer |
| **9** | TE02_Total SeqB | AAGGAGCAGCGTGGAGGATATAGGCAGTGGTTTGA CGTCCGCATGTTGGGAATAGCCACGCCTTTAGGGT GTGTCGTCGTGGTAAGCCGGTCCTAGCAA | Modified Aptamer |
| **10** | DLet7a T10 ChoPro | ChoPro TTTTTTTTTTTGAGGTAGTAGGTTGTATAGTT | Cholesterol oligonucleoti de |
| **11** | Bc12 | CATTCTGGACTG | Trigger/sign al |
| **12** | CBc12-2PS4 | C*A*G*T*CCAGAATG - CAGTCCAGAA p | aT |
| **13** | pTBc12T5S P | T*T*T*TT-CAGTCCAGAATG p | pT |
| **14** | MBBcBsml Atto633 | Atto633*A*T*TCTGAATGCAGTCCAGAAT BHQ2 | rT |
| **15** | TSBtoBc-2+2P | TGCAGTCCAGAAGTTTGACTCATTGCTAGGACCGGC TT p | cT |
| **16** | TD05 | AAGGAGCAGCGTGGAGGATAAACACCGTGGAGGAT AGTTCGGTGGCTGTTCAGGGTCTCCTCCCGGTGTTA GGGTGTGTCGTCGTGGTAA | aptamer |
| **17** | TE02 | AAGGAGCAGCGTGGAGGATATAGGCAGTGGTTTGA CGTCCGCATGTTGGGAATAGCCACGCCTTTAGGGT GTGTCGTCGTGGTAA | aptamer |
| **18** | Part_TotalS eqB | GCCGGTCCTAGCAA | activator oligonucleoti de |
| **19** | Cbe12-1S4bioteg | Bioteg *C*G*A*TCCTGAATGCGATCCTGAAT *p* | oligonucleoti de |

### Cell culture

The base medium for Ramos and Jurkat cell lines is ATCC-formulated RPMI-1640 Medium (ATCC 30-2001).The complete medium consists RPMI-1640 supplemented with 10% fetal bovine serum (ATCC 30-2020). The cells are suspended in the complete medium and seeded on 25 cm² or 75 cm² culture flask. The culture is maintained in an incubator at 37°C with 5% CO₂. On reaching 75-80% confluency the cells are passaged. After retrieving from cell culture, the cells were finally re-suspended in TBS 1x at the concentration of 2.10⁶ cells.mL⁻¹.

### Aptamer Folding

Aptamers were diluted to 500 nM in aptamer folding buffer (1× Tris Borate Saline (TBS), 5 mM MgCl₂, 4.5 g.L⁻¹ glucose) and heated to 95°C for five minutes (Thermomixer, Eppendorf) then cooled on ice for 10 min. This protocol was adapted from the literature (Delley et al. (2018) Scientific Reports 8 :1-8).

### Cells staining

### Aptamer staining:

All staining steps were performed at 4°C in 0.5 mL DNA LoBind tubes (Eppendorf). 2.10⁵ cells were resuspended in 500 µL of staining buffer (1× TBS, 5 mM MgCl₂, 4.5 g.L⁻¹ glucose, 1 mg.ml⁻¹ BSA (SigmaAldrich) and 0.1 mg.ml⁻¹ salmon sperm DNA with a concentration of 21 nM of aptamer and let 30 minutes on ice. Cells were then washed three times with the staining buffer (centrifugation 300 rpm, 5 min, 4°C, resuspension in Staining Buffer) and two more times with TBS 1X. The cells were finally diluted in tubes at the desired concentration in TBS 1X.

### Antibody staining:

Two million cells (jurkat/ramos/human T cells) resuspended in their respective media were retrieved from the cell culture. The cells are centrifuged at 250 rcf for 5 minutes at 37°C, supernatant discarded and the cells are resuspended in 1 ml 1X TBS. The cells are again centrifuged at 250 rcf for 5 minutes at 37°C, supernatant discarded and finally resuspended in 200 µl 1X TBS. 100µl Biolegend staining buffer containing 2.7 pmol antibody is added to the cell suspension and incubated at 4°C for 30 minutes. Post incubation the cells are centrifuged at 250 rcf for 5 minutes at 4°C, supernatant discarded and the cells are resuspended in 1 ml 1X TBS. The washing step is repeated 5 times with 1ml 1X TBS to ensure complete removal of unbound antibodies. Finally the cells are resuspended in 100µl 1X TBS and kept on ice for further use.

### Reaction mixture assembly

All reaction mixtures were assembled at 4°C in 200 µL PCR tubes (Bio-Rad). The templates (cT, aT, pT and rT) and the cells were mixed with the reaction buffer (FS Buffer 1X (NEB), TBS 0.2X (coming from the cell suspension) or 12.5% of cellular media (RPMI + 10% Fetal Bovine Serum + 1% Penicillin / Streptomycin), 25 µM each dNTP (NEB), 3 mM MgSO₄ and 200 U.µl⁻¹ BSA (NEB)), together with 1% Evagreen 20X (Biotium) and the enzymes (200 U.ml⁻¹ Nb.Bsml (NEB), 10 U.ml⁻¹ Nt.BstNBI (NEB), 80 U.ml⁻¹ Vent(exo-) (NEB) or 36 U.ml⁻¹ Bst large fragment (NEB) and 23 nM ttRecJ (in-house brewed)).

The mixtures were then incubated in a thermocycler (CFX 96 wells, Biorad) at 37°C and Atto633 as well as Evagreen fluorescence were monitored.

### Example 1: Compatibility of DNA toolbox reactions in the presence of cells and maintenance of cell viability during the reaction

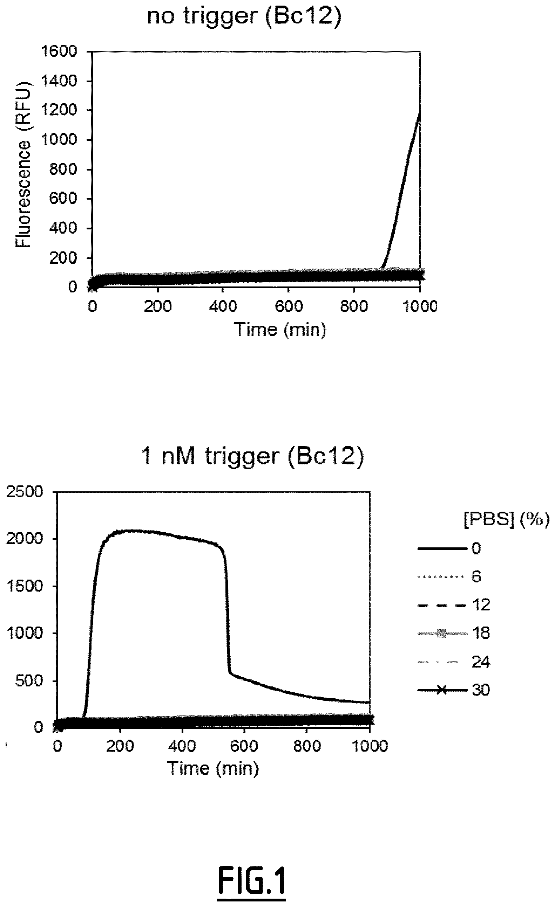

DNA toolbox reactions are usually performed between 45°C to 50°C in a specific buffer, with particular ion concentrations. It uses thermophilic enzymes, whose optimal temperatures are 65°C , 75°C, 55°C, 65°C and 60°C for Bst polymerase large fragment, Vent (exo-) polymerase, Nt.BstNBI, Nb.Bsml and ttRecJ, respectively. The inventors also discovered that small amounts of ruptured cells inhibit the DNA toolbox reaction. To avoid rupturing cells, it was thus necessary to adapt the DNA toolbox to physiological temperature and conditions and to add some components to stabilize the cells. In addition, the inventors observed that phosphate buffer saline, which is generally used as a resuspension medium for cell suspensions, has a strong inhibitory effect on the DNA toolbox reactions (**Figure 1**).

The experimental conditions were as follows:
Amplification mix

| | |
|---|---|
| Tris-HCl pH 8.41 | 50 mM |
| KCl | 75 mM |
| MgCl₂ | 3 mM |
| DTT | 3 mM |
| dNTP (each) | 25 µM |
| Evagreen | 0.2 X |
| BSA9000S | 200 µg/ml |
| Nb.BSMI | 200 U/ml |
| Vent (Exo-) | 80 U/ml |
| ttRecJ | 13 nM |
| Nt.BstNBI | 10 U/ml |
| PBS | 0-30% |
| Netropsin | 2 µM |
| Temperature | 45°C |

Oligonucleotides

| | |
|---|---|
| CBc12-2PS4 | 50 nM |
| pTBc12T5SP | 10 nM |
| MBBcBsmIAtto633 | 50 nM |
| Bc12 | 0-1 nM |

The inventors observed that in absence of PBS, the amplification reaction occurred (~800 min in absence of Bc12 and ~100 min with 1 nM of Bc12 to initiate the autocatalysis). In presence of a concentration of PBS as low as 6%, no amplification was recorded during the experiment duration (1000 minutes). Given that the salt concentration is known to not inhibit the reaction (8.2 mM Na⁺, 0.3 mM K⁺ and 8.4 mM Cl⁻ for 6 % PBS), the toxic effect may be attributed to the presence of phosphate ions (0.7 mM for 6% PBS). Nevertheless, concentrations of phosphate ions lower that 0.7 mM (6 % PBS) do not inhibit the amplification reaction (see **Figure 42**).

Therefore, it was necessary to find conditions that would allow cell handling and maintain cell viability while allowing proper functioning of the DNA toolbox reaction.

First, the buffer composition for cell manipulation was changed to Tris Buffer Saline (50mM Tris, 150 mM sodium chloride), and prepared carefully so as to provide nutrients for survival of cells as well as proper functioning of the molecular circuit.

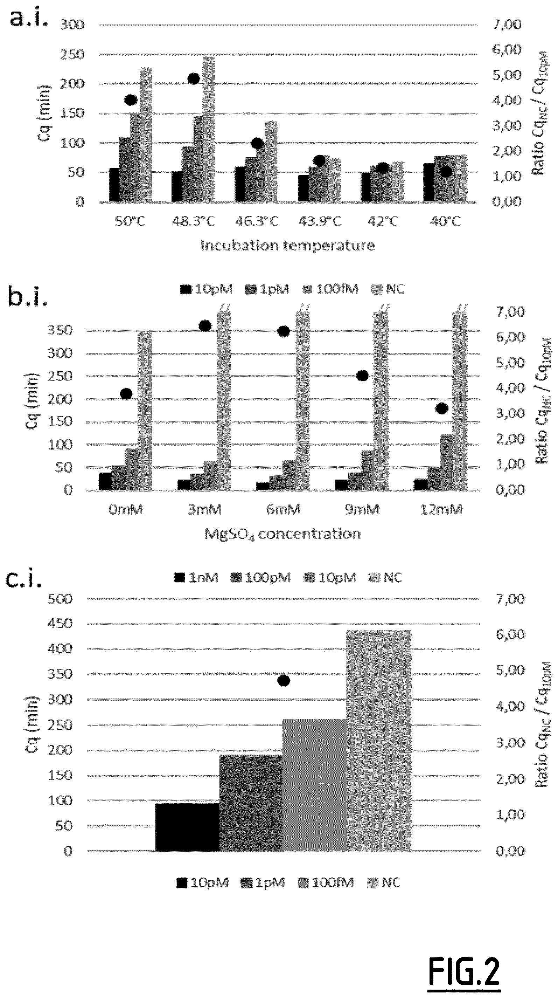

Second, a previously used molecular program, which only performs between 42°C and 50°C (Baccouche et al. (2014) Methods 67:234-249; Montagne et al. (2011) Molecular Systems Biology 7:466; Padirac et al. (2012) Proceedings of the National Academy of Sciences 109:E3212-E3220) was adapted to work at 37°C. A range of temperature was realized for a molecular program optimized to detect TotalSeqB at 50°C. As it can been seen in **Figure 2**a, there is a clear dose response between the concentration of TotalSeqB target and the amplification time (Cq) at high temperature (>46.3°C). At lower temperature, there is no substantial difference between the amplification time of the samples with different concentration of TotalSeqB, highlighting the importance of the temperature on this amplification system. In particular, the inventors observed that the lower the temperature, the sooner the amplification of negative control (called false positive, in absence of target). The false positive is due to spurious reactions that produce a few trigger oligonucleotides nonspecifically. This phenomenon, known as leak, is increased when the temperature is lower and prevent the sensitive detection of the TotalSeqB target. Additionally, the enzymes, when used much below their optimal temperature, loose activity and become prone to product inhibition. Altogether, the inventors can conclude that classic DNA toolbox designs do not perform at 37°C.

However, decreasing the melting temperature of the trigger on the corresponding autocatalytic template, which can be obtained by shortening the trigger or adjusting their sequence toward higher AT/GC content, was found to solve this issue. The consequence of the lower melting temperature, is that triggers produced nonspecifically are not recaptured efficiently on the autocatalytic template and are most likely to be deactivated by the pseudo-template under a dynamical process, previously reported (Montagne et al. (2016) Nature Communications 7 :13474). Additionally, this change also solved the enzyme issue since nicked products can spontaneously dehybridize, avoiding enzyme inhibition.

Moreover, to ensure compatibility with subsequent step of the process, for example transcriptomic analysis of cellular RNA, it was necessary to use a different buffer than the one used classically for DNA toolbox systems (Gines et al., (2020) Science Advances*),* and to change it the buffer compatible with reverse transcription. It was first necessary to supplement the RT buffer with dNTPs, and MgSO₄. A range of MgSO₄ was realized in order to calibrate the concentration that was needed (**Figure 2b**). Supplementing the buffer with 3 mM of MgSO₄ gives the best signal-to-noise ratio. In this optimized buffer, a range of TotalSeqB was realized at 37°C with the new molecular program. It was observed that the higher the concentration of TotalSeqB, the lower the Cq, demonstrating the efficient sensing of the target down to 100 fM at 37°C in this buffer compatible with cell integrity and reverse transcription (**Figure 2**c).

Third, it was found that that adding a proportion of cellular media in the molecular program buffer could maintain cell viability, while avoiding negative impact on the molecular program efficiency.

First, a range of cellular media (RPMI + 10% FBS + 1 % penicillin/streptomycin [P/S]) were tested with respectively 0, 1 and 10 pM of TotalSeqB in order to assess the effect of the cellular media on the specificity of the amplification reaction (**Figure 3**). Atto633 and EvaGreen fluorescence were monitored to look both at the starting time of the amplification and an eventual unspecific DNA production of the system (called "divergence" (Urtel et al. (2019) Biochemistry 58:2675-2681). It has been shown that at 12.5% of cellular media and under, specific amplification from the molecular program occurred and no divergence was monitored. However, above 12.5% of cellular media (25% and 50% in this case), divergence occurred and at 50 % of it, no specific amplification took place.

Next, the inventors monitored cell death with / without 12.5% of cellular media in FS Buffer 1x with 3 mM MgSO₄. Jurkat and 3T3 cells were let overnight (16h) at room temperature in those two buffers and stained with propidium iodide. Cells were analyzed through flow cytometry and proportions of alive and dead cells were estimated (**Figure 3**b, 3T3 cells (i) and Jurkat cells (ii)). The inventors observed that in the presence of 12.5% cellular media, the proportion of alive cells is increasing from 27% to 79% for 3T3 cells and from 6% to 25% for Jurkat cells.

Finally, the inventors performed the detection of Ramos cells, either unstained or stained with the cholesterol-modified oligonucleotide (see example 5), with the molecular program in the presence of 12.5% of cellular media. While no amplification was observed with unstained cells (monitoring time = 730 minutes), the concentration of stained cells was inversely correlated to the amplification time (i.e. the more cells, the sooner the amplification). These results demonstrate that it is possible to use the molecular program for specific detection of cell bound oligonucleotides, while maintaining cell viability by inclusion of cellular media in the reaction buffer.

### Example 2: DNA toolbox can detect antibody-labeled oligonucleotides as well as free oligonucleotides

The process of phenotyping specific cells types with the DNA toolbox can be achieved by using DNA-tagged antibodies specific to cell surface markers of target cells; the DNA tags can then be used as inputs for detection by the DNA toolbox. The inventors tested the feasibility using commercially available TotalSeqB tagged antibodies (from Biolegend). The goal of the experiment is to detect DNA labeled antibodies using the DNA toolbox, where the tagged DNA will serve as the trigger for the toolbox reaction.

The inventors first examined the detection of unconjugated trigger oligonucleotide (TotalSeqB) by the molecular program at 37°C, in the reverse transcription buffer. Tenfold serial dilutions in milliQ water of the TotalSeqB Oligos and antibody were spiked in an amplification mixture composed of the reverse transcription buffer, the 4 oligonucleotides and 4 enzymes of the molecular program. Samples of 10 µL were incubated at 37°C in BioRad CFX96 real time PCR and the fluorescence of the probe (Atto633) was recorded every 2 minutes. Figure 4a shows the amplification time traces. As expected, the more concentrated is the TotalSeqB oligonucleotide target, the sooner the amplification, demonstrating the specificity of the amplification reaction.

The inventors then verified that antibody-conjugated oligonucleotides retain their capacity to trigger the amplification reaction. Figure 4b depicts the amplification time traces for samples spike with various concentrations of serially diluted TotalSeqB anti-PD1 antibody (Biolegend cat no. 329961). In absence of antibody, no amplification was observed. As expected, the amplification time (Cq) is negatively correlated to the antibody-conjugated oligonucleotide, demonstrating that the tethered TotalSeqB sequence is able to trigger the amplification.

In conclusion, the DNA toolbox can detect TotalSeqB labeled antibodies as well as the free floating DNA tag TotalSeqB. The molecular circuit clearly distinguishes various concentrations of the oligos/antibody producing florescent signals at clearly demarcated time points. The system was sensitive enough to detect very low concentration of oligos (100 fM) and antibodies (50 pg) in the reaction.

### Reaction setup: for 100 µl reaction

**Table 2**

| Components | Concentrations |
|---|---|
| **Buffer** | |
| Tris-HCl (pH 8.3) | 50 mM |
| KCl | 75 mM |
| MgCl₂ | 3 mM |
| MgSO₄ | 3 mM |
| DTT | 10 mM |

| **Molecular Program** | |
|---|---|
| CB11a-2PS3 | 50 nM |
| pTB11aPS3 | 12 nM |
| MBB11aBsmIAtto633 | 50 nM |
| TSBtoB11a-2+2P | 0.5 nM |

| **DNA toolbox enzymes** | |
|---|---|
| BSA | 200 µg/mL |
| Nb.BsmI | 200 u/mL |
| Bst Large Fragment | 36 u/mL |
| ttRecJ | 13 nM |
| Nt.BstNBI | 10 u/mL |
| dNTP each | 50µM |
| Netropsin | 2 µM |

### Example 3: DNA toolbox can detect cells stained with cell-specific oligo-labeled antibody

In this example, the use of DNA-tagged antibodies for cell phenotyping using the DNA toolbox was tested (**Figure 5**).
Materials and methods: 1x10⁶ Ramos cells were labeled with TotalSeqB anti-CD3 antibody (cat no. 300477) with repeated washing steps to remove any unbound/weakly bound antibodies (Biolegend cell staining buffer (cat no. 420201)). The cells were finally resuspended in 1X FS buffer. 8 µl of master mix and 2 µl of oligos/antibody dilution were mixed and run at 37°C in BioRad CFX96 real time PCR.

### Reaction setup

**Table 3**

| Components | Concentrations |
|---|---|
| **Buffer** | 1X |
| Tris-HCl (pH 8.3) | 50 mM |
| KCl | 75 mM |
| MgCl₂ | 3 mM |
| DTT | 10 mM |
| MgSO₄ | 6 mM |

| **Molecular Program** | |
|---|---|
| CB11a-2PS3 | 50 nM |
| pTB11aPS3 | 12 nM |
| MBB11aBsmIAtto633 | 50 nM |
| TSBtoB11a-2+2P | 0.5 nM |

| **DNA toolbox enzymes** | |
|---|---|
| BSA9000S | 200 µg/mL |
| Nb.BSMI | 200 u/mL |
| Bst Large Fragment/5 | 36 u/mL |
| ttRecJ | 13 nM |
| NBI | 10 u/mL |
| dNTP | 0.5mM |
| Netropsin | 2 µM |

As can be seen from the results displayed on Figure 5, the DNA toolbox could phenotype CD3 expressing Ramos cells and it could differentiate between number of cells on the basis of number of CD3 positive antibody present on the surface of the cells. The system can successfully be tested for expression of surface markers on the surface of heterogeneous cells.

### Example 4: DNA toolbox can detect cells stained with a cell-specific aptamer

In this example, the use of modified cell-specific aptamers for cell phenotyping using the DNA toolbox was tested.

A proof of concept has been performed with Ramos cells: two Ramos-specific aptamers (TD05 and TE02) reported in the literature (Tang et al. (2007) Analytical Chemistry 79:4900-4907) have been tested. The complementary sequence of the 3' extremity of the cT "TSBtoB11a-2+2P"was appended to the 3' part of these aptamer sequences (**Figure 6a**). As a result, the presence of the aptamer should lead to the production of trigger B11a by the cT "TSBtoB11a-2+2P. (**Figure 6**b). When Ramos cells were stained with either of the aptamers, the amplification occurred sooner than with unstained cells. The effect was significantly higher with TD05 in comparison to TE02, demonstrating the specific triggering of the amplification by cell-bound TD05. (**Figure 6**c). **Figure 6**d shows the amplification start in presence of an increasing concentration of Ramos cells, either unstained or stained with TD05_TotalSeqB strand. For each cell concentration > 0, stained cells induced faster amplification (lower Cq) in comparison to unstained cells. Furthermore, higher concentrations of stained cells were correlated with earlier amplification. Altogether, these results demonstrate that we succeed in staining cells with the modified specific aptamer, and that the cell-bound modified aptamer is able to specifically trigger the amplification.

### Example 5: Detection of cells stained non-specifically, using an oligonucleotide conjugated with cholesterol

In this example, the staining and detection of cells using a cholesterol-conjugated oligonucleotide (DLet7aT10ChoPro) was demonstrated (**Figure 7a**). Due to its properties, it is already known that the cholesterol moiety inserts into the cell membranes (You et al. (2017) Nature Nanotechnology 12:453-459) thereby attaching the oligonucleotide to its surface (**Figure 7b**). The 3' part of DLet7aT10ChoPro oligonucleotide was used as input for the cT Let7atoB11a, inducing the production of B11a trigger (**Figure 7c**). **Figure 7d** depicts an experiment where varying concentration of Ramos cells, either unstained or stained with the cholesterol-modified oligonucleotide, were incubated together with the amplification mixture. While no amplification was observed with unstained cells (up to 730 minutes), the stained cells could trigger the amplification. Moreover, the concentration of stained cells was inversely correlated to the amplification time (i.e. the more cells, the sooner the amplification). These results demonstrate the specific triggering of the amplification by cell-anchored DNA strands.

### Example 6: Phenotyping single cells encapsulated in microdroplets by DNA toolbox.

In order to phenotype individual cells in a heterogeneous population, the assay has to be designed for single cells. Thus, to implement DNA toolbox for phenotyping single cells, the molecular program was used in a droplet microfluidics system (**Figure 8a**). The experiment was designed to generate thousands of droplets where each droplet encapsulates a single cell together with DNA toolbox reagents.

### Materials and Methods

*Cell Staining:* 2x10⁶ T-cells (containing both PD-1 positive and negative cells) were resuspended in 200 µl 1x TBS buffer and stained with Anti-PD1 TotalSeqB antibody (Biolegend Cat No. 329961) by incubation at 4°C for 30 minutes. After the incubation the cells were pelleted down by centrifugation at 4°C, 300 g for 10 min and the supernatant was discarded. For removal of unbound antibodies, the cell pellet was resuspended in 1 ml 1X TBS (kept on ice), centrifuged at 4°C, 300 g for 10 min and the supernatant was discarded. This washing step was repeated three times to completely get rid of any unbound antibodies. After washing, the cells were resuspended in 1X FS buffer (Invitrogen).

*Master Mix:* The master mix was prepared as follows:

**Table 4**

| Components | Concentrations |
|---|---|
| **Buffer** | |
| Tris-HCl (pH 8.3) | 50 mM |
| KCl | 75 mM |
| MgCl₂ | 3 mM |
| MgSO₄ | 3 mM |

| **Molecular Program** | |
|---|---|
| CB11a-2PS3 | 50 nM |
| pTB11aPS3 | 12 nM |
| MBB11aBsmIAtto633 | 50 nM |
| TSBtoB11a-2+2P | 0.5 nM |

| **DNA toolbox enzymes** | |
|---|---|
| BSA | 200 µg/mL |
| Nb.BsmI | 200 u/mL |
| Bst Large Fragment | 36 u/mL |
| Nt.BstNBI | 10 u/mL |
| dNTP each | 50µM |
| Netropsin | 2 µM |

The master mix was divided in four equal parts to create droplets of various test populations as follows:
**Aqueous Phase:** The following reaction mixes were prepared for droplet encapsulation:
   ***a. Positive Control:*** This reaction mix consisted of the master mix, anti-PD1 TotalseqB antibody, Cascade blue and FITC. This reaction mix was used to create droplets that serve as a DNA toolbox positive control, producing Cy5 florescence by the molecular program triggered by the DNA tag present on the anti-PD1 antibody. In order to segregate the droplets of this population from others, the reaction mix was barcoded with a mix of cascade blue and FITC. Hence, during analysis, only the Cy5 florescence generated from the droplets that are cascade blue and FITC positive are considered as the Positive Control.
   ***b. Negative Control:*** This reaction mix consisted of only the master mix. No signal molecules were provided to trigger the reaction. Thus, the droplets produced from this mix will produce Cy5 florescence at very late time point due to self-triggering. During analysis, the florescence generated from droplets that are only Cy5 positive are considered as the Negative Control.
   ***c. Stained:*** This reaction mix consisted of the master mix, T-cells stained with anti-PD1 TotalseqB antibody and FITC. The number of cells added to the reaction mix was adjusted to achieve a λ of 0.2 to 0.4 which gives a Poisson distribution of 67% empty droplets, 27% containing one cell and 6% containing more than one cell. During analysis, the florescence generated from droplets that are FITC and Cy5 positive are considered as the Stained Test.
   ***d. Unstained:*** This reaction mix consisted of master mix, unstained T-cells and cascade blue. Here also the number of cells added to the reaction was adjusted to achieve a λ of 0.2 to 0.4. During analysis, the fluorescence generated from droplets that are cascade blue and Cy5 positive are considered as Unstained.
**Droplet Creation and chamber filling:** Droplets were created by hydrodynamic flow-focusing on a microfluidic device with a nozzle 25 µm wide, 30 µm deep, and 40 µm long, fabricated using soft-lithography in poly-(dimethylsiloxane) (PDMS). The continuous phase comprised of 2% (w/w) 008-FluoroSurfactant (RAN Biotechnologies) in HFE7500 fluorinated oil. Each population of aqueous phase was co-flowed on separate chips on a PDMS device to produce droplets of 50-80 pL volume and collected separately. Finally, the droplets of each population were pooled together, mixed by pipetting using a 1000 µl tip. The mouth of the tip was cut to create larger orifice so as not to create pressure leading to droplet breakage. After gently mixing for 2-3 times the pooled droplets were injected into a chamber where they were immobilized in a two-dimensional (2D) array by a slight physical confinement in the z-direction within the chamber, allowing them to be observed over time, and kinetic analysis of droplets.
**Data acquisition and analysis.** Imaging was performed using a Nikon (Ti-E) inverted microscope with a motorized stage. Excitation light was provided by a LED source (SOLA light engine, Lumencor Inc.). Fluorescence for the specific channels were recorded using appropriate band-pass filters (GFP and TRITC filter sets, Nikon, and Cy5 filter set, Semrock) and camera settings (Orca R2, Hamamatsu) at 37°C and ambient oxygen concentrations. Images were acquired using a 10× objective (NA 0.45). The fluorescent microscope was programmed to take images every 10 min using blue (cascade Blue barcode), green (FITC barcode), red (probe signal) and bright field channels for 7 hours. The array was imaged in one channel throughout, and the channel was changed afterwards and the array reimaged. The images were then analyzed using a custom Matlab script (Mathworks). Fluorescence gating was performed according to the fluorescent barcodes used for each population (i.e Stained Test = FITC + Cy5; Unstained = Cascade Blue + Cy5; Positive Control = FITC + Cascade Blue + Cy5; Negative Control = Cy5). The average fluorescence of each droplet of a particular population at each time point was calculated. Average fluorescence vs time point was plotted to analyze the time difference between different reaction conditions.

### Results

The DNA toolbox encapsulated in picolitre droplets successful produced a fluorescent read-out and each reaction population could be identified according to their assigned respective color barcodes (**Figure 8**).The fluorescence signal appeared, on average, earlier in droplets containing Stained cells than in droplets containing Unstained cells (**Figure 8** **b**). The fluorescence signal of the Positive Control droplets appeared, on average, slightly later than the Stained cells, due to the lower concentration of antibody in the Positive Control droplets (it is distributed across all droplets, not just those containing cells) (**Figure 8** **b**). Most of the negative control droplets showed no signal, although a few droplets generated false positive signals which may arise due to cell debris or droplet fusions (**Figure 8** **b**).As reflected by the scatter plot (data not shown), fluorescence signal of some droplets of the Stained population (bc1) were possibly classified with the Negative population (bc3), also explaining the false positive observations.

### Example 7: DNA toolbox in presence of fixed cells and compatibility with reverse transcription.

The detection of fixed cells by DNA toolbox was also tested. The experiment was designed for phenotyping of fixed cells using cell surface marker specific antibodies. For further genotyping post phenotyping, the inventors wanted to see whether the DNA toolbox can function in presence of reverse transcription reagents and the process cDNA synthesis can occur in same reaction setup.

### Material and Method:

**Fixation:** 800 µl absolute methanol (pre-chilled at -20°C) was added drop wise to 10⁶ cells resuspended in 200 µl Tris-buffer saline (50 mM Tris, 150 mM Sodium Chloride, pH 7.4). The 1000 µl suspension was then incubated at -20°C for 30 min.

**Resuspension of fixed cells:** The fixed cells were centrifuged at 4°C for 10 min at 300g. The pelleted cells were re-suspended in Tris-buffer saline (50 mM Tris, 150 mM Sodium Chloride, pH 7.4).

### Master Mix for 100µl reaction

**Table 5**

| Components | Concentrations |
|---|---|
| **Buffer** | 1X |
| Tris-HCl (pH 8.3) | 50 mM |
| KCI | 75 mM |
| MgCl₂ | 3 mM |
| DTT | 10 mM |
| MgSO₄ | 6 mM |
| **Molecular Program** | |
| CBc12-2PS3 | 50 nM |
| pTBc12T5SP | 12nM |
| MBB11aBsmIAtto633 | 50nM |
| BL_Total_Seq_BtoBc-2+2P | 0.5nM |
| **DNA toolbox enzymes** | |
| BSA9000S | 1% |
| Nb.BSMI | 2% |
| Vent | 4% |
| ttRecJ/140 | 1.5% |
| NBI/10 | 1% |
| **Reverse Transcription Reagents** | |
| Reverse transcriptase | 10U/µl |
| Olido dT | 5µM |
| dNTP | 0.5mM |
| Netropsin | 2mM |

8µl of the above master and 5.10⁴ fixed Jurkat cells (resuspended in 1x FS buffer) were mixed in Biorad CFX tubes and run in BioRad CFX real-time PCR machine with following program:

**Table 6**

| Reaction | Temperature | Time |
|---|---|---|
| DNA TOOLBOX | 37°C | 180 min |
| REVERSE TRANSCRIPTION | 55°C | 60 min |
| DEACTIVATION OF ENZYMES | 72°C | 10 min |

After completion of above reaction, 2 µl reaction mix was taken as template and subjected to PCR with SYBR green dye, ampliTaq gold DNA polymerase, dNTPs and house-keeping gene specific primers.

### Results

The Jurkat cells were viable during the DNA toolbox reaction which is reflected by positive fluorescent signal shown in **Figure 9a****.** The increase in temperature from 37°C to 55°C led to lysis of cells and release of mRNA which converted into cDNA by reverse transcription. The success of reverse transcription is reflected by positive amplification curves in the PCR (**Figure 9b**). These results clearly reflect that DNA toolbox and reverse transcription can proceed sequentially and phenotyping of live cells can be performed followed by genotyping.

### Example 8: Single-stranded DNA release from microparticles

In this example, the release single-stranded oligodeoxyribonucleotides from hydrogel particles, conditional to the presence of an input strand produced by an endogenous DNA-toolbox amplification reaction, is demonstrated (**Figure 10a**). Streptavidin-modified hydrogel particles (Streptavidin Sepharose High Performance affinity resin, GE Healthcare) were grafted with a biotinylated oligonucleotide (Cbe12-1S4bioteg, Biomers) at an average density of 375 attomoles (~2.3.10⁸ strands) per particles. The oligonucleotide is designed to include a releasable 3' input site (complementary to the sequence α, where α is 5'ATTCAGGATCG3', SEQ ID NO: 20) and a 5' output site complementary to the sequence α' (where α' is 5'CATTCAGGATCG3', SEQ ID NO: 21). The oligonucleotide also includes a restriction site of the enzyme Bsml (New England Biolabs, NEB) between the input and output sites. These DNA-grafted particle are immersed in a reaction mixture containing the reaction buffer, and a DNA toolbox amplification mixture (an amplification template for strand α, a DNA polymerase [Bst warm start, NEB], a nicking enzyme [Nb.Bsml, NEB], the restriction enzyme [Bsml, NEB], an exonuclease [ttRecJ] and a double strand specific dye [Evagreen, Biotium]) (see table below).

**Table 7**

| Components | Concentrations |
|---|---|
| **Enzymes** | |
| Nb.Bsml nickase | 400 u/ml |
| Bst 2.0 warm start polymerase | 2.8 u/ml |
| ttRecJ exonuclease | 25 nM |
| Bsml | 500 u/ml |
| **Streptavidin-conjugated sepharose particles** | |
| Cbe12-1S4bioteg | 375 amol/part |
| Bioteg *C*G*A*TCCTGAATGCGATCCTGAAT *p* | |
| corresponding to sequence SEQ ID NO: 19 | |
| **Reaction buffer and temperature** | |
| Tris-HCl (pH 7.9) | 20 mM |
| (NH₄)₂SO₄ | 10 mM |
| KCI | 10 mM |
| MgSO₄ | 8.4 mM |
| NaCl | 50 mM |
| Dithiothreitol | 3 mM |
| Synperonic F108 | 0.1% |
| BSA | 800 µg/ml |
| Evagreen | 0.4X |
| dATP, dCTP, dGTP, dTTP, | 50 µM |
| dUTP | 5 µM |
| Temperature | 45°C |

The reaction is incubated, and the beads are observed by fluorescence microscopy (**Figure 10 b-c**). Initially, there is only a small amount of strand alpha and the fluorescence of the beads, induced by EvaGreen, is stable. Subsequently the amplification reaction, driven by polymerization/nicking cycles, kicks in and many α' strands are produced. These α' strands bind to the complementary sequence of the bead-grafted oligonucleotides and are extended by the polymerase. This reaction converts the single-stranded oligonucleotide to a double-stranded form, resulting in a temporary increase of the fluorescence of the particle. The newly formed duplexes are cleaved by the restriction enzyme, releasing the 3' part of the particle-bound oligonucleotide in solution, which is observed by a decrease of the fluorescence intensity of the beads. From the bell-shape of the fluorescence time trace of the particles (**Figure 10c**), it can be concluded that single strand DNA is efficiently released from the hydrogel particle, conditionally to the production of the strand α, in presence of the restriction enzyme.

### Example 9: Phenotyping cell type targeting two surface markers using proximity oligo-conjugated antibody pair.

A schematic representation of phenotyping a cell targeting two surface markers using a pair of oligo-conjugated antibodies is shown in **Figure 15****.**

As described previously in example 2, specific cell types can be phenotyped targeting a cell surface marker using totalseqB oligo tagged antibody. Here, the inventors targeted two surface markers for cell phenotyping using a pair of antibodies tagged with single-stranded oligos that complement each other when the antibodies bind to cell surface makers in close proximity. The single-stranded oligos are designed such that their complementary hybridization will lead to strand extension by Bst large fragment polymerase, producing a NbBsml nicking site, followed by nickase mediated release of the extension (B11a), which in turn triggers the DNA toolbox. This approach can be used to target one cell surface marker (using only one antibody, half of the molecules tagged with one of the complementary strands and half with the other) or two cell surface markers (using two different antibodies specific to two different surface markers, each tagged with one of the complementary strands). This will allow the inventors to phenotype even more specific subtype of cells from a heterogeneous population.

The inventors first examined the proximity oligo design and reaction parameters in bulk. Human anti-CD3-TotalseqB and Human anti-CD45-TotalseqB antibodies were used for the assay to target cell surface markers CD3 and CD45. The following oligo sequences were designed such that they contain totalseqB complementary sequence, so that the oligo can be hybridized to totalseqB antibodies.

**Table 8**

| *Name* | *sequence* | *Function* |
|---|---|---|
| *aTSB-B11b-2* | TTGCTAGGACCGGCCTTAAAGCTTTTTTTTTTTTCTTCAAG TAG (SEQ ID NO: 22) | Activator |
| *aTSB-B11b-2toa* | TTGCTAGGACCGGCCTTAAAGCTTTTTTTTTTTT/sp18/CTA ACTGAATGCTACTTGAATG/Phos/ (SEQ ID NO: 23/sp18/SEQ ID NO: 114/Phos/) | cT |

The common oligonucleotide sequence in *aTSB-B11b-2* and *aTSB-B11b-2toa* (5'TTGCTAGGACCGGCCTTAAAGC, (SEQ ID NO: 24)) is complementary to TotalseqB sequence and is used to hybridize the strands on the antibodies. TTTTTTTTTTTT (SEQ ID NO: 25) acts as a flexible linker. Downstream to the common sequence is the activator sequence in *aTSB-B11b-2,* which gets extended by DNA polymerase. In *aTSB-B11b-2toa* strand, downstream to the common sequence is a non-oligo nucleotide spacer sp18 (18-atom hexa-ethyleneglycol spacer) followed by B11b to B11a converter sequence. The sp18 does not allow the activator sequence to get extended by DNA polymerase into the converter sequence.

Jurkat cells were phenotyped targeting CD3 and CD45 surface markers using:
*CD3-totalseqB-aTSB-B11b-2* and *CD3-totalseqB-aTSB-B11b-2toa (CD3-CD3)* combination or,
*CD3-totalseqB-aTSB-B11b-2* and *CD45-totalseqB-aTSB-B11b-2toa (CD3-CD45)* combination
The above oligo conjugated antibodies were prepared as follows:
***CD3-totalseqB-aTSB-B11b-2***

**Table 9**

| **Components** | **Volume** (µl) |
|---|---|
| 10µM aTSB-B11b-2 | 4 |
| anti-human CD3-totalseqB Ab(0.5mg/ml) | 2 |
| [UCHT1, 300477] | |
| 1x TBS | 14 |
| Total | 20 |

***CD3-totalseqB-aTSB-B11b-2toa***

**Table 10**

| **Components** | **Volume** (µl) |
|---|---|
| 10µM aTSB-B11b-2toa | 4 |
| anti-human CD3-totalseqB Ab(0.5mg/ml) [UCHT1, 300477] | 2 |
| 1x TBS | 14 |
| Total | 20 |

***CD45-totalseqB-aTSB-B11b-2toa***

**Table 11**

| **Components** | **Volume** (µl) |
|---|---|
| 10µM aTSB-B11b-2toa | 4 |
| anti-human CD45-totalseqB Ab(0.5mg/ml) [Hl30, 304066] | 2 |
| 1x TBS | 14 |
| Total | 20 |

The above conjugates were incubated over-night at room temperature and stored at 4°C before using. 1x10⁶ Jurkat cells suspended in 200µl 1X TBS and 200µl Biolegend staining buffer were stained with 2 µl of each antibody pair and incubated at 4°C for 30 mins. Unbound antibodies were removed from the cell suspension by washing 2 times in 1ml 1X TBS and centrifuging at 250rcf for 5 mins. The cell pellet was finally resuspended in 50µl 1X TBS.

The following master mix was prepared:

**Table 12**

| **Components** | **Volume in µl and final concentration** |
|---|---|
| 5x FS Buffer (Invitrogen) | 20 |
| 120mM MgSO4 (NEB B1003S) | 2.5 (3mM) |
| 1mM dNTP | 1.5 (15µM each) |
| 200µM Netropsin | 1 (2µM) |
| 10µM CB11a-2noPS3 | 1.5 (150nM) |
| 10µM rT B11 aAtto633S++ | 1 (100nM) |
| 1µM pTB11aA3 | 0.4 (4nM) |
| BSA (NEB B9000S) | 1 |
| NbBsml (NEB R0706S) | 2.5 |
| Nt.BstNBI (NEB 0607S) | 1 |
| Bst LF (M0275S) | 1 |
| Water | 36.6 |
| Total | 70 |

### Modified Sequences:

**Table 13**

| Name | Sequence | Function |
|---|---|---|
| CB11a-2noPS3 | 5'C*T*A*ACTGAATGCTAACTGAA3' | aT |
| | (SEQ ID NO: 26, not showing the PTO modifications) | |
| rT B11aAtto633 S++ | 5'-Atto663*C*A*T*CGATATAC*TA*AC*TG*AA*TGCGA*T*G* BHQ2-3' | rT |
| | (Atto663 SEQ ID NO: 27 BHQ2-3', not showing the PTO modifications) | |
| pTB11aA3 | 5'A*A*A*C*TAACTGAATG3' | pT |
| | (SEQ ID NO: 28, not showing the PTO modifications) | |

| | | |
|---|---|---|
| ***"*" indicates phosphothioate (PTO) modifications on the bases*** | | |

Approximately 50000 labelled cells were spiked in an amplification mixture composed of the above master mix. Samples of 10 µL were incubated at 37°C in BioRad CFX96 real time PCR and the fluorescence of the probe (Atto633) was recorded every 2 minutes.

Cq values from amplification curves shows successful phenotyping of cells targeting two surface markers using the proximity oligos conjugated on the antibody pairs (see **Figures 16** **and** **17**). The similarity of Cq values in CD3-CD3 and CD3-CD45 experiments reflects the reproducibility of the proximity oligos irrespective of the antibody target. The reaction is only triggered when both the oligo conjugates are present; presence of one conjugate does not trigger the molecular program as shown by the negative control (CD3).

### Example 10: Phenotyping microdroplet encapsulated single cells, targeting two surface markers using proximity oligo-conjugated antibody pair in droplets.

To increase the phenotypic specificity of single cell subtypes from a heterogeneous population, the inventors introduced single cell phenotyping using proximity extension assay targeting two surface markers on cells encapsulated in microdroplets. As described in the example above, the antibody-oligo conjugate pair was used to phenotype droplet encapsulated single cells. 1x10⁶ Jurkat cells were stained using *CD3-totalseqB-aTSB-B11b-2* and *CD3-totalseqB-aTSB-B11b-2toa (CD3-CD3)* combination and unbound antibodies removed as explained before. 1x10⁶ Jurkat cells were stained using *CD3-totalseqB-aTSB-B11b-2* (CD3); this population served as negative control. The following master mix was prepared:

**Table 14**

| **Components** | **Volume in µl and final concentration** |
|---|---|
| 5x FS Buffer (Invitrogen) | 20 |
| 120mM MgSO4 (NEB B1003S) | 2.5 (3mM) |
| 1mM dNTP | 1.5 (15µM each) |
| 200µM Netropsin | 1 (2µM) |
| 10µM CB11a-2noPS3 | 1.5 (150nM) |
| 10µM rT B11 aAtto633S++ | 1 (100nM) |
| 1µM pTB11aA3 | 0.4 (4nM) |
| BSA (NEB B9000S) | 1 |
| NbBsml (NEB R0706S) | 2.5 |
| Nt.BstNBI (NEB 0607S) | 1 |
| Bst LF (M0275S) | 1 |
| Carboxymethylcellulose (20mg/ml) | 20 |
| Water | 16.6 |
| Total | 70 |

20% Carboxymethylcellulose (CMC) was used to maintain the density of master mix and avoid cellular precipitation during the process of droplet encapsulation. CMC was found to have no effect on the molecular program (data not shown). Encapsulation of cells with master mix, chamber filling, data acquisition and analysis was performed as explained before. The droplet emulsion containing CD3-CD3 labelled cells were colour barcoded as colourless whereas droplet emulsion containing CD3 labelled cells were colour barcoded blue using 1% cascade blue.

As seen in **Figure 18****,** the Cy5 fluorescence intensity of individual droplets was digitally acquired using a droplet analysis pipeline. Fluorescence intensities was normalized by dividing the fluorescence intensity values of droplets at each time point by fluorescence intensity values at time point zero. A threshold of 1.5 was considered for plotting number of drops of each population above the threshold over the time period of imaging. Maximum drops containing cells labelled with proximity oligo tagged antibody pair (CD3-CD3) gets triggered and cross threshold between 20 to 38 minutes time points whereas only few drops containing cells labelled with one antibody (CD3) cross the threshold post 70 minutes. This indicates a time window of approximately 30 minutes between specific and non-specific reaction. It also indicates the assay has less than 3% false positive drops.

### Example 11: Detection of secretory cytokines using proximity extension assay and DNA toolbox in bulk reactions

Phenotyping cells on the basis of their ability to secrete cytokines can be achieved using oligo-conjugated antibodies. The ability of a cell type in a heterogenous population or subtypes of cells in a population to release cytokines provides information on its activation status and signaling. The inventors use a combination of proximity extension and DNA toolbox to detect cell secretory molecules. A pair of single single-stranded oligo conjugated antibodies specific to two different epitopes on a secretory molecule hybridize when the antibody binds to its target, leading to polymerase mediated strand extension and nickase mediated release of signal sequence (B11a) which triggers the DNA toolbox and in turn produces a fluorescent readout confirming the secretion of cytokine by the cell.

**Figure 19** is a schematic diagram explaining the concept of proximity assay based detection of target protein. A pair of antibodies specific to two different epitopes of the target protein are used. These antibodies are covalently grafted with single stranded oligos having base complementary ends. The antibodies on binding to the target protein will lead to complementary base-pairing of the oligos. DNA polymerase mediated strand extension will lead to creation of a double stranded nicking site. Nickase mediated release of the extended strand activates the DNA toolbox which produces a fluorescent read-out confirming the presence of target protein. In absence of target protein, the antibody pairs do not interact, thus the DNA toolbox remain inactivated causing no fluorescence.

The fluorescent readout is a function of the concentration of cytokine produced by the cell, with higher cytokine levels leading to earlier fluorescent switching. Using this approach cells can be phenotyped and categorized on the basis of cytokine expression. A cascade of oligo conjugated antibodies specific to multiple cytokines can also be used along with the DNA toolbox.

The inventors used Biolegend anti-human monoclonal TNFα antibodies (MAb1 and MAb11; 1mg/ml) specific to two different epitopes on TNFα. Using Abcam oligonucleotide conjugation kit (ab218260) a 5' aminated 30 bp common sequence called "link30" was attached to the antibodies. After antibody-oligo conjugation, link30 complementary cLink30-T12-B11b-2 was hybridized to MAb1 and cLink30-T12-B11b-2toa was hybridized to MAb11 by incubating overnight at room temperature. Oligo-conjugated antibody was precipitated (to remove unbound oligos) and resuspended following manufacturer protocol.

**Table 15**

| ***Name*** | ***Sequence*** | ***Function*** |
|---|---|---|
| link30-aminated | /5AmMC6/CCCACAGGCACGCTCAACGCTAACCAAACC (/5AmMC6/SEQ ID NO: 29, wherein 5AmMC6 is a 5' Amino linker with a hexane spacer | Linker (for covalent modification of antibody) |
| cLink30-T12-B11b-2 | GGT TTG GTT AGC GTT GAG CGT GCC TGT GGG TTT TTT TTT TTT CTT CAA GTA G (SEQ ID NO: 30) | Activator |
| cLink30-T12-B11b-2toa | GGT TTG GTT AGC GTT GAG CGT GCC TGT GGG TTT TTT TTT TTT /Sp18/CT AAC TGA ATG CTA CTT GAA TG/3Phos/ (SEQ ID NO: 31/Sp18/SEQ ID NO: 115/3Phos/) | cT |

As explained above the common sequence in both the oligos are used to hybridize the strands on cLink30 conjugated on antibodies and the non-oligo nucleotide spacer sp18 prevents the complementary strand from getting extended beyond the converter sequence.

The inventors found the concentration of oligo-conjugated antibody used in the assay is an important parameter, as there is no washing step to remove unbound antibodies. Too many antibody molecules in the reaction can trigger false proximity. So, the maximum concentration of antibodies which does not trigger false proximity was first researched. Anti-TNF antibody-oligo conjugates were serially diluted and incubated with or without 15nM recombinant TNFα (rTNFα) at 4°C for 30. After incubation the following master mix was added and fluorescence intensity of DNA toolbox reporter probe was recorded using CFX96 at 37°C every 2 minutes. The Cq values of the amplification curve of each reaction was extracted and plotted. 1:10⁴ fold dilution of each oligo-conjugated antibody was found to detect and differentiate between 0 and 15nM rTNFα. Higher concentrations of the antibodies produced false proximity.

### Master Mix:

**Table 16**

| **Components** | **Volume (µl)** |
|---|---|
| 5x FS Buffer (Invitrogen) | 20 |
| 120mM MgSO4 (NEB B1003S) | 2.5 (3mM) |
| 1mM dNTP | 1.5 (15µM each) |
| 200µM Netropsin | 1 (2µM) |
| 10µM CB11a-2noPS3 | 1.5 (150nM) |
| 10µM rT B11 aAtto633S++ | 1 (100nM) |
| 1µM pTB11aA3 | 0.2 (2nM) |
| BSA (NEB B9000S) | 1 |
| NbBsml (NEB R0706S) | 2.5 |
| Nt.BstNBI (NEB 0607S) | 1 |
| Bst LF (M0275S) | 1 |
| Water | 36.8 |
| Total | 70 |

It was first determined antibody dilution for rTNFα detection using proximity assay (see **Figure 20**). 1:10⁴ fold dilution of each oligo-conjugated antibody was found to detect and differentiate between 0 and 15nM rTNFα. Lower dilutions of the antibodies produced false proximity.

The inventors then used 1:10⁴ dilution of the antibodies to detect various concentrations of rTNFα. The master mix composition, reaction setup and data acquisition were performed as describe above. The assay was clearly able to detect rTNFα in the reaction mix. Auto triggering of the system happened after 90 minutes giving a time window of at least 40 minutes.

Presence of 30nM TNFα causing proximity mediated hybridization of the conjugated oligos resulting in triggering of DNA toolbox at approximately 50 minutes. Auto-triggering of the reaction occurs after at least 90 minutes (**see** **Figure 21**).

### Example 12: Detection of rTNFα using proximity extension assay and DNA toolbox in droplets

For the purpose of detecting secretory cytokines by proximity assay in droplets, the inventors used the anti-TNFα antibodies (MAb1 & MAb11) conjugated proximity oligos (as explained before) to detect 30nM recombinant TNFα. 30nM rTNFα was incubated with 1:10⁴ dilution of each antibody at room temperature for 30 minutes and mixed with the following master mix. Antibodies mixed with 1X TBS was similarly incubated with the antibodies as a negative control. 1% cascade blue was mixed with the negative control reaction. 80pL droplets were produced from the mix containing master mix, 30nM rTNFα and antibodies (drop code colourless) and mix containing master mix, 1X TBS and antibodies (drop code blue) using separate PDMS devices. The droplet emulsions from both the mixes were collected together and analyzed by 2D time lapse imaging as explained before. The proximity oligo conjugated antibodies successfully detected rTNFα in drops as shown in **figure 22****.** Majority of the drops containing 30nM cytokine produced fluorescent signal within 30 to 48 minutes. Drops not containing the cytokine produced fluorescent signals after 430 minutes. This proves that proximity assay containing oligos conjugated on antibodies is efficient in detecting cytokines and trigger specific reaction in presence of the target within an hour. False proximity triggered reaction occurs at very late timepoints.

### Master Mix:

**Table 17**

| **Components** | **Volume (µl)** |
|---|---|
| 5x FS Buffer (Invitrogen) | 20 |
| 120mM MgSO4 (NEB B1003S) | 2.5 (3mM) |
| 1mM dNTP | 1.5 (15µM each) |
| 200µM Netropsin | 1 (2µM) |
| 10µM CB11a-2noPS3 | 1.5 (150nM) |
| 10µM rT B11aAtto633S++ | 1 (100nM) |
| 1µM pTB11aA3 | 0.3 (2nM) |
| BSA (NEB B9000S) | 1 |
| NbBsml (NEB R0706S) | 2.5 |
| Nt.BstNBI (NEB 0607S) | 1 |
| Bst LF (M0275S) | 1 |
| Water | 36.7 |
| Total | 70 |

### Example 13: Aptamer-based proximity extension assay

Figure 23 shows the principle scheme of proximity extension assay based PUMA.

In this example, the inventors examined the possibility of detecting human alpha-Thrombin via an aptamer-proximity extension assay. This design works with the Nb.Bsml nickase enzyme, at 37°C in FS Buffer 1x and with the Bst Large Fragment (Bst LF) polymerase. The cT and the activator are hybridizing to each other on 9 bases. Due to the Nb.Bsml nicking site sequence, the design is reversible. In that case, when the activator hybridizes to the cT, is extended by a polymerase and then nicked to release a trigger, the oligonucleotides return to their initial state, i.e. as before the elongation by the polymerase. Trigger production therefore does not shift the equilibrium between the two oligonucleotides, reducing background amplification.

For a proof of principle with aptamers, the inventors decided to target the human alpha- Thrombin with two aptamers: the 15mer and the 29mer, reported in the same review (Deng , 2014). The activator and cT sequences optimized by the inventors, as well as a T12 linker were added to the sequences of the two selected aptamers, as illustrated on figure 23.

**Table 18**

| Name | Sequence | Function |
|---|---|---|
| 15mer-cT | CTAACTGAATGCTACTTGAATGTTTTTTTTTTTTGGTTGGT GTGGTTGG (SEQ ID NO: 32) | cT |
| 29mer-cT | CTAACTGAATGCTACTTGAATGTTTTTTTTTTTTAGTCCGT GGTAGGGCAGGTTGGGGTGACT (SEQ ID NO: 33) | cT |
| 15mer-act | GTTGGTGTGGTTGGTTTTTTTTTTTTTTCAAGTAG | Activator |
| | (SEQ ID NO: 34) | |
| 29mer-act | AGTCCGTGGTAGGGCAGGTTGGGGTGACTTTTTTTTTTTT TTTCAAGTAG (SEQ ID NO: 35) | Activator |

The cT is added at the 5' extremity of the aptamer while the activator is added at the 3' extremity. The Kd of the 15mer-activator (15mer-act) / 29mer-cT as well as the Kd of 15mer-cT / 29mer-act have been calculated (see table 2), giving a Kd of 104 nM at 37°C for both of the oligonucleotide sets. The first step has been to carry a range of human alpha-Thrombin, respectively 0, 1, 10 and 100 nM in presence of 0, 1, 10 and 100 nM of an equimolar mix of 15mer-act / 29mer-cT (**figure 24** **b.i.**) or in presence of 0, 1, 10 and 100 nM of an equimolar mix of 15mer-cT / 29mer-acT (**figure 24****, b.ii.**). The combination 15mer-act / 29mer-cT did not allow human alpha-Thrombin detection. However, 10 nM and 100 nM of 15mer-cT / 29mer-act allowed the detection of 100 nM of human alpha-Thrombin. 0 and 1 nM of 15mer-cT / 29mer-act did not allow human alpha-Thrombin detection. The scores_{100nM}, for the detection of 100 nM of human alpha-Thrombin, have been calculated and reported on figure 24 b.ii. The highest score was obtained with 10 nM of 15mer-cT / 29mer-act, we therefore selected these conditions for further optimization. The necessity to test both combinations 15mer-cT / 29mer-act and 15mer-act / 29mer-cT is illustrated: it is possible, due to the spatial conformations of the oligonucleotides, that only one of these combinations works. Moreover, it is also possible that adding a linker, a cT or an activator to an aptamer modifies its properties, thus preventing its binding to the targeted protein.

All reaction mixtures were assembled at 4°C in 200 µl PCR tubes. miRBuffer 4x was diluted in mQ water to reach a 1x final concentration. The templates were then added to reach the desired concentrations of aptamer1-cT, and aptamer2-act, 100 nM of aT, 30 nM of pT and 50 nM of rT. After homogenization, BSA (200 mg/mL) and the enzymes were added [Nb.Bsml (300 U/mL), Bst large fragment (36 U/mL), ttRecJ (23 nM)]. The human alpha-Thrombin was first diluted 10 times in mQ water and serially diluted on parafilm in 5 % glycerol using low-binding tips (Eppendorf).

The target protein was first added to the thermocyler tube (Bio-rad) and the mixture containing the buffer, templates and enzymes was added, reaching a final volume of 10 µL. The samples were incubated at 37°C in a thermocycler (CFX96 Touch, Bio-Rad) and the fluorescence was recorded in real-time. The time traces were normalized and the At determined as 20 % of the maximum fluorescence signal.

As the results displayed in figure 24 were obtained with miR Buffer 1x, the inventors decided to realize a range of human alpha-Thrombin, with 10 nM of 15mer-cT / 29mer-act, in FS Buffer 1x and in miRBuffer 1x (**figure 25** **c.i.**). The scores_{100nM}, for the detection of 100 nM of human alpha-Thrombin, have been calculated and reported on the figure. As the highest score was obtained in FS Buffer 1x, the inventors decided to continue to perform the PEA in this buffer. Noteworthy, the LOD is lower than for the results of **figure 24** **b.ii.** This is due to the use of new low binding pipet tips, Corning, allowing the inventors to realize more precise protein dilution. Moreover, an inherent inhibitory effect appears for 1 µM of Human alpha-Thrombin. In order to reduce the nonspecific production of trigger, the inventors compared the detection of human alpha-Thrombin with 10 nM of 15mer-cT and 10 nM of either 29mer-act of 29mer-act- V2. The 29mer-act-V2 has an activator which matches the cT on 8 bases, one base less than the 29mer-act (**figure 25** **c.ii.**). This missing base is in 5' of the activator and thus cannot be recovered by polymerase extension. The scores_{100nM} have been calculated and reported on the figure. The score is higher for the 15mer-cT / 29mer-act oligonucleotide set. Moreover, the detection of 10 nM of human alpha-Thrombin with 15mer-cT / 29mer-act-V2 is approximately 5 times slower than the detection with 15mer-act / 29mer-cT. The Kd of the 15mer-cT / 29mer-act-V2 were calculated (see table 21), giving a Kd of 156 nM at 37°C. With a higher Kd than the 15mer-cT / 29mer-act oligonucleotide set, it is logic for the production of trigger to be slower and thus for the amplification times to be higher.

Finally, a range of human alpha-Thrombin was realized in FS Buffer containing 10 nM of 15mer-cT and 29mer-act. Controls with 10 nM of scrambled aptamers have been realized. Scrambled aptamers are indicated by the "S" before the name of the aptamer (**figure 25** **c.iii.**). The detection of 100 nM of human alpha-Thrombin was achieved only for the aptamers, not for the scrambled versions, demonstrating the specificity of the detection.

**Table 19**

| Name | Sequence | Functio n |
|---|---|---|
| S-29mer-act | GAGTCGGGAGCGCTAGAGGTGTGGTTCG TTTTTTTTTTTTT TTCAAGTAG (SEQ ID NO: 36) | Activato r |
| S-15mer-cT | CTAACTGAATGCTACTTGAATGTTTTTTTTTT TT GGTGTTGGTTTGGGG (SEQ ID NO: 37) | cT |
| 29mer -act-V2 | AGTCCGTGGTAGGGCAGGTTGGGGTGAC TTTTTTTTTTTTT ATCAAGTAG (SEQ ID NO: 38) | Activato r |

All reaction mixtures were assembled at 4°C in 200 µl PCR tubes, either in FS Buffer 5x (NEB) diluted in mQ water to reach a 1x final concentration and supplemented with 25 µM of dNTPS, 3 mM of MgSO4 and 2µM of netropsin or in mirBuffer 4x diluted in mQ water to reach a 1x final concentration.

The templates were then added to reach the desired concentrations of 10 nM of aptamer1-cT, 10 nM of aptamer2-act, 100 nM of aT, 30 nM of pT and 50 nM of rT. After homogenization, BSA (200 mg/mL) and the enzymes were added [Nb.Bsml (300 U/mL), Bst large fragment (36 U/mL), ttRecJ (23 nM)]. The human alpha-Thrombin was first diluted 10 times in mQ water and serially diluted on parafilm in 5 % glycerol using low-binding tips (Eppendorf).

The target protein was first added to the thermocyler tube (Bio-rad) and the mixture containing the buffer, templates and enzymes was added, reaching a final volume of 10 µL. The samples were incubated at 37°C in a thermocycler (CFX96 Touch, Bio-Rad) and the fluorescence was recorded in real-time. The time traces were normalized and the At determined as 20 % of the maximum fluorescence signal.

In an attempt to accelerate the speed of detection of human alpha-Thrombin, the inventors designed two sets of activator / cT working with Nt.BstNBI: one containing the sequence GAGA between the trigger complementary and the Nt.BstNBI nicking site and one containing the sequence GATT (See example Self-primed template). The Kd of the GAGA activator / cT and the GATT activator / cT sets were calculated (see table 21), giving Kd of respectively 29 and 69 nM at 37°C. The lower the Kd, the higher the affinity of the two oligonucleotides and the more likely the nonspecific production of trigger. It is to be noted that the Nt.BstNBI PEA design is not reversible. Therefore, the nonspecific trigger production is increased compared to the Nb.Bmsl design.

Those two sets were tested (**figure 26** **d.**). The GAGA activator / cT set is faster than the GATT activator / cT set with and without human alpha-Thrombin. The scores_{1µM}, for the detection of 1 µM of human alpha-Thrombin, have been calculated and reported on the figure. The higher score is obtained for the GAGA oligonucleotide set. Moreover, the GAGA oligonucleotide set allows the detection of 10 nM of human alpha-Thrombin and is not sensitive to the inhibitory effect induced by 1 µM. The GATT oligonucleotide set does not allow the detection of 10 nM of human alpha-Thrombin and is sensitive to the inhibitory effect induced by 1 µM.

**Table 20**

| Name | Sequence | Function |
|---|---|---|
| 15mer-cT-GAGA | CTAACTGAATG GAGA  GACTC TTTTTTTTTTTTGGTTGGTGTGGTTGG (SEQ ID NO: 39) | cT |
| 29mer-act-GAGA | AGTCCGTGGTAGGGCAGGTTGGGGTGACTTTTTTTTTTTTTTT GAGTCTCTC (SEQ ID NO: 40) | Activator |
| 15mer-cT-GATT | CTAACTGAATG GATT  GACTC TTTTTTTTTTTTGGTTGGTGTGGTTGG (SEQ ID NO: 41) | cT |
| 29mer-act-GATT | AGTCCGTGGTAGGGCAGGTTGGGGTGACTTTTTTTTTTTTTTT GAGTCAATC (SEQ ID NO: 42) | Activator |

All reaction mixtures were assembled at 4°C in 200 µl PCR tubes. FS Buffer 5x (NEB) was diluted in mQ water to reach a 1x final concentration and supplemented with 25 µM of dNTPS, 3 mM of MgSO4 and 2µM of netropsin. The templates were then added to final concentrations of 10 nM of aptamer1-cT, 10 nM of aptamer2-act 100 nM of aT, 30 nM of pT and 50 nM of rT. After homogenization, BSA (200 mg/mL) and the enzymes were added [Nb.Bsml (300 U/mL), Nt.BstNBI (10 U/mL), Bst large fragment (36 U/mL), ttRecJ (23 nM)]. The human alpha-Thrombin was first diluted 10 times in mQ water and serially diluted on parafilm in 5 % glycerol using low-binding tips (Eppendorf).

The target protein was first added to the thermocyler tube (Bio-rad) and the mixture containing the buffer, templates and enzymes was added, reaching a final volume of 10 µL. The samples were incubated at 37°C in a thermocycler (CFX96 Touch, Bio-Rad) and the fluorescence was recorded in real-time. The time traces were normalized and the At determined as 20 % of the maximum fluorescence signal.

**Table 21: Kd for two different oligonucleotides at a given temperature. The data were obtained via Nupack. The concentration of Na+ was set to 0.08 M and the concentration of Mg++ at 0.01M. The concentrations of the different oligonucleotides were set up to 1 µM.**

| Oligonu cleotide n°1 | Sequence | Oligonuc leotide n°2 | Sequence | T( °C ) | dG kcal/mo I (Nupac k) | Kd (Nupa ck) |
|---|---|---|---|---|---|---|
| 15mer-cT | CTAACTGAATGCT ACTTGAATGTTTT TTTTTTTTGGTTG GTGTGGTTGG (SEQ ID NO: 32) | 29mer-act -V2 | AGTCCGTGGTAGGG CAGGTTGGGGTGACT TTTTTTTTTTTTATCAA GTAG (SEQ ID NO: 43) | 37 | -9,66 | 1,56E-07 |
| 15mer-cT | CTAACTGAATGCT ACTTGAATGTTTT | 29mer-act | AGTCCGTGGTAGGG CAGGTTGGGGTGACT | 37 | -9,91 | 1,04E-07 |
| | TTTTTTTTGGTTG GTGTGGTTGG (SEQ ID NO: 32) | | TTTTTTTTTTTTTTTCAA GTAG (SEQ ID NO: 44) | | | |
| 15mer-act | GGTTGGTGTGGTT GGTTTTTTTTTTTT TTCAAGTAG (SEQ ID NO: 45) | 29mer-cT | CTAACTGAATGCTAC TTGAATGTTTTTTTTT TTTAGTCCGTGGTAG GGCAGGTTGGGGTG ACT (SEQ ID NO: 33) | 37 | -9,91 | 1,04E-07 |
| 15mer-cT-GAGA | CTAACTGAATG GAGA GACTC TTTTTTTTTTTTGG TTGGTGTGGTTGG (SEQ ID NO: 39) | 29mer-act-GAGA | AGTCCGTGGTAGGG CAGGTTGGGGTGACT TTTTTTTTTTTTTT GAGTCTCTC (SEQ ID NO: 40) | 37 | -12,11 | 2,93E-09 |
| 15mer-cT-GATT | CTAACTGAATG GATT GACTC TTTTTTTTTTTTGG TTGGTGTGGTTGG (SEQ ID NO: 41) | 29mer-act-GATT | AGTCCGTGGTAGGG CAGGTTGGGGTGACT TTTTTTTTTTTTTT GAGTCAATC (SEQ ID NO: 42) | 37 | -11,58 | 6,92E-09 |

### Example 14: Self-primed template

The cT is the main component responsible for the nonspecific amplification of triggers. To tackle this issue, the inventors moved towards a method without cT. Indeed, by taking advantage of the washing steps of the cells staining, a self-primed template (sT) can be included. This template is composed of 3 parts, respectively in the 5' to 3' order: a part complementary to the trigger, the Nt.BstNBI nicking site and a stem loop allowing the 3' extremity to hybridize to the nicking site and to be ready to be extended by a polymerase. A biotin must be added to the 5' of the sT in order to bind it to a biotinylated antibody via a streptavidin. Thus, in presence of the target protein, the sT is present, whereas in absence of the target protein, it is absent. The various PUMA components are then added, allowing trigger production from the sT. The principle is illustrated on **figure 27** **a.**

The inventors investigated the effect of the environing bases of the nicking recognition site on the production of trigger. To do this, the inventors chose a sequence of sT (sequence n°1 in table 1) and ordered 48 sequences with 2 bases modification compared to the sequence n°1 and 5 sequences with more modifications (sequences n°49 to n°53 in table 1). They then looked at the amplification time (**figure 27** **b**) for 1 pM of sT with a PUMA and looked at the linear trigger production rate (**figure 27** **c**) for 1 nM of sT for the Bc switch at 50°C. The production rate in function of the inverse of the amplification time was then plotted to see if they were correlated (**figure 27** **d**). As expected, there is a correlation between these two parameters: the higher the production rate, the lower the amplification time. This confirms that the production rate of a sT is a relevant parameter to assess the efficiency of its detection by a PUMA. A very strong disparities in terms of production rates depending on the sequences was observed (table 1).

**Table 22: sT production rate in function of sT sequence for two different polymerases for the Bc switch.**

| N° | Sequence | **Producti on rate with Vent** (number of trigger produced per sT per minute) | **Product ion rate with Bst 2.0 WS** (number of trigger produce d per sT per minute) | **Functi on** |
|---|---|---|---|---|
| 1 | TGCAGTCCAGAA GAAT GACTC AC ATTGCTTCATTTTGAAGCAAT (SEQ ID NO: 46) | 0,68 | 3,36 | sT |
| 2 | TGCAGTCCAGAA AAAT GACTC AC ATTGCTTCATTTTGAAGCAAT (SEQ ID NO: 47) | 0,03 | 0,0s1 | sT |
| 3 | TGCAGTCCAGAA ATAT GACTC AC ATTGCTTCATTTTGAAGCAAT (SEQ ID NO: 48) | 0,04 | 0,04 | sT |
| 4 | TGCAGTCCAGAA AGAT GACTC AC ATTGCTTCATTTTGAAGCAAT (SEQ ID NO: 49) | 0,53 | 2,46 | sT |
| 5 | TGCAGTCCAGAA ACAT GACTC AC ATTGCTTCATTTTGAAGCAAT (SEQ ID NO: 50) | 0,45 | 1,41 | sT |
| 6 | TGCAGTCCAGAA TAAT GACTC AC ATTGCTTCATTTTGAAGCAAT (SEQ ID NO: 51) | 0,06 | 0,06 | sT |
| 7 | TGCAGTCCAGAA TTAT GACTC AC ATTGCTTCATTTTGAAGCAAT (SEQ ID NO: 52) | 0,04 | 0,05 | sT |
| 8 | TGCAGTCCAGAA TGAT GACTC AC ATTGCTTCATTTTGAAGCAAT (SEQ ID NO: 53) | 0,63 | 3,21 | sT |
| 9 | TGCAGTCCAGAA TCAT GACTC AC ATTGCTTCATTTTGAAGCAAT (SEQ ID NO: 54) | 0,31 | 1,30 | sT |
| 10 | TGCAGTCCAGAA GTAT GACTC AC ATTGCTTCATTTTGAAGCAAT (SEQ ID NO: 55) | 0,42 | 2,22 | sT |
| 11 | TGCAGTCCAGAA GGAT GACTC AC ATTGCTTCATTTTGAAGCAAT (SEQ ID NO: 56) | 0,57 | 2,99 | sT |
| 12 | TGCAGTCCAGAA GCAT GACTC AC ATTGCTTCATTTTGAAGCAAT (SEQ ID NO: 57) | 0,60 | 2,99 | sT |
| 13 | TGCAGTCCAGAA CAAT GACTC AC ATTGCTTCATTTTGAAGCAAT (SEQ ID NO: 58) | 0,50 | 0,96 | sT |
| 14 | TGCAGTCCAGAA CTAT GACTC AC ATTGCTTCATTTTGAAGCAAT (SEQ ID NO: 59) | 0,41 | 1,52 | sT |
| 15 | TGCAGTCCAGAA CGAT GACTC AC ATTGCTTCATTTTGAAGCAAT (SEQ ID NO: 60) | 0,72 | 1,38 | sT |
| 16 | TGCAGTCCAGAA CCAT GACTC AC ATTGCTTCATTTTGAAGCAAT | 0,65 | 1,45 | sT |
| | (SEQ ID NO: 61) | | | |
| 17 | TGCAGTCCAGAA GAAA GACTC AC ATTGCTTCATTTTGAAGCAAT (SEQ ID NO: 62) | 0,72 | 2,25 | sT |
| 18 | TGCAGTCCAGAA GAAG GACTC AC ATTGCTTCATTTTGAAGCAAT (SEQ ID NO: 63) | 0,42 | 2,23 | sT |
| 19 | TGCAGTCCAGAA GAAC GACTC AC ATTGCTTCATTTTGAAGCAAT (SEQ ID NO: 64) | 0,51 | 3,48 | sT |
| 20 | TGCAGTCCAGAA GATA GACTC AC ATTGCTTCATTTTGAAGCAAT (SEQ ID NO: 65) | 0,56 | 3,26 | sT |
| 21 | TGCAGTCCAGAA GATT GACTC AC ATTGCTTCATTTTGAAGCAAT (SEQ ID NO: 66) | 0,41 | 3,01 | sT |
| 22 | TGCAGTCCAGAA GATG GACTC AC ATTGCTTCATTTTGAAGCAAT (SEQ ID NO: 67) | 0,60 | 3,42 | sT |
| 23 | TGCAGTCCAGAA GATC GACTC AC ATTGCTTCATTTTGAAGCAAT (SEQ ID NO: 68) | 0,53 | 3,00 | sT |
| 24 | TGCAGTCCAGAA GAGA GACTC AC ATTGCTTCATTTTGAAGCAAT (SEQ ID NO: 69) | 1,04 | 1,56 | sT |
| 25 | TGCAGTCCAGAA GAGT GACTC AC ATTGCTTCATTTTGAAGCAAT (SEQ ID NO: 70) | 0,98 | 3,17 | sT |
| 26 | TGCAGTCCAGAA GAGG GACTC AC ATTGCTTCATTTTGAAGCAAT (SEQ ID NO: 71) | 0,68 | 3,07 | sT |
| 27 | TGCAGTCCAGAA GAGC GACTC AC ATTGCTTCATTTTGAAGCAAT (SEQ ID NO: 72) | 0,78 | 3,09 | sT |
| 28 | TGCAGTCCAGAA GACA GACTC AC ATTGCTTCATTTTGAAGCAAT (SEQ ID NO: 73) | 0,80 | 4,07 | sT |
| 29 | TGCAGTCCAGAA GACT GACTC AC ATTGCTTCATTTTGAAGCAAT (SEQ ID NO: 74) | 0,57 | 3,68 | sT |
| 30 | TGCAGTCCAGAA GACG GACTC AC ATTGCTTCATTTTGAAGCAAT (SEQ ID NO: 75) | 0,61 | 3,41 | sT |
| 31 | TGCAGTCCAGAA GACC GACTC AC ATTGCTTCATTTTGAAGCAAT (SEQ ID NO: 76) | 0,40 | 2,44 | sT |
| 32 | TGCAGTCCAGAA GAAT GACTC AA ATTGCTTCATTTTGAAGCAAT (SEQ ID NO: 77) | 0,49 | 2,67 | sT |
| 33 | TGCAGTCCAGAA GAAT GACTC AT ATTGCTTCATTTTGAAGCAAT (SEQ ID NO: 78) | 0,46 | 2,68 | sT |
| 34 | TGCAGTCCAGAA GAAT GACTC AG ATTGCTTCATTTTGAAGCAAT (SEQ ID NO: 79) | 0,52 | 2,79 | sT |
| 35 | TGCAGTCCAGAA GAAT GACTC TA ATTGCTTCATTTTGAAGCAAT (SEQ ID NO: 80) | 0,49 | 2,59 | sT |
| 36 | TGCAGTCCAGAA GAAT GACTC TT ATTGCTTCATTTTGAAGCAAT (SEQ ID NO: 81) | 0,93 | 1,82 | sT |
| 37 | TGCAGTCCAGAA GAAT GACTC TG ATTGCTTCATTTTGAAGCAAT (SEQ ID NO: 82) | 0,56 | 2,44 | sT |
| 38 | TGCAGTCCAGAA GAAT GACTC TC ATTGCTTCATTTTGAAGCAAT (SEQ ID NO: 83) | 1,00 | 1,42 | sT |
| 39 | TGCAGTCCAGAA GAAT GACTC GA ATTGCTTCATTTTGAAGCAAT | 0,41 | 2,43 | sT |
| | (SEQ ID NO: 84) | | | |
| 40 | TGCAGTCCAGAA GAAT GACTC GT ATTGCTTCATTTTGAAGCAAT (SEQ ID NO: 85) | 0,31 | 1,44 | sT |
| 41 | TGCAGTCCAGAA GAAT GACTC GG ATTGCTTCATTTTGAAGCAAT (SEQ ID NO: 86) | 0,44 | 2,68 | sT |
| 42 | TGCAGTCCAGAA GAAT GACTC GC ATTGCTTCATTTTGAAGCAAT (SEQ ID NO: 87) | 0,49 | 3,00 | sT |
| 43 | TGCAGTCCAGAA GAAT GACTC CA ATTGCTTCATTTTGAAGCAAT (SEQ ID NO: 88) | 0,34 | 1,99 | sT |
| 44 | TGCAGTCCAGAA GAAT GACTC CT ATTGCTTCATTTTGAAGCAAT (SEQ ID NO: 89) | 0,61 | 2,79 | sT |
| 45 | TGCAGTCCAGAA GAAT GACTC CG ATTGCTTCATTTTGAAGCAAT (SEQ ID NO: 90) | 0,51 | 2,65 | sT |
| 46 | TGCAGTCCAGAA GAAT GACTC CC ATTGCTTCATTTTGAAGCAAT (SEQ ID NO: 91) | 0,82 | 1,01 | sT |
| 47 | TGCAGTCCAGAA GGTT GACTC AC ATTGCTTCATTTTGAAGCAAT (SEQ ID NO: 92) | 0,65 | 3,19 | sT |
| 48 | TGCAGTCCAGAA GGAA GACTC AC ATTGCTTCATTTTGAAGCAAT (SEQ ID NO: 93) | 0,71 | 2,89 | sT |
| 49 | TGCAGTCCAGAA GGGA GACTC TC ATTGCTTCATTTTGAAGCAAT (SEQ ID NO: 94) | 0,57 | 0,70 | sT |
| 50 | TGCAGTCCAGAA CGGA GACTC TC ATTGCTTCATTTTGAAGCAAT (SEQ ID NO: 95) | 0,25 | 0,06 | sT |
| 51 | TGCAGTCCAGAA GGGA GACTC TT ATTGCTTCATTTTGAAGCAAT (SEQ ID NO: 96) | 0,62 | 0,87 | sT |
| 52 | TGCAGTCCAGAA GCGA GACTC TC ATTGCTTCATTTTGAAGCAAT (SEQ ID NO: 97) | 0,51 | 0,12 | sT |
| 53 | TGCAGTCCAGAA GGGA GACTC TG ATTGCTTCATTTTGAAGCAAT (SEQ ID NO: 98) | 0,94 | 1,77 | sT |

### Production rate:

All reaction mixtures were assembled at 4°C in 200 *µ*l PCR tubes. miRBuffer 4x was diluted in mQ water to reach a 1x final concentration. 50 nM of rT (MBBcBsmIAtto633) was then added. After homogenization, BSA (200 mg/mL) and the enzymes were added [Nt.BstNBI (10 U/mL) and Vent(exo-) (80 U/mL) ]. The targeted sT was serially diluted on parafilm in 1X Tris-EDTA buffer (Sigma Aldrich) using low-binding DNA tips (Eppendorf). sT was first added to the thermocyler tube (Bio-rad) to reach a final concentration of 1 nM and the mixture containing the buffer, templates and enzymes was added, reaching a final volume of 10 *µ*L. The samples were incubated at 50°C in a thermocycler (CFX96 Touch, Bio-Rad) and the fluorescence was recorded in real-time. The time traces were normalized, derived and the production rates extracted.

**Table 23: mirBuffer 4x composition**

| Component | Final concentration |
|---|---|
| Tris HCl pH8.9 | 20mM |
| (NH4)2SO4 | 10mM |
| KCI | 160mM |
| NaCl | 40mM |
| MgSO4 | 40mM |
| dNTP | 100µM |
| Synperonic | 0.4%- |
| Netropsin | 8µM |

### Amplification time:

All reaction mixtures were assembled at 4°C in 200 *µ*l PCR tubes. miRBuffer 4x was diluted in mQ water to reach a 1x final concentration. The templates were then added to reach final concentrations of 50 nM of CBc12-2PS4, 10 nM of pTBc12T5SP and 50 nM of MBBcBsmIAtto633. After homogenization, BSA (200 mg/mL) and the enzymes were added [Nb.Bsml (300 U/mL), Nt.BstNBI (10 U/mL), Vent(exo-) (80 U/mL), ttRecJ (23 nM)]. The targeted sT was serially diluted on parafilm in 1X Tris-EDTA buffer Sigma Aldrich) using low-binding DNA tips (Eppendorf) to reach a final concentration of 1 pM of sT. sT was first added to the thermocyler tube (Bio-rad) and the mixture containing the buffer, templates and enzymes was added, reaching a final volume of 10 *µ*L. The samples were incubated at 50°C in a thermocycler (CFX96 Touch, Bio-Rad) and the fluorescence was recorded in real-time. The time traces were normalized and the At determined as 20 % of the maximum fluorescence signal.

**Table 24**

| Name | Sequence | Function |
|---|---|---|
| NBltoBc-2+2_GAAT_AC S1 | T*GCAGTCCAGAAGAAT GACTC AC ATTGCTTCATTTTGAAGCAAT (SEQ ID NO: 99, with PTO modification on the first base) | sT |
| NBltoBc-2+2_GAAT_AC S2 | T*G*CAGTCCAGAAGAAT GACTC AC ATTGCTTCATTTTGAAGCAAT (SEQ ID NO: 99 with PTO modifications on the two first bases) | sT |
| NBltoBc-2+2_GAAT_AC S3 | T*G*C*AGTCCAGAAGAAT GACTC AC ATTGCTTCATTTTGAAGCAAT (SEQ ID NO: 99 with PTO modification on the three first bases) | sT |
| NBltoBc-2+2_GAAT_AC S4 | T*G*C*A*GTCCAGAAGAAT GACTC AC ATTGCTTCATTTTGAAGCAAT (SEQ ID NO: 99 with PTO modification on the four first bases) | sT |
| NBltoBc-2+2_GAAT_AC S5 | T*G*C*A*G*TCCAGAAGAAT GACTC AC ATTGCTTCATTTTGAAGCAAT | sT |
| | (SEQ ID NO: 99 with PTO modification on the five first bases) | |
| NBltoBc-2+2_GAAT_AC S6 | T*G*C*A*G*T*CCAGAAGAAT GACTC AC ATTGCTTCATTTTGAAGCAAT (SEQ ID NO: 99 with PTO modification on the six first bases) | sT |

| | | |
|---|---|---|
| **"*" *indicates phosphothioate (PTO) modifications on the bases*** | | |

The inventors then looked at the possibility of adding phosphorotioates to the 5' extremity of the sT to accelerate trigger production by lowering the melting temperature between the produced trigger and the self-primed template (Jaroszewski, 1996). To this end, a production rate experiment was performed by adding from 1 to 6 phosphorotioates to the same self-primed template (**figure 28** **e.i.**). The results are displayed on **figure 28** **e.ii.**

The inventors found that the presence of phosphorotioates does not further increase the production rate. This suggests that the release of triggers from the sT is not limited by the dehybridization rate of the output.

The production rates were obtained as explained above.

**Table 25**

| Name | Sequence | Function |
|---|---|---|
| NBltoBc-2+2 Sp18-T5-Biot | TTTTT/intsp18/ TGCAGTCCAGAA GTTT GACTCACATTGCTTCATTTTGAAGCAATGT GAGT (TTTTT/intsp18/SEQ ID NO: 100) | sT |
| NBltoBc-2+2 int-Biot-dT | TGCAGTCCAGAA GTTT GACTCACATTGCTTCATTT/biot/TTTGAAGCAATGT GAGT (SEQ ID NO: 101/biot/SEQ ID NO: 102) | sT |

The inventors wanted to graft this sT onto the detection antibody via a biotin-streptavidin linkage. Thus, they needed to add a biotin to the sT. 2 locations for this biotin were tested: at the 5' extremity, with a T5-Sp18 linker (TTTTT-sp18 linker) (**figure 29** **f.i**.) and at the inner position of the loop via an amino-dT building block (**figure 29** **f.ii.**). Ranges of these two different oligonucleotides were performed in presence or absence of 0.25 streptavidin equivalent. As it can be seen in **figure 29 g****,** without streptavidin, there is no significant difference between the two possibilities. However, in presence of streptavidin, the oligonucleotide with a biotin at the 5' extremity is much better detected than the oligonucleotide with a biotin at the internal position (**figure 29** **h**)**.** As already showed by the inventors, the presence of a biotin-streptavidin group on an oligonucleotide can hinder the polymerase (Gines, 2017). The T5-Sp18 linker at the 5' extremity of the sT does not seem to induce steric hindrance.

The amplification times were obtained as explained for example d. In the case of sT-Biot conjugated for streptavidin, a solution of sT-Biot : Streptavidin (1 : 0.25) at 40 nM was prepared and serially diluted on parafilm in 1X Tris-EDTA buffer Sigma Aldrich) using low-binding DNA tips (Eppendorf).

In an attempt to get faster detection, the inventor then looked at the use of another polymerase: Bst 2.0 WS. They performed a production rate experiment in presence of 0.5% Bst 2.0 WS as well as in presence of 4% Vent(exo-) for all the sequences presented in table 1. As it can be seen in **figure 30** **i.,** for a large majority of the tested sequence, the production rate is higher in presence of Bst 2.0 WS. This is encouraging for the detection of a sT. Noteworthy, the production rate obtained with Bst 2.0 WS does not strongly correlate with the production rate obtained with Vent(exo-) except for a few sequences that are very slow in both cases. This suggests that the influence of the sequence of the self-primed template on the production rate is mostly due to the polymerase.

Building on the results obtained on sT sequence optimization for the Bc switch at 50°C, the inventors investigated the possibility of transposing them to the B11a switch, operating at 37°C. They thus looked at the trigger production rate of B11a trigger obtained with Bst LF at 37°C in function of the production rates of Bc trigger obtained with Vent at 50°C for different sT. 5 different sequences were tested for the bases environing the Nt.BstNBI nicking site of the sT. These sequences are given in table 3. As it can be seen in **figure 30** **j.,** the results are quite similar between the two production rates: the sequence environing the Nt.BstNBI nicking site with the higher production rate for the B11a trigger is also the one given the higher production rate with the Bc trigger.

**Table 26: sT production rate in function of sT sequence for the B11a switch.**

| Name | Sequence | Production rate (number of Trigger produced per sT per minute) | Function |
|---|---|---|---|
| NBltoB11a-2+2_GAGT_AC | TGCTAACTGAA GAGT GACTC ACATTGCTTCATTTTGAAGCAAT (SEQ ID NO: 103) | 0,80 | sT |
| NBltoB11a-2+2_GAGA_AC | TGCTAACTGAA GAGA GACTC ACATTGCTTCATTTTGAAGCAAT (SEQ ID NO: 104) | 1,45 | sT |
| NBltoB11a-2+2_GAGC_AC | TGCTAACTGAA GAGC GACTC ACATTGCTTCATTTTGAAGCAAT (SEQ ID NO: 105) | 0,83 | sT |
| NBltoB11a-2+2_GATT_AC | TGCTAACTGAA GATT GACTC ACATTGCTTCATTTTGAAGCAAT (SEQ ID NO: 106) | 0,66 | sT |
| NBltoB11a-2+2_TCAT_AC | TGCTAACTGAA TCAT GACTC ACATTGCTTCATTTTGAAGCAAT (SEQ ID NO: 107) | 0,34 | sT |

The production rates for the Bc switch at 50°C with Bst 2.0 WS were obtained as explained for above. The Vent(exo-) (80 U/mL) was replaced by Bst 2.0 WS (40 U/mL).

The production rates for the the B11a switch at 37°C were obtained as follow:
All reaction mixtures were assembled at 4°C in 200 *µ*l PCR tubes. FS Buffer 5x (NEB) was diluted in mQ water to reach a 1x final concentration and supplemented with 25 µM of dNTPS and 3 mM of MgSO4. 50 nM of rT (MBB11aBsmlAtto633) was then added. After homogenization, BSA (200 mg/mL) and the enzymes were added [Nt.BstNBI (10 U/mL) and Bst large fragment (36 U/mL)]. The targeted sT was serially diluted on parafilm in 1X Tris-EDTA buffer (Sigma Aldrich) using low-binding DNA tips (Eppendorf). sT was first added to the thermocyler tube (Bio-rad) to reach a final concentration of 1 nM and the mixture containing the buffer, templates and enzymes was added, reaching a final volume of 10 *µ*L. The samples were incubated at 37°C in a thermocycler (CFX96 Touch, Bio-Rad) and the fluorescence was recorded in real-time. The time traces were normalized, derived and the production rates extracted.

**Table 27**

| Name | Sequence | Function |
|---|---|---|
| NBltoB11a-2+2 Sp18-T5-Biot GTTT | TTTTT\intsp18\TGCTAACTGAA GTTT GACTC ACATTGCTTCATTTTGAAGCAATGTGAGT (TTTTT\intsp18\SEQ ID NO: 108) | sT |
| NBltoB11a-2+2 Sp18-T5-Biot GAGA | TTTTT\intsp18\TGCTAACTGAA GAGA GACTC ACATTGCTTCATTTTGAAGCAAT (TTTTT\intsp18\SEQ ID NO: 109) | sT |

Following the results obtained for the sequence optimisation of the bases between the trigger complementary and the Nt.BstNBI nicking site for the production of B11a trigger with Bst LF at 37°C, the inventors decided to use these optimised self-primed template. Two designs of B11a switch self-primed template have been tested in presence of 0.25 equivalent of streptavidin (**figure 31** **k.**): the GTTT and the GAGA designs with a Sp18-T5 linker and a biotin at their 5' extremities. The faster design is the GAGA, highlighting the importance of the sT sequence for the speed of detection. This result suggests that the NBltoB11a-2+2 Sp18-T5-Biot GAGA oligonucleotide can be used for cell phenotyping.

### Amplification time:

All reaction mixtures were assembled at 4°C in 200 *µ*l PCR tubes. FS Buffer 5x (NEB) was diluted in mQ water to reach a 1x final concentration and supplemented with 25 µM of dNTPS, 3 mM of MgSO4 and 2µM of netropsin. The templates were then added to reach final concentrations of 50 nM of CB11a-2PS3, 10 nM of pTB11aPS3 and 50 nM of MBB11aBsmIAtto633. After homogenization, BSA (200 mg/mL) and the enzymes were added [Nb.Bsml (300 U/mL), Nt.BstNBI (10 U/mL), Bst large fragment (36 U/mL), ttRecJ (23 nM)]. The targeted sT was serially diluted on parafilm in 1X Tris-EDTA buffer Sigma Aldrich) using low-binding DNA tips (Eppendorf). sT was first added to the thermocyler tube (Bio-rad) and the mixture containing the buffer, templates and enzymes was added, reaching a final volume of 10 *µ*L. The samples were incubated at 37°C in a thermocycler (CFX96 Touch, Bio-Rad) and the fluorescence was recorded in real-time. The time traces were normalized and the At determined as 20 % of the maximum fluorescence signal.

### Example 15: Antibody conjugated self-primed template for phenotyping

**Table 28**

| **Name** | **sequence** | **Function** |
|---|---|---|
| NBltoB11a-2+2 Sp18-T5-Biot GAGA | Biotin TTTTT Sp18 TGCTAACTGAA GAGA GACTC ACATTGCTTCATTTTGAAGCAAT ( Biotin TTTTT Sp18 SEQ ID NO: 110) | sT |
| TSB_full_toB1 1a-2+2 | TGCTAACTGAA GAGA GACTC ACTTGCTAGGACCGGCCTTAAAGC (SEQ ID NO: 111) | cT |

TotalseqB converter template TSBtoB11a-2+2P was removed from the system by introducing two new designs of templates to be conjugated to antibody:
NBltoB11a-2+2 Sp18-T5-Biot GAGA is a biotinylated hairpin template that produces the DNA toolbox trigger B11a in presence of DNA polymerase without the need of converter template (**figure 33**). Presence of sp18 provides some flexibility while preventing the hairpin loop from being extended further, thus generating the B11a sequence. The biotinylated template can be conjugated on streptavidin tagged antibodies by overnight incubation at room temperature. This design removes the requirement of a TotalseqB tag on the antibody.

TSB_full_toB11a-2+2 is a converter template complementary to the totalseqB tag and can be hybridized to the antibody by overnight incubation at room temperature (**figure** 32). The antibody conjugated converter template produces B11a without the need of any additional converter templates.

The templates are called "self-primed template". These two designs were successfully tested to phenotype cells in bulk as well as droplet reaction as explained before.

**Figure 34** shows single cell identification using anti-CD3 totalseqB antibody hybridized to TSB_full_toB11a-2+2 and DNA toolbox. More than 95% drops containing CD3-self-primed template labelled cells crossed the Cy5 fluorescence threshold in 20 to 50 minutes; whereas majority of drops containing unlabeled cells cross the threshold after 200 minutes.

**Figure 35** shows phenotyping Jurkat cells using streptavidin tagged anti-CD3 antibody conjugated with NBltoB11a-2+2Sp18T5Biot GAGA. Labelled cells trigger the DNA toolbox in 24 minutes whereas unlabeled cells do not trigger the reaction.

### Example 16: DNA toolbox mediated phenotyping specific cell type in a heterogenous population

The inventors created a heterogenous pool of cells by mixing jurkat and mouse (P388D1) cells (1x10⁶ cell each). Anti-CD3-self-primed template antibody (as explained before) was used to phenotype jurkat cells in the heterogeneous pool. Mouse cells were stained with CalceinAM, allowing to differentiate them from jurkat cells during imaging. After labelling the heterogenous pool of cells with anti-CD3-self-primed template antibody by incubating at 4°C for 30 minutes, the unbound antibodies were removed by washing the cells 3 times with 1X TBS by centrifugation at 200g for 5 mins. The cells were then encapsulated in 80pL drops containing DNA toolbox master mix. The droplet emulsion was analyzed by 2D time lapse imaging as explained before. Anti-CD3-self-primed template antibody selectively labelled CD3 expressing jurkat cells in the heterogenous pool. Thus, only drops containing jurkat cells were able to trigger the DNA toolbox and produce Cy5 fluorescence in 20 minutes. Drops without cells did not produce any Cy5 fluorescence indicating the specificity of the assay. So, the DNA toolbox is efficient in selectively phenotyping a specific cell type in a heterogenous population.

As shown in **figure 36****,** at time point zero (T=0 mins), all drops had zero Cy5 fluorescence (i.e no background in droplets). After 20 minutes reaction (T=20 mins), the drops containing jurkat cells produced DNA toolbox mediated fluorescence (i.e background colour in droplets), whereas the drops containing mouse cells did not activate the DNA toolbox and remain unchanged in appearance.

### Selective phenotyping and genotyping

### Concept:

The final objective of selectively phenotyping cells from a heterogenous population is sequencing. This will allow obtaining genomic or transcriptomic information of the subpopulation of interest. Single cell selective phenotyping and genotyping will be achieved by droplet encapsulation of cells, barcoded hydrogel beads and DNA toolbox master mix. One droplet will contain one cell, one barcoded hydrogel bead and master mix containing the DNA toolbox templates, buffer and enzymes. The cells will be phenotyped either by targeting cell surface markers, secretory molecules, cellular organelles, etc using single/multiple oligo conjugated antibodies or aptamers or cholesterol conjugated oligos which will trigger the DNA toolbox. The output of the DNA toolbox (B11a) can be used with a reporter fluorescent probe for a fluorescent readout or it can be used with hydrogel beads with barcoded primers to capture specific genes or total mRNA. For selective primer release, the output of DNA toolbox (B11a) is converted to a sequence called "seqshave" by a converter template. Complementary hybridization of seqshave to a region on the barcoded primer leads to polymerase mediated strand extension creating a BcII resctriction site, followed by release of primers due to Bcll restriction enzyme present in the master mix. Like fluorescent readout, barcoded primer release will happen in drops containing phenotyped cells. The captured genes will be linearly amplified or mRNA converted to cDNA in drops. Due to selective primer release, barcoded DNA from phenotyped cells will be in solution whereas DNA from non-phenotyped cells will remain hybridized on beads. Centrifugation after emulsion break will segregate the genetic information of phenotyped cells from non-phenotyped ones. The barcoded DNA will be converted into sequencing libraries and sequenced.

Hydrogel beads are 10pL acrylamide beads containing acrydited random sequence followed by Bcll restriction site, DNA toolbox output hybridization site, barcode, unique molecular index, primer. The barcoding is performed by split and pool method (Grosselin, K. *et al, 2019).* So each bead has a specific barcode which gives information of the cell encapsulated with it and the UMI helps in removing PCR duplicates.

### Example 17: Phenotyped cell mediated selective primer release from hydrogel beads

In order to observe DNA toolbox mediated release of barcoded primer, the inventors ligated 200 µM synthetic primers labelled with Cy5 fluorophore (at 3' end) on hydrogel beads. 20µl bulk reactions were conducted containing different concentrations of synthetic seqshave, 20000 beads with Cy5 labelled primers and master mix as follows:

**Table 29**

| **Components** | **Volume (µl)** |
|---|---|
| 5X FS Buffer | 4 |
| Barcoded beads | 1 |
| Synthetic Seqshave | 2 |
| BcII HF | 1 |
| BstLF | 1 |
| 1mM dNTP | 2 |
| 120mM MgSO4 | 1 |
| Water | 8 |
| Total | 20 |

The reaction mix was incubated at 37°C for 2 hours and the median fluorescence of beads were evaluated using the flow cytometer. The difference in median fluorescence of beads at time point zero and at time point 2 hours was used to calculate the percentage of primer released when treated with a range of synthetic seqshave concentrations and plotted. 40pmol synthetic seqshave leads to approximately 90% primer release which does not significantly change on increasing the concentration (**Figure 37**). This provided initial proof of concept to the inventors.

Using the seqshave converter template (B11a-3toseqshave-6TTS4), the possibility of DNA toolbox mediated primer release was evaluated using flow cytometer redout. The inventors optimized the design of the seqshave converter template (B11a-3toseqshave-6TTS4) in bulk reactions and found the release of barcoded primer to be directly proportional to the concentration of converter template (**Figure 38**).

### Example 18: Phenotyped cell mediated primer release in droplets

Jurkat cells labelled with CD3-self-primed template antibody (composed of TSB labelled antibody hybridized TSB_full_toB11a-2+2) were encapsulated in 100pL droplets with Cy5 primer ligated hydrogel beads and master mix such that one droplet contains at most one cell and one hydrogel bead. The droplet emulsion was packed in 2D chamber and evaluated by time lapse imaging every 5 mins for 3 hours as explained before. The average fluorescence intensity of the droplet space without the fluorescent hydrogel bead was evaluated using ImageJ. The average fluorescent intensities of droplet containing bead and antibody labelled cell and droplet containing only bead was plotted over time. The intensity of Cy5 fluorescence in drops containing bead and labelled cell increased with time whereas there was no change in Cy5 fluorescence in drops containing only bead (**figures 39 to 41**). This proves phenotyped cell mediated release of primers from hydrogel bead.

### Master Mix:

**Table 30**

| **Components** | **Volume (µl)** |
|---|---|
| 5x FS Buffer (Invitrogen) | 20 |
| 120mM MgSO4 (NEB B1003S) | 2.5 (3mM) |
| 1mM dNTP | 3 (30µM each) |
| 200µM Netropsin | 1 (2µM) |
| 10µM CB11a-2noPS3 | 1 (100nM) |
| B11a-3toseqshave-6TTS4 | 5 (500nM) |
| 1µM pTB11aA3 | 1.2 (12nM) |
| BSA (NEB B9000S) | 1 |
| NbBsml (NEB R0706S) | 2.5 |
| Nt.BstNBI (NEB 0607S) | 1 |
| Bst LF (M0275S) | 1 |
| Superscript III (Invitrogen) | 2 |
| Superaseln | 1 |
| BcIIHF | 4 |
| Carboxymethylcellulose (20mg/ml) | 20 |
| Water | 30 |
| Total | 100 |

**Table 31**

| Name | Sequence | Function |
|---|---|---|
| TSB_full_toB11a-2+2 | TGCTAACTGAA GAGA GACTC ACTTGCTAGGACCGGCCTTAAAGC (SEQ ID NO: 112) | cT |
| B11a-3toseqshave-6TTS4 | G*T*G*G*TCTGAGTTTGACTCTAACTG (SEQ ID NO: 113, not showing the PTO modifications) | cT (produces seqshave in presence of B11a) |

| | | |
|---|---|---|
| ***"*" indicates phosphothioate (PTO) modifications on the bases*** | | |

### Bibliography:

K. Grosselin, A. Durand, J. Marsolier, A. Poitou, E. Marangoni, F. Nemati, ... & A. Gérard. High-throughput single-cell ChlP-seq identifies heterogeneity of chromatin states in breast cancer. Nature genetics, 51(6), 1060-1066, 2019
G. Gines, A. S. Zadorin, J.-C. Galas, T. Fujii, A. Estevez-Torres, and Y. Rondelez. Microscopic agents programmed by DNA circuits. Nature Nanotechnology, 12(4):351- 359, May 2017.
Bin Deng, Yanwen Lin, Chuan Wang, Feng Li, Zhixin Wang, Hongquan Zhang, Xing-Fang Li, and X. Chris Le. Aptamer binding assays for proteins: The thrombin example-A review. Analytica Chimica Acta, 837:1-15, July 2014.
J. W. Jaroszewski, V. Clausen, J. S. Cohen, and O. Dahl. NMR investigations of duplex stability of phosphorothioate and phosphorodithioate DNA analogues modified in both strands. Nucleic Acids Research, 24(5):829-834, March 1996.
Lu Li, Qian Wang, Jie Feng, Lili Tong, and Bo Tang. Highly Sensitive and Homogeneous Detection of Membrane Protein on a Single Living Cell by Aptamer and Nicking Enzyme Assisted Signal Amplification Based on Microfluidic Droplets. Analytical Chemistry, 86:5101-5107, 2014.
Zhen-zhu Zhang and Chun-yang Zhang. Highly sensitive detection of protein with aptamer-based target-triggering two-stage amplification. Analytical Chemistry, 84(3):1623-1629, 2012.

## Claims

1. A method for the selective analysis of a subpopulation of cells or organelles based on determining the level of at least one cell or organelle marker, said method comprising:
a) partitioning said cells or organelles into a plurality of compartments, so that the majority of the compartments do not comprise more than a single cell of one cellular type or a single organelle of one cellular type,
together with at least one cell or organelle marker-specific ligand coupled to at least one activator oligonucleotide or repressor oligonucleotide and
with a DNA toolbox mixture comprising at least one polymerase, at least one restriction or nicking enzyme, at least one amplification oligonucleotide able to bind and amplify a signal oligonucleotide, and a buffer comprising less than 0.7 mM phosphate ions,
b) in each compartment, letting the activator oligonucleotide(s) and/or the repressor oligonucleotide(s) bound to the cell or organelle marker, interact together and with the components of the DNA toolbox mixture, to generate one or more signal oligonucleotides and/or one or more anti-signal oligonucleotides and to amplify said signal oligonucleotide(s),
c) analyzing the cells or organelles of the compartments in which at least one signal oligonucleotide is present at a concentration over or below a threshold value.

2. The method according to claim 1, wherein the DNA toolbox mixture comprises a buffer that does not comprise phosphate ions.

3. The method according to claim 1 or 2, wherein the DNA toolbox mixture comprises one or more leak-absorption oligonucleotides able to bind the signal oligonucleotides.

4. The method according to any one of claims 1 to 3, wherein the cell or organelle marker is selected from the group consisting of a surface marker, an internal marker, a secreted or released marker and a membrane marker.

5. The method according to any one of claims 1 to 4, wherein the level of at least one cell or organelle marker is modified by the interaction between at least two co-compartmentalized cells or organelles and wherein step c) allows analysis of the interaction between the cells or organelles.

6. The method according to any one of claims 1 to 5, wherein the marker is not a secreted or released marker and wherein said method comprises, before step a):
- mixing said cells or organelles with at least one cell or organelle marker-specific ligand coupled to an activator or repressor oligonucleotide, and
- optionally, washing the cells or organelles to remove unbound cell or organelle marker-specific ligands coupled to an activator or repressor oligonucleotide.

7. The method according to any one of claims 1 to 6, wherein
- the activator oligonucleotide coupled to the cell or organelle marker-specific ligand generates one or more signal oligonucleotide by acting as template and/or as a primer for the polymerase, and/or
- the repressor oligonucleotide coupled to the cell or organelle marker-specific ligand generates one or more anti-signal oligonucleotide by acting as a template and/or as a primer for the polymerase.

8. The method according to any one of claims 1 to 7, wherein the cell or organelle marker-specific ligands coupled to at least one activator oligonucleotide or repressor oligonucleotide comprise at least one pair of ligands, said pair of ligands comprising a first cell or organelle marker-specific ligand and a second cell or organelle marker-specific ligand, and wherein the binding of said first and second ligands to their marker induces proximity extension or proximity ligation between the activator or repressor oligonucleotide coupled to said first ligand and the activator or repressor oligonucleotide coupled to said second ligand, said proximity extension or proximity ligation leading to the generation of one or more signal oligonucleotides or one or more anti-signal oligonucleotide(s) and to amplification of said signal oligonucleotide(s).

9. The method according to any one of claims 1 to 8, wherein the cell or organelle marker-specific ligand is a membrane anchor and in step b) the amount of activator or repressor oligonucleotide(s) present in the compartment is proportional to the surface of the membrane of the enclosed cell(s), so that the concentration of the signal oligonucleotide(s) in step c) is a function of the presence or absence of a compartmentalized cell and/or of the number of compartmentalized cell(s) and/or of the size of the compartmentalized cell(s).

10. The method according to any one of claims 1 to 9, wherein the signal oligonucleotide is a master signal oligonucleotide produced from at least two different signal oligonucleotides.

11. The method according to any one of claims 1 to 10, wherein in step a) the DNA toolbox mixture further comprises at least one enzyme selected from the group consisting an exonuclease, an endonuclease, a reverse transcriptase, a ligase, a recombinase, a glycosylase and a DNA-processing enzyme.

12. The method according to any one of claims 1 to 11, wherein the compartment is a microdroplet, a microcompartment or a cage.

13. The method according to any one of claims 1 to 12, wherein step b) is performed at a temperature comprised from 20°C to 45°C.

14. The method according to any one of claims 1 to 13, wherein step c) comprises determining the concentration of a signal oligonucleotide by adding a reporter probe, wherein said reporter probe is specifically activated by said signal oligonucleotide, and detecting a signal emitted by the reporter probe bound to said signal oligonucleotide.

15. The method according to any one of claims 1 to 14, wherein step c) comprises recovering the contents of the compartments in which at least one signal oligonucleotide is present at a concentration over or below a threshold value.

16. The method according to claim 15, wherein the contents of said recovered contents are analyzed by sequencing cellular or organellar nucleic acids and/or labeling nucleic acid sequence(s).

17. The method according to claim any one of claims 1 to 16, wherein step c) comprises recovering the cells of the compartments in which at least one signal oligonucleotide is present at a concentration over or below a threshold value and cultivating the cells prior to further analysis.

18. The method according to claim 17, wherein the cellular or organellar nucleic acids and/or labeling nucleic acid sequence(s) located in a same compartment are associated with a compartment-specific barcode sequence.

19. The method according to claim 18, wherein the cellular or organellar nucleic acids and/or labeling nucleic acid comprise a compartment-specific barcode sequence further to extension of a cDNA on a barcoded primer, extension of a cDNA on a barcoded template switching oligonucleotide, ligation of a barcoded adapter, or recombination with a barcoded recombination site.

20. The method according to claim 19, wherein the barcoded primer, the barcoded template-switching oligonucleotide, the barcoded adaptor or the barcoded recombination site comprises at least one photolabile protecting group and wherein in step c), for the compartments wherein the signal(s) from the reporter probe(s) is above or below a threshold value, photodeprotection of said barcoded primer, barcoded template-switching oligonucleotide, barcoded adaptor or barcoded recombination site, thereby allowing the selective association of the cellular or organellar nucleic acids and/or labeling nucleic acids with a compartment-specific barcode sequence.

21. The method according to claim 18, wherein the barcoded primer, the barcoded template-switching oligonucleotide, the barcoded adaptor or the barcoded recombination site comprises photocleavable linkage and, wherein in step c), for the compartments wherein the signal(s) from the reporter probe(s) is above or below a threshold value, photocleavage of said barcoded primer sequence, barcoded template-switching oligonucleotide, barcoded adaptor or barcoded recombination site occurs, thereby allowing the selective release of the cellular or organellar nucleic acids and/or labeling nucleic acid comprising primer sequences, template-switching oligo sequences, adapter sequences or recombination sites and a compartment-specific sequence barcode from a solid support.

22. The method according to claim 17, wherein in step c), the binding, and optionally extension, of the signal oligonucleotide, results in the selective release of the cellular or organellar nucleic acids and/or labeling nucleic acids comprising a barcoded primer, a barcoded template-switching oligonucleotide, a barcoded adaptor or a barcoded recombination site and a compartment-specific sequence barcode from a solid support.

23. The method according to claim 21 or 22, wherein the solid support is a bead, each compartment comprising no more than a single bead, and the barcoded primers, the barcoded template-switching oligonucleotides, the barcoded adaptors or the barcoded recombination sites carry a barcode sequence specific to the bead of said compartment.

24. The method according to any one of claims 21 to 23, wherein the solid support is the wall of a microcompartment or cage, and the barcoded primers, barcoded template-switching oligonucleotides, barcoded adaptors or barcoded recombination sites carry a compartment-specific barcode sequence.

25. The method according to any one of claims 18 to 24, wherein
- the DNA toolbox mixture in step a) comprises a polymerase with reverse transcriptase activity,
- each compartment in step a) comprises oligonucleotides carrying a compartment-specific barcode sequence and a region able to bind RNA, wherein said oligonucleotides are attached to a solid support,
- in step c), the cells or organelles are lysed thereby releasing RNA and the oligonucleotides carrying a compartment-specific barcode sequence and a region able to bind RNA are used to prime cDNA synthesis, catalysed by a polymerase with reverse transcriptase activity, on the released RNA, resulting in cDNA carrying the compartment-specific barcode,
- in step c), the cDNA carrying the compartment-specific barcode is released from the solid support when at least a signal oligonucleotide is present at a concentration over or below a threshold value and the released barcoded cDNAs and/or the non-released barcoded cDNAs are analyzed by sequencing.

26. The method according to any one of claims 15 to 25 wherein the recovered contents of the compartments are analyzed by sequencing to determine the concentration of the signal oligonucleotide.

27. The method according to any one of claims 15 to 26, wherein step c) comprises determining the concentration of said at least one signal oligonucleotide by adding a quantification oligonucleotide, wherein said quantification oligonucleotide is specifically amplified when said at least one signal oligonucleotide is present at a concentration over or below a threshold value.

28. The method according to claim 27, wherein the quantification oligonucleotides located in a same compartment are associated with a compartment-specific barcode sequence.

29. The method according to claim 28, wherein the quantification oligonucleotide comprises a compartment-specific barcode sequence further to extension of a cDNA on a barcoded primer, extension of a cDNA on a barcoded template switching oligonucleotide, ligation of a barcoded adapter, or recombination with a barcoded recombination site.

30. A kit for implementing the method of any one of claims 18 to 29, comprising:
a) at least one polymerase, at least one nicking enzyme or restriction enzyme, and optionally at least one enzyme selected from the group consisting of an exonuclease, an endonuclease, a reverse transcriptase, a ligase, a recombinase, a glycosylase and a DNA-processing enzyme,
b) at least one cell or organelle marker-specific ligand coupled to at least one activator or repressor oligonucleotide,
c) at least one amplification oligonucleotide able to bind and amplify a signal oligonucleotide, and, optionally, at least one oligonucleotide selected from the group consisting of a leak-absorption oligonucleotide to avoid nonspecific amplification, a conversion oligonucleotide able to convert the activator or repressor oligonucleotide into a signal anti-signal oligonucleotide, a reporting probe allowing detection of a signal oligonucleotide, and a quantification oligonucleotide allowing determining the concentration of a signal oligonucleotide,
d) a set of barcoded nucleic acids comprising a barcoded primer, a barcoded template switching oligonucleotide, a barcoded adapter or a barcoded recombination site, and
e) optionally, a microfluidic device.

## Patentansprüche

1. Verfahren zur selektiven Analyse einer Subpopulation von Zellen oder Organellen basierend auf dem Bestimmen des Niveaus von mindestens einem Zell- oder Organellenmarker, wobei das Verfahren umfasst:
a) Aufteilen der Zellen oder Organellen in eine Vielzahl von Kompartimenten, so dass die Mehrzahl der Kompartimente nicht mehr als eine einzige Zelle eines Zelltyps oder eine einzige Organelle eines Zelltyps umfasst,
zusammen mit mindestens einem zell- oder organellenmarkerspezifischen Liganden, der an mindestens ein Aktivator-Oligonukleotid oder Repressor-Oligonukleotid gekoppelt ist, und
mit einer DNA-Toolbox-Mischung, die mindestens eine Polymerase, mindestens ein Restriktions- oder Nicking-Enzym, mindestens ein Amplifikations-Oligonukleotid, das ein Signal-Oligonukleotid binden und amplifizieren kann, und einen Puffer umfasst, der weniger als 0,7 mM Phosphationen umfasst,
b) in jedem Kompartiment Interagierenlassen des/der Aktivator-Oligonukleotide und/oder des/der Repressor-Oligonukleotide, die an den Zell- oder Organellenmarker gebunden sind, miteinander und mit den Komponenten der DNA-Toolbox-Mischung, um ein oder mehrere Signal-Oligonukleotide und/oder ein oder mehrere Antisignal-Oligonukleotide zu erzeugen und das/die Signal-Oligonukleotide zu amplifizieren,
c) Analysieren der Zellen oder Organellen der Kompartimente, in denen mindestens ein Signal-Oligonukleotid in einer Konzentration über oder unter einem Schwellenwert vorhanden ist.

2. Verfahren nach Anspruch 1, wobei die DNA-Toolbox-Mischung einen Puffer umfasst, der keine Phosphationen umfasst.

3. Verfahren nach Anspruch 1 oder 2, wobei die DNA-Toolbox-Mischung ein oder mehrere Leckabsorptions-Oligonukleotide umfasst, die in der Lage sind, die Signal-Oligonukleotide zu binden.

4. Verfahren nach einem der Ansprüche 1 bis 3, wobei der Zell- oder Organellenmarker ausgewählt ist aus der Gruppe, bestehend aus einem Oberflächenmarker, einem internen Marker, einem sekretierten oder freigesetzten Marker und einem Membranmarker.

5. Verfahren nach einem der Ansprüche 1 bis 4, wobei das Niveau von mindestens einem Zell- oder Organellenmarker durch die Interaktion zwischen mindestens zwei kokompartimentierten Zellen oder Organellen modifiziert wird und wobei Schritt c) eine Analyse der Interaktion zwischen den Zellen oder Organellen ermöglicht.

6. Verfahren nach einem der Ansprüche 1 bis 5, wobei der Marker kein sekretierter oder freigesetzter Marker ist und wobei das Verfahren vor Schritt a) umfasst:
- Mischen der Zellen oder Organellen mit mindestens einem zell- oder organellenmarkerspezifischen Liganden, der an ein Aktivator- oder Repressor-Oligonukleotid gekoppelt ist, und
- optional Waschen der Zellen oder Organellen, um ungebundene zell- oder organellenmarkerspezifische Liganden, die an ein Aktivator- oder Repressor-Oligonukleotid gekoppelt sind, zu entfernen.

7. Verfahren nach einem der Ansprüche 1 bis 6, wobei
- das an den zell- oder organellenmarkerspezifischen Liganden gekoppelte Aktivator-Oligonukleotid ein oder mehrere Signal-Oligonukleotide erzeugt, indem es als Matrize und/oder als Primer für die Polymerase fungiert, und/oder
- das an den zell- oder organellenmarkerspezifischen Liganden gekoppelte Repressor-Oligonukleotid ein oder mehrere Antisignal-Oligonukleotide erzeugt, indem es als Matrize und/oder als Primer für die Polymerase fungiert.

8. Verfahren nach einem der Ansprüche 1 bis 7, wobei die zell- oder organellenmarkerspezifischen Liganden, die an mindestens ein Aktivator-Oligonukleotid oder Repressor-Oligonukleotid gekoppelt sind, mindestens ein Paar von Liganden umfassen, wobei das Paar von Liganden einen ersten zell- oder organellenmarkerspezifischen Liganden und einen zweiten zell- oder organellenmarkerspezifischen Liganden umfasst, und wobei die Bindung des ersten und zweiten Liganden an ihren Marker eine Proximity-Extension oder Proximity-Ligation zwischen dem Aktivator- oder Repressor-Oligonukleotid, das an den ersten Liganden gekoppelt ist, und dem Aktivator- oder Repressor-Oligonukleotid, das an den zweiten Liganden gekoppelt ist, bewirkt, wobei die Proximity-Extension oder Proximity-Ligation zur Erzeugung eines oder mehrerer Signal-Oligonukleotide oder eines oder mehrerer Antisignal-Oligonukleotide und zur Amplifikation des/der Signal-Oligonukleotide führt.

9. Verfahren nach einem der Ansprüche 1 bis 8, wobei der zell- oder organellenmarkerspezifische Ligand ein Membrananker ist und in Schritt b) die Menge des/der im Kompartiment vorhandenen Aktivator- oder Repressor-Oligonukleotide proportional zur Oberfläche der Membran der eingeschlossenen Zelle(n) ist, so dass die Konzentration des/der Signal-Oligonukleotide in Schritt c) eine Funktion des Vorhandenseins oder Nichtvorhandenseins einer kompartimentierten Zelle und/oder der Anzahl der kompartimentierten Zelle(n) und/oder der Größe der kompartimentierten Zelle(n) ist.

10. Verfahren nach einem der Ansprüche 1 bis 9, wobei das Signal-Oligonukleotid ein Master-Signal-Oligonukleotid ist, das aus mindestens zwei unterschiedlichen Signal-Oligonukleotiden hergestellt ist.

11. Verfahren nach einem der Ansprüche 1 bis 10, wobei in Schritt a) die DNA-Toolbox-Mischung ferner mindestens ein Enzym umfasst, das ausgewählt ist aus der Gruppe, bestehend aus einer Exonuklease, einer Endonuklease, einer reversen Transkriptase, einer Ligase, einer Rekombinase, einer Glycosylase und einem DNA-verarbeitenden Enzym.

12. Verfahren nach einem der Ansprüche 1 bis 11, wobei das Kompartiment ein Mikrotropfen, ein Mikrokompartiment oder ein Käfig ist.

13. Verfahren nach einem der Ansprüche 1 bis 12, wobei Schritt b) bei einer Temperatur von 20 °C bis 45 °C durchgeführt wird.

14. Verfahren nach einem der Ansprüche 1 bis 13, wobei Schritt c) das Bestimmen der Konzentration eines Signal-Oligonukleotids durch Hinzufügen einer Meldesonde, wobei die Meldesonde durch das Signal-Oligonukleotid spezifisch aktiviert wird, und das Nachweisen eines Signals, das von der an das Signal-Oligonukleotid gebundenen Meldesonde abgegeben wird, umfasst.

15. Verfahren nach einem der Ansprüche 1 bis 14, wobei Schritt c) das Zurückgewinnen des Inhalts der Kompartimente umfasst, in denen mindestens ein Signal-Oligonukleotid in einer Konzentration über oder unter einem Schwellenwert vorhanden ist.

16. Verfahren nach Anspruch 15, wobei der Inhalt des zurückgewonnenen Inhalts durch Sequenzieren von zellulären oder organellaren Nukleinsäuren und/oder (einer) Markierungsnukleinsäuresequenz(en) analysiert wird.

17. Verfahren nach einem der Ansprüche 1 bis 16, wobei Schritt c) das Zurückgewinnen der Zellen der Kompartimente, in denen mindestens ein Signal-Oligonukleotid in einer Konzentration über oder unter einem Schwellenwert vorhanden ist, und das Kultivieren der Zellen vor der weiteren Analyse umfasst.

18. Verfahren nach Anspruch 17, wobei die zellulären oder organellaren Nukleinsäuren und/oder Markierungsnukleinsäuresequenz(en), die sich im selben Kompartiment befinden, einer kompartimentspezifischen Barcode-Sequenz zugeordnet sind.

19. Verfahren nach Anspruch 18, wobei die zellulären oder organellaren Nukleinsäuren und/oder die Markierungsnukleinsäure eine kompartimentspezifische Barcode-Sequenz umfassen, zusätzlich zur Extension einer cDNA an einem barcodierten Primer, Extension einer cDNA an einem barcodierten Matrizenwechsel-Oligonukleotid, Ligation eines barcodierten Adapters oder Rekombination mit einer barcodierten Rekombinationsstelle.

20. Verfahren nach Anspruch 19, wobei der barcodierte Primer, das barcodierte Matrizenwechsel-Oligonukleotid, der barcodierte Adaptor oder die barcodierte Rekombinationsstelle mindestens eine photolabile Schutzgruppe umfasst und wobei in Schritt c) für die Kompartimente, in denen das/die Signale von der/den Meldesonden über oder unter einem Schwellenwert liegen, eine Photoentschützung des barcodierten Primers, des barcodierten Matrizenwechsel-Oligonukleotids, des barcodierten Adaptors oder der barcodierten Rekombinationsstelle erfolgt, wodurch die selektive Zuordnung der zellulären oder organellaren Nukleinsäuren und/oder Markierungsnukleinsäuren mit einer kompartimentspezifischen Barcode-Sequenz ermöglicht wird.

21. Verfahren nach Anspruch 18, wobei der barcodierte Primer, das barcodierte Matrizenwechsel-Oligonukleotid, der barcodierte Adaptor oder die barcodierte Rekombinationsstelle eine photospaltbare Verknüpfung umfasst und wobei in Schritt c) für die Kompartimente, in denen das/die Signale von der/den Meldesonden über oder unter einem Schwellenwert liegen, eine Photospaltung der barcodierten Primersequenz, des barcodierten Matrizenwechsel-Oligonukleotids, des barcodierten Adaptors oder der barcodierten Rekombinationsstelle erfolgt, wodurch die selektive Freisetzung der zellulären oder organellaren Nukleinsäuren und/oder der Markierungsnukleinsäure, umfassend Primersequenzen, Matrizenwechsel-Oligosequenzen, Adaptersequenzen oder Rekombinationsstellen und einen kompartimentspezifischen Sequenzbarcode, von einem festen Träger ermöglicht wird.

22. Verfahren nach Anspruch 17, wobei in Schritt c) die Bindung, und optional Extension, des Signal-Oligonukleotids zur selektiven Freisetzung der zellulären oder organellaren Nukleinsäuren und/oder Markierungsnukleinsäuren, umfassend einen barcodierten Primer, ein barcodiertes Matrizenwechsel-Oligonukleotid, einen barcodierten Adaptor oder eine barcodierte Rekombinationsstelle und einen kompartimentspezifischen Sequenz-Barcode, von einem festen Träger führt.

23. Verfahren nach Anspruch 21 oder 22, wobei der feste Träger eine Perle ist, wobei jedes Kompartiment nicht mehr als eine einzige Perle umfasst und wobei die barcodierten Primer, die barcodierten Matrizenwechsel-Oligonukleotide, die barcodierten Adaptoren oder die barcodierten Rekombinationsstellen eine Barcode-Sequenz tragen, die für die Perle des Kompartiments spezifisch ist.

24. Verfahren nach einem der Ansprüche 21 bis 23, wobei der feste Träger die Wand eines Mikrokompartiments oder Käfigs ist und die barcodierten Primer, barcodierten Matrizenwechsel-Oligonukleotide, barcodierten Adaptoren oder barcodierten Rekombinationsstellen eine kompartimentspezifische Barcode-Sequenz tragen.

25. Verfahren nach einem der Ansprüche 18 bis 24, wobei
- die DNA-Toolbox-Mischung in Schritt a) eine Polymerase mit reverser Transkriptaseaktivität umfasst,
- jedes Kompartiment in Schritt a) Oligonukleotide umfasst, die eine kompartimentspezifische Barcode-Sequenz und eine Region tragen, die RNA binden kann, wobei die Oligonukleotide an einen festen Träger gebunden sind,
- in Schritt c) die Zellen oder Organellen lysiert werden, wodurch RNA freigesetzt wird, und die Oligonukleotide, die eine kompartimentspezifische Barcode-Sequenz und eine Region tragen, die RNA binden kann, verwendet werden, um cDNA-Synthese, katalysiert durch eine Polymerase mit reverser Transkriptaseaktivität, an der freigesetzten RNA auszulösen, wodurch cDNA entsteht, die den kompartimentspezifischen Barcode trägt,
- in Schritt c) die den kompartimentspezifischen Barcode tragende cDNA von dem festen Träger freigesetzt wird, wenn mindestens ein Signal-Oligonukleotid in einer Konzentration über oder unter einem Schwellenwert vorhanden ist, und die freigesetzten barcodierten cDNAs und/oder die nicht freigesetzten barcodierten cDNAs durch Sequenzieren analysiert werden.

26. Verfahren nach einem der Ansprüche 15 bis 25, wobei der zurückgewonnene Inhalt der Kompartimente durch Sequenzieren analysiert wird, um die Konzentration des Signal-Oligonukleotids zu bestimmen.

27. Verfahren nach einem der Ansprüche 15 bis 26, wobei Schritt c) das Bestimmen der Konzentration des mindestens einen Signal-Oligonukleotids durch Hinzufügen eines Quantifizierungs-Oligonukleotids umfasst, wobei das Quantifizierungs-Oligonukleotid spezifisch amplifiziert wird, wenn das mindestens eine Signal-Oligonukleotid in einer Konzentration über oder unter einem Schwellenwert vorhanden ist.

28. Verfahren nach Anspruch 27, wobei die Quantifizierungs-Oligonukleotide, die sich im selben Kompartiment befinden, einer kompartimentspezifischen Barcode-Sequenz zugeordnet werden.

29. Verfahren nach Anspruch 28, wobei das Quantifizierungs-Oligonukleotid eine kompartimentspezifische Barcode-Sequenz umfasst, zusätzlich zur Extension einer cDNA an einem barcodierten Primer, Extension einer cDNA an einem barcodierten Matrizenwechsel-Oligonukleotid, Ligation eines barcodierten Adapters oder Rekombination mit einer barcodierten Rekombinationsstelle.

30. Kit zum Implementieren des Verfahrens nach einem der Ansprüche 18 bis 29, umfassend:
a) mindestens eine Polymerase, mindestens ein Nicking-Enzym oder Restriktionsenzym und optional mindestens ein Enzym, ausgewählt aus der Gruppe bestehend aus einer Exonuklease, einer Endonuklease, einer reversen Transkriptase, einer Ligase, einer Rekombinase, einer Glycosylase und einem DNA-verarbeitenden Enzym,
b) mindestens einen zell- oder organellenmarkerspezifischen Liganden, der an mindestens ein Aktivator- oder Repressor-Oligonukleotid gekoppelt ist,
c) mindestens ein Amplifikations-Oligonukleotid, das ein Signal-Oligonukleotid binden und amplifizieren kann, und optional mindestens ein Oligonukleotid, ausgewählt aus der Gruppe bestehend aus einem Leckabsorptions-Oligonukleotid zur Vermeidung unspezifischer Amplifikation, einem Konversions-Oligonukleotid, das das Aktivator- oder Repressor-Oligonukleotid in ein Signal-Antisignal-Oligonukleotid umwandeln kann, einer Meldesonde, die das Nachweisen eines Signal-Oligonukleotids ermöglicht, und einem Quantifizierungs-Oligonukleotid, das das Bestimmen der Konzentration eines Signal-Oligonukleotids ermöglicht,
d) einen Satz barcodierter Nukleinsäuren, umfassend einen barcodierten Primer, ein barcodiertes Matrizenwechsel-Oligonukleotid, einen barcodierten Adapter oder eine barcodierte Rekombinationsstelle, und
e) optional eine Mikrofluidvorrichtung.

## Revendications

1. Procédé d'analyse sélective d'une sous-population de cellules ou d'organites en fonction de la détermination du niveau d'au moins un marqueur de cellule ou d'organite, ledit procédé comprenant :
a) le partitionnement desdites cellules ou desdits organites en une pluralité de compartiments, de sorte que la majorité des compartiments ne comprennent pas plus d'une seule cellule d'un type cellulaire ou d'un seul organite d'un type cellulaire,
conjointement avec au moins un ligand spécifique d'un marqueur de cellule ou d'organite couplé à au moins un oligonucléotide activateur ou un oligonucléotide répresseur et
avec un mélange de boîte à outils des ADN comprenant au moins une polymérase, au moins une enzyme de restriction ou d'entaillage, au moins un oligonucléotide d'amplification capable de se lier à et d'amplifier un oligonucléotide de signal, et un tampon comprenant moins de 0,7 mM d'ions phosphate,
b) dans chaque compartiment, le fait de laisser le(s) oligonucléotide(s) activateur(s) et/ou le(s) oligonucléotide(s) répresseur(s) lié(s) au marqueur de cellule ou d'organite, interagir ensemble et avec les composants du mélange de boîte à outils des ADN, pour générer un ou plusieurs oligonucléotides de signal et/ou un ou plusieurs oligonucléotides anti-signal et pour amplifier ledit(lesdits) oligonucléotide(s) de signal,
c) l'analyse des cellules ou organites des compartiments dans lesquels au moins un oligonucléotide de signal est présent à une concentration supérieure ou inférieure à une valeur seuil.

2. Procédé selon la revendication 1, dans lequel le mélange de boîte à outils des ADN comprend un tampon qui ne comprend pas d'ions phosphate.

3. Procédé selon la revendication 1 ou 2, dans lequel le mélange de boîte à outils des ADN comprend un ou plusieurs oligonucléotides d'absorption des fuites capables de se lier aux oligonucléotides de signal.

4. Procédé selon l'une quelconque des revendications 1 à 3, dans lequel le marqueur de cellule ou d'organite est choisi dans le groupe constitué d'un marqueur de surface, d'un marqueur interne, d'un marqueur sécrété ou libéré et d'un marqueur de membrane.

5. Procédé selon l'une quelconque des revendications 1 à 4, dans lequel le niveau d'au moins un marqueur de cellule ou d'organite est modifié par l'interaction entre au moins deux cellules ou organites co-compartimentés et dans lequel l'étape c) permet d'analyser l'interaction entre les cellules ou organites.

6. Procédé selon l'une quelconque des revendications 1 à 5, dans lequel le marqueur n'est pas un marqueur sécrété ou libéré et dans lequel ledit procédé comprend, avant l'étape a) :
- le mélange desdites cellules ou desdits organites avec au moins un ligand spécifique d'un marqueur de cellule ou d'organite couplé à un oligonucléotide activateur ou répresseur, et
- éventuellement, le lavage des cellules ou des organites pour éliminer les ligands spécifiques du marqueur de cellule ou d'organite non liés couplés à un oligonucléotide activateur ou répresseur.

7. Procédé selon l'une quelconque des revendications 1 à 6, dans lequel
- l'oligonucléotide activateur couplé au ligand spécifique du marqueur de cellule ou d'organite génère un ou plusieurs oligonucléotides de signal en agissant comme matrice et/ou comme amorce pour la polymérase, et/ou
- l'oligonucléotide répresseur couplé au ligand spécifique du marqueur de cellule ou d'organite génère un ou plusieurs oligonucléotides anti-signal en agissant comme matrice et/ou comme amorce pour la polymérase.

8. Procédé selon l'une quelconque des revendications 1 à 7, dans lequel les ligands spécifiques du marqueur de cellule ou d'organite couplés à au moins un oligonucléotide activateur ou oligonucléotide répresseur comprennent au moins une paire de ligands, ladite paire de ligands comprenant un premier ligand spécifique du marqueur de cellule ou d'organite et un second ligand spécifique du marqueur de cellule ou d'organite, et dans lequel la liaison desdits premier et second ligands à leur marqueur induit une extension de proximité ou une ligature de proximité entre l'oligonucléotide activateur ou répresseur couplé audit premier ligand et l'oligonucléotide activateur ou répresseur couplé audit second ligand, ladite extension de proximité ou ligature de proximité conduisant à la génération d'un ou plusieurs oligonucléotides de signal ou d'un ou plusieurs oligonucléotides anti-signal et à l'amplification dudit(desdits) oligonucléotide(s) de signal.

9. Procédé selon l'une quelconque des revendications 1 à 8, dans lequel le ligand spécifique du marqueur de cellule ou d'organite est un ancrage membranaire et dans l'étape b) la quantité d'oligonucléotide(s) activateur(s) ou répresseur(s) présent(s) dans le compartiment est proportionnelle à la surface de la membrane de la(des) cellule(s) enfermée(s), de sorte que la concentration de l'oligonucléotide ou des oligonucléotides de signal à l'étape c) est fonction de la présence ou de l'absence d'une cellule compartimentée et/ou du nombre de cellules compartimentées et/ou de la taille de la ou des cellules compartimentées.

10. Procédé selon l'une quelconque des revendications 1 à 9, dans lequel l'oligonucléotide de signal est un oligonucléotide de signal maître produit à partir d'au moins deux oligonucléotides de signal différents.

11. Procédé selon l'une quelconque des revendications 1 à 10, dans lequel, à l'étape a), le mélange de boîte à outils des ADN comprend en outre au moins une enzyme choisie dans le groupe constitué d'une exonucléase, d'une endonucléase, d'une transcriptase inverse, d'une ligase, d'une recombinase, d'une glycosylase et d'une enzyme de traitement de l'ADN.

12. Procédé selon l'une quelconque des revendications 1 à 11, dans lequel le compartiment est une microgouttelette, un microcompartiment ou une cage.

13. Procédé selon l'une quelconque des revendications 1 à 12, dans lequel l'étape b) est réalisée à une température comprise entre 20 °C et 45 °C.

14. Procédé selon l'une quelconque des revendications 1 à 13, dans lequel l'étape c) comprend la détermination de la concentration d'un oligonucléotide de signal en ajoutant une sonde rapporteur, dans lequel ladite sonde rapporteur est spécifiquement activée par ledit oligonucléotide de signal, et la détection d'un signal émis par la sonde rapporteur liée audit oligonucléotide de signal.

15. Procédé selon l'une quelconque des revendications 1 à 14, dans lequel l'étape c) comprend la récupération des contenus des compartiments dans lesquels au moins un oligonucléotide de signal est présent à une concentration supérieure ou inférieure à une valeur seuil.

16. Procédé selon la revendication 15, dans lequel les contenus desdits contenus récupérés sont analysés par séquençage des acides nucléiques cellulaires ou organellaires et/ou de la ou des séquences d'acides nucléiques de marquage.

17. Procédé selon l'une quelconque des revendications 1 à 16, dans lequel l'étape c) comprend la récupération des cellules des compartiments dans lesquels au moins un oligonucléotide de signal est présent à une concentration supérieure ou inférieure à une valeur seuil et la culture des cellules avant de procéder à une analyse supplémentaire.

18. Procédé selon la revendication 17, dans lequel les acides nucléiques cellulaires ou organellaires et/ou la ou les séquences d'acides nucléiques de marquage situés dans un même compartiment sont associés à une séquence de code-barres spécifique au compartiment.

19. Procédé selon la revendication 18, dans lequel les acides nucléiques cellulaires ou organellaires et/ou l'acide nucléique de marquage comprennent une séquence de code-barres spécifique au compartiment après l'extension d'un ADNc sur une amorce à code-barres, l'extension d'un ADNc sur un oligonucléotide de commutation de matrice à code-barres, la ligature d'un adaptateur à code-barres ou la recombinaison avec un site de recombinaison à code-barres.

20. Procédé selon la revendication 19, dans lequel l'amorce à code-barres, l'oligonucléotide de commutation de matrice à code-barres, l'adaptateur à code-barres ou le site de recombinaison à code-barres comprend au moins un groupe de protection photolabile et dans lequel, à l'étape c), pour les compartiments dans lesquels le(s) signal(s) de la(des) sonde(s) rapporteuse(s) est(sont) supérieur(s) ou inférieur(s) à une valeur seuil, la photodéprotection de ladite amorce à code-barres, dudit oligonucléotide de commutation de matrice à code-barres, dudit adaptateur à code-barres ou dudit site de recombinaison à code-barres, permettant ainsi l'association sélective des acides nucléiques cellulaires ou organellaires et/ou des acides nucléiques de marquage avec une séquence de code-barres spécifique au compartiment.

21. Procédé selon la revendication 18, dans lequel l'amorce à code-barres, l'oligonucléotide de commutation de matrice à code-barres, l'adaptateur à code-barres ou le site de recombinaison à code-barres comprend une liaison photoclavable et, dans lequel, à l'étape c), pour les compartiments dans lesquels le(s) signal(s) de la(des) sonde(s) rapporteuse(s) est(sont) supérieur(s) ou inférieur(s) à une valeur seuil, le photoclavage de ladite séquence d'amorce à code-barres, dudit oligonucléotide de commutation de matrice à code-barres, ledit adaptateur à code-barres ou ledit site de recombinaison à code-barres se produit, permettant ainsi la libération sélective des acides nucléiques cellulaires ou organellaires et/ou de l'acide nucléique de marquage comprenant des séquences d'amorces, des séquences d'oligos de commutation de matrice, des séquences d'adaptateurs ou des sites de recombinaison et un code-barres de séquence spécifique au compartiment, à partir d'un support solide.

22. Procédé selon la revendication 17, dans lequel à l'étape c), la liaison, et éventuellement l'extension, de l'oligonucléotide de signal, entraîne la libération sélective des acides nucléiques cellulaires ou organellaires et/ou des acides nucléiques de marquage comprenant une amorce à code-barres, un oligonucléotide de commutation de matrice à code-barres, un adaptateur à code-barres ou un site de recombinaison à code-barres et un code-barres de séquence spécifique au compartiment, à partir d'un support solide.

23. Procédé selon la revendication 21 ou 22, dans lequel le support solide est une bille, chaque compartiment ne comprenant pas plus d'une seule bille, et les amorces à code-barres, les oligonucléotides de changement de matrice à code-barres, les adaptateurs à code-barres ou les sites de recombinaison à code-barres portent une séquence de code-barres spécifique à la bille dudit compartiment.

24. Procédé selon l'une quelconque des revendications 21 à 23, dans lequel le support solide est la paroi d'un microcompartiment ou d'une cage, et les amorces à code-barres, les oligonucléotides de changement de matrice à code-barres, les adaptateurs à code-barres ou les sites de recombinaison à code-barres portent une séquence de code-barres spécifique au compartiment.

25. Procédé selon l'une quelconque des revendications 18 à 24, dans lequel
- le mélange de boîte à outils des ADN à l'étape a) comprend une polymérase ayant une activité de transcriptase inverse,
- chaque compartiment de l'étape a) comprend des oligonucléotides portant une séquence de code-barres spécifique au compartiment et une région capable de se lier à l'ARN, dans lequel lesdits oligonucléotides sont attachés à un support solide,
- à l'étape c), les cellules ou organites sont lysés, ce qui libère l'ARN, et les oligonucléotides portant une séquence de code-barres spécifique au compartiment et une région capable de se lier à l'ARN sont utilisés pour amorcer la synthèse de l'ADNc, catalysée par une polymérase ayant une activité de transcriptase inverse, sur l'ARN libéré, ce qui donne un ADNc portant le code-barres spécifique au compartiment,
- à l'étape c), l'ADNc portant le code-barres spécifique au compartiment est libéré du support solide lorsqu'au moins un oligonucléotide de signal est présent à une concentration supérieure ou inférieure à une valeur seuil et les ADNc à code-barres libérés et/ou les ADNc à code-barres non libérés sont analysés par séquençage.

26. Procédé selon l'une quelconque des revendications 15 à 25 dans lequel les contenus récupérés des compartiments sont analysés par séquençage pour déterminer la concentration de l'oligonucléotide de signal.

27. Procédé selon l'une quelconque des revendications 15 à 26, dans lequel l'étape c) comprend la détermination de la concentration dudit au moins un oligonucléotide de signal en ajoutant un oligonucléotide de quantification, dans lequel ledit oligonucléotide de quantification est spécifiquement amplifié lorsque ledit au moins un oligonucléotide de signal est présent à une concentration supérieure ou inférieure à une valeur seuil.

28. Procédé selon la revendication 27, dans lequel les oligonucléotides de quantification situés dans un même compartiment sont associés à une séquence de code-barres spécifique au compartiment.

29. Procédé selon la revendication 28, dans lequel l'oligonucléotide de quantification comprend une séquence de code-barres spécifique au compartiment après l'extension d'un ADNc sur une amorce à code-barres, l'extension d'un ADNc sur un oligonucléotide de commutation de matrice à code-barres, la ligature d'un adaptateur à code-barres, ou la recombinaison avec un site de recombinaison à code-barres.

30. Kit pour la mise en œuvre du procédé selon l'une quelconque des revendications 18 à 29, comprenant :
a) au moins une polymérase, au moins une enzyme d'entaillage ou de restriction, et éventuellement au moins une enzyme choisie dans le groupe constitué d'une exonucléase, d'une endonucléase, d'une transcriptase inverse, d'une ligase, d'une recombinase, d'une glycosylase et d'une enzyme de traitement de l'ADN,
b) au moins un ligand spécifique d'un marqueur de cellule ou d'organite couplé à au moins un oligonucléotide activateur ou répresseur,
c) au moins un oligonucléotide d'amplification capable de se lier à et d'amplifier un oligonucléotide de signal et, éventuellement, au moins un oligonucléotide choisi dans le groupe constitué d'un oligonucléotide d'absorption de fuite pour éviter l'amplification non spécifique, un oligonucléotide de conversion capable de convertir l'oligonucléotide activateur ou répresseur en un oligonucléotide de signal anti-signal, une sonde rapporteuse permettant la détection d'un oligonucléotide de signal, et un oligonucléotide de quantification permettant de déterminer la concentration d'un oligonucléotide de signal,
d) un ensemble d'acides nucléiques à code-barres comprenant une amorce à code-barres, un oligonucléotide de commutation de matrice à code-barres, un adaptateur à code-barres ou un site de recombinaison à code-barres, et
e) éventuellement, un dispositif microfluidique.
